# EUROPEAN PATENT SPECIFICATION

(11) **EP 2 342 331 B1**
(45) Date of publication and mention of the grant of the patent: **11.11.2015**
(21) Application number: 09792933.5
(22) Date of filing: 24.09.2009
(51) Int. Cl.: C12N 9/88, C12N 15/60, C12N 15/82, A01H 5/00, A01H 5/10

(54) **HERBICIDE-RESISTANT AHAS-MUTANTS AND METHODS OF USE**
HERBIZIDRESISTENTE AHAS-MUTANTEN UND VERWENDUNGSVERFAHREN
MUTANTS AHAS RÉSISTANTS AUX HERBICIDES ET LEURS PROCÉDÉS D'UTILISATION

(30) Priority: 26.09.2008 US 100541 P
(43) Date of publication of application: 13.07.2011
(60) Divisional application: 13198892.5
(73) Proprietor: BASF Agrochemical Products, B.V., 6035 EA Arnhem (NL)
(72) Inventor: BEETHAM, Peter, Carlsbad, CA 92011 (US); CARLSON, Dale, Apex, NC 27502 (US); GOCAL, Greg, San Diego, CA 92129 (US); MC ELVER, John, Durham, NC 27712 (US); PEARCE, James, San Diego, CA 92109 (US); SCHOPKE, Christian, San Diego, CA 92126 (US); SINGH, Bijay, Cary, NC 27519 (US); WALKER, Keith, San Diego, CA 92130 (US)
(74) Representative: Williams, Richard Andrew Norman
(86) International application number: PCT/US2009/058169
(87) International publication number: WO 2010/036771

(56) References cited:
- WO-A1-2009/046334
- WO-A2-01/25460
- WO-A2-2007/005581
- WO-A2-2008/124495
- WO-A2-2009/031031
- US-A- 5 013 659
- US-B1- 6 613 963
- US-B2- 7 355 098
- LI D. ET AL.: "A mutation at the Ala122 position of acetohydroxyacid synthase (AHAS) located on chromosome 6D of wheat: improved resistance to imidazolinone and a faster assay for marker assisted selection" MOLECULAR BREEDING, vol. 22, 21 March 2008 (2008-03-21), pages 217-225, XP002557654
- TAN S ET AL: "IMIDAZOLINONE-TOLERANT CROPS: HISTORY, CURRENT STATUS AND FUTURE" PEST MANAGEMENT SCIENCE, WILEY & SONS, BOGNOR REGIS, GB, vol. 61, no. 3, 1 January 2005 (2005-01-01), pages 246-257, XP009058795 ISSN: 1526-498X
- RAY K ET AL: "Mutant acetolactate synthase gene is an efficient in vitro selectable marker for the genetic transformation of Brassica juncea (oilseed mustard)" JOURNAL OF PLANT PHYSIOLOGY, FISCHER, STUTTGART, DE, vol. 161, no. 9, 20 September 2004 (2004-09-20), pages 1079-1083, XP004955445 ISSN: 0176-1617
- BOUTSALIS PETER ET AL: "Molecular basis of resistance to acetolactate synthase-inhibiting herbicides in Sisymbrium orientale and Brassica tournefortii" PESTICIDE SCIENCE, vol. 55, no. 5, May 1999 (1999-05), pages 507-516, XP002571860 ISSN: 0031-613X
- HATTORI J ET AL: "AN ACETOHYDROXY ACID SYNTHASE MUTANT REVEALS A SINGLE SITE INVOLVED IN MULTIPLE HERBICIDE RESISTANCE" MOLECULAR AND GENERAL GENETICS, SPRINGER VERLAG, BERLIN, DE, vol. 246, 1 January 1995 (1995-01-01), pages 419-425, XP002008914 ISSN: 0026-8925
- DUGGLEBY ET AL: "Structure and mechanism of inhibition of plant acetohydroxyacid synthase" PLANT PHYSIOLOGY AND BIOCHEMISTRY, GAUTHIER-VILLARS, PARIS, FR, vol. 46, no. 3, 14 January 2008 (2008-01-14), pages 309-324, XP022550175 ISSN: 0981-9428

## Description

### FIELD OF THE INVENTION

This invention relates to herbicide-resistant Brassica plants and novel polynucleotide sequences that encode AHAS-inhibiting herbicide-resistant Brassica acetohydroxyacid synthase large subunit proteins, seeds of such plants, and methods using such plants.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (Singh (1999) "Biosynthesis of valine, leucine and isoleucine," in Plant Amino Acid, Singh, B.K., ed., Marcel Dekker Inc. New York, New York, pp. 227-247). AHAS is the site of action of five structurally diverse herbicide families including the sulfonylureas (Tan et al. (2005) Pest Manag. Sci. 61:246-57; Mallory-Smith and Retzinger (2003) Weed Technology 17:620-626; 'LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288); Tan et al. (2005) Pest Manag. Sci. 61:246-57; Mallory-Smith and Retzinger (2003) Weed Technology 17:620-626); and the sulfonylamino-carbonyltriazolinones (Tan et al. (2005) Pest Manag. Sci. 61:246-57; Mallory-Smith and Retzinger (2003) Weed Technology 17:620-626); and the N-sulfonylamino carbonyls. Imidazolinone and sulfonylurea herbicides are widely used in modem agriculture due to their effectiveness at very low application rates and relative non-toxicity in animals. By inhibiting AHAS activity, these families of herbicides prevent further growth and development of susceptible plants including many weed species, see e.g. US7,355,098 relating to PMi1/PM2 AHAS mutants.

To enable farmers greater flexibility in the types and rates of imidazolinone and sulfonylurea herbicides they use, a stronger herbicide tolerance is often desired. Also, plant breeders who develop herbicide tolerant varieties want to work with mutations that provide greater herbicide tolerance, allowing them greater flexibility in the germplasm backgrounds they use to develop their varieties. To produce such AHAS-inhibiting herbicide-resistant varieties, plant breeders need to develop additional breeding lines, preferably with increased AHAS-inhibiting herbicide-resistance. Thus, additional AHAS-inhibiting herbicide-resistant breeding lines and varieties of crop plants, as well as methods and compositions for the production and use of AHAS-inhibiting herbicide-resistant breeding lines and varieties, are needed.

### SUMMARY OF THE INVENTION

The present invention provides isolated nucleic acid molecules encoding an acetohydroxyacid synthase large subunit (AHASL) protein comprising a mutation at a position corresponding to position A 122 of SEQ ID NO: 23 and a mutation at a position corresponding to position S653 of SEQ ID NO: 23 as defined in claim 1.

In another embodiment, the present invention provides expression vectors comprising an isolated nucleic acid molecule encoding a *Brassica* acetohydroxyacid synthase large subunit (AHASL) protein comprising a mutation at a position corresponding to position A122 of SEQ ID NO: 23 and a mutation at a position corresponding to position S653 of SEQ ID NO: 23.

In still another embodiment, the present invention provides *Brassica* plants comprising a mutagenized nucleic acid molecule encoding an herbicide tolerant *Brassica* acetohydroxyacid synthase large subunit (AHASL) protein comprising a mutation at a position corresponding to position A122 of SEQ ID NO: 23 and a mutation at a position corresponding to position S653 of SEQ ID NO: 23.

In a further embodiment, the present invention provides *Brassica* seeds capable of producing a *Brassica* plant comprising a mutagenized nucleic acid molecule encoding a *Brassica* acetohydroxyacid synthase large subunit (AHASL) protein having a mutation at a position corresponding to position A122 of SEQ ID NO: 23 and a mutation at a position corresponding to position S653 of SEQ ID NO: 23.

In another embodiment, the present invention provides containers of *Brassica* seeds wherein the container comprises at least 10% seeds capable of producing *Brassica* plants comprising a mutated nucleic acid molecule encoding a *Brassica* acetohydroxyacid synthase large subunit (AHASL) protein having a mutation at a position corresponding to position A122 of SEQ ID NO: 23 and a mutation at a position corresponding to position S653 of SEQ ID NO: 23.In still a further embodiment, the present invention provides methods of controlling weeds in the vicinity of a *Brassica* plant, said method comprising applying an effective amount of an AHAS-inhibiting herbicide to the weeds and to the *Brassica* plant, wherein the *Brassica* plant comprises in its genome at least one copy of a mutagenized acetohydroxyacid synthase large subunit (AHASL) encoding nucleic acid molecule that encodes an herbicide resistant AHASL protein having a mutation at a position corresponding to position A122 of SEQ ID NO: 23 and a mutation at a position corresponding to position S653 of SEQ ID NO: 23.

The present disclosure also provides method of breeding a *Brassica* plant, wherein the method comprises: crossing a first *Brassica* line with a second *Brassica* line, wherein the first *Brassica* line is a *Brassica* plant obtained from growing a seed of mutant *Brassica* line; and obtaining seeds. Such mutant *Brassica* lines include BnCL120C7, a sample of said seed having been deposited under ATCC Accession No. PTA-9278; BnCL131A1, a sample of said seed having been deposited under ATCC Accession No. PTA-9279; BnCL140B3, a sample of said seed having been deposited under ATCC Accession No. PTA-9402; and BnCL140C7, a sample of said seed having been deposited under ATCC Accession No. PTA-9403.

Also provided are seeds of mutant *Brassica* plant lines designated BnCL120C7, a sample of said seed having been deposited under ATCC Accession No. PTA-9278; BnCL131A1, a sample of said seed having been deposited under ATCC Accession No. PTA-9279; BnCL140B3, a sample of said seed having been deposited under ATCC Accession No. PTA-9402; BnCL140C7, a sample of said seed having been deposited under ATCC Accession No. PTA-9403; PM1PM2/BnCL131A1, a sample of said seed having been deposited under ATCC Accession No. PTA-10321.

The present disclosure also provides isolated nucleic acid molecules encoding a *Brassica* acetohydroxyacid synthase large subunit (AHASL) protein having a threonine at a position corresponding to position 122 of SEQ ID NO: 23.

The present disclosure further provides *Brassica* plants comprising a mutated nucleic acid molecule encoding a *Brassica* acetohydroxyacid synthase large subunit (AHASL) protein having a threonine at a position corresponding to position 122 of SEQ ID NO: 23.

In addition, the present disclosure provides *Brassica* seed capable of producing a *Brassica* plant comprising a mutated nucleic acid molecule encoding a *Brassica* acetohydroxyacid synthase large subunit (AHASL) protein having a threonine at a position corresponding to position 122 of SEQ ID NO: 23.

The present disclosure further provides methods of breeding a *Brassica* plant, wherein the method comprises: crossing a first *Brassica* line with a second *Brassica* line, wherein the first *Brassica* line is a *Brassica* plant obtained from growing a seed of mutant line BnCL131A1, a sample of said seed having been deposited under ATCC Accession No. PTA-9279; and obtaining seeds.

The present invention also provides non-transgenic *Brassica* plants comprising a mutagenized nucleic acid molecule encoding an AHAS protein having a mutation at a position corresponding to position A122 of SEQ ID NO: 23 and a mutation at a position corresponding to position S653 of SEQ ID NO: 23.

Also provided are methods for producing a non-transgenic *Brassica* plant that is tolerant of at least one AHAS-inhibiting herbicide, comprising: (a) introducing into *Brassica* plant cells a gene repair oligonucleobase with a targeted mutation in the AHAS gene to produce plant cells with a mutant AHAS gene that expresses an AHAS protein that is mutated at one or more amino acid positions, said positions corresponding to a position selected from the group consisting of A122, R199, T203, S653, and G654 of SEQ ID NO: 23; (b) identifying a plant cell having substantially normal growth as compared to a corresponding wild-type plant cell in the presence of an AHAS-inhibiting herbicide; and (c) regenerating a non-transgenic herbicide tolerant *Brassica* plant having a mutated AHAS gene from said plant cell.

Further provided are methods of producing an F₁ hybrid *Brassica* seed comprising, crossing a first *Brassica* plant having a mutagenized nucleic acid molecule encoding an herbicide tolerant *Brassica* acetohydroxyacid synthase large subunit (AHASL) protein having a mutation at a position corresponding to position A122 of SEQ ID NO: 23 and a mutation at a position corresponding to position S653 of SEQ ID NO: 23, with a second *Brassica* plant; and obtaining seeds.

Also provided are plant cells comprising a mutagenized nucleic acid molecule encoding an AHAS protein having a mutation at a position corresponding to position A122 of SEQ ID NO: 23 and a mutation at a position corresponding to position S653 of SEQ ID NO: 23.

Also provided are methods for increasing the herbicide-resistance of a plant comprising crossing a first *Brassica* plant to a second *Brassica* plant, wherein the first *Brassica* plant comprises in its genome at least one copy of a mutagenized acetohydroxyacid synthase large subunit (AHASL) encoding nucleic acid molecule that encodes an herbicide resistant AHASL protein having a mutation at a position corresponding to position A122 of SEQ ID NO: 23 and a mutation at a position corresponding to position S653 of SEQ ID NO: 23; and obtaining seeds resulting from the cross.

In addition, *Brassica* plants are provided that comprise a mutagenized nucleic acid molecule encoding an herbicide tolerant *Brassica* AHASL protein comprising a mutation at a position corresponding to position A122 of SEQ ID NO: 23 and a mutation at a position corresponding to position S653 of SEQ ID NO: 23 and at least one additional nucleic acid molecule encoding a protein of interest.

Further provided are methods of controlling weeds in the vicinity of a *Brassica* plant, the method comprising applying an effective amount of at least one AHAS-inhibiting herbicide to the weeds and to the *Brassica* plant, wherein the *Brassica* plant comprises in its genome at least one copy of a first AHASL nucleic acid molecule that encodes an herbicide resistant AHASL protein having a mutation at amino acid position corresponding to A122 and S653 of SEQ ID NO: 23, and at least one second AHASL nucleic acid molecule that encodes a second herbicide resistant AHASL protein having a mutation selected from the group consisting of A122T, P197S, A205V, W574L, S653N, and combinations thereof.

### BRIEF DESCRIPTION THE DRAWINGS

Figure 1 provides a schematic representation of AHAS activity in *Brassica* seedling extracts.
Figure 2 provides the results of herbicide spray analyses of wild-type *Brassica* plants and an example of a *Brassica* plant containing a mutated nucleic acid molecule of the present invention.
Figure 3 provides a nucleic acid sequence alignment between nucleic acid molecules of the present invention (SEQ ID NOS 11-16, respectively, in order of appearance).
Figure 4 provides an amino acid sequence alignment between AHAS proteins of the present invention (SEQ ID NOS 17-21, respectively, in order of appearance).
Figure 5 provides the nucleic acid and amino acid sequence of the *Arabidopsis thaliana* AHASL protein having the amino acid sequence set forth in Figure 5. DNA sequence disclosed as SEQ ID NO: 22 and amino acid sequence disclosed as SEQ ID NO: 23.

### DETAILED DESCRIPTION

The present disclosure relates to isolated nucleotides encoding mutant acetohydroxyacid synthase (AHAS) proteins from AHAS-inhibiting herbicide resistant plants and methods related thereto. In one embodiment, the isolated nucleic acid molecules encode AHAS proteins having one or more amino acid substitutions with respect to the wild-type (i.e. non-mutated) *Brassica napus* AHAS sequence. The disclosure also provides recombinant vectors comprising such nucleic acid molecules, as well as transgenic plants containing such vectors and nucleic acid molecules.

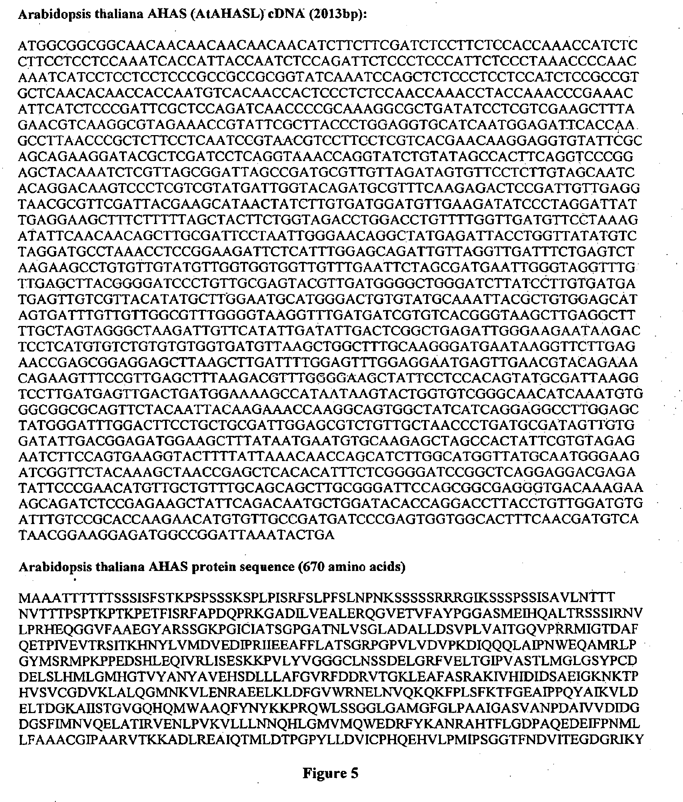

The present disclosure also provides non-transgenic plants comprising a mutated AHAS coding sequence, for example, a mutagenized AHAS III coding sequence, having two or more amino acid substitutions with respect to the wild-type sequence. The mutated AHAS coding sequence may have an amino acid substitution at a position corresponding to position 122 and at a position corresponding to position 653 in the *Arabidopsis* AHAS protein sequence (Figure 5; SEQ ID NO: 23). Also provided are methods of preparing such plants, and breeding methods using such plants.

In some embodiments, the plants, seeds, methods, and compositions of the present invention employ the use of nucleic acid molecules that encode AHAS proteins having mutations that result in the expression of AHAS proteins having an alanine-to-threonine substitution at a position corresponding to position 122 and a serine-to-asparagine substitution at a position corresponding to position 653 (also sometimes referred to herein as CLB-1). In other embodiments, the plants, seeds, methods, and compositions of the present invention employ the use of nucleic acid molecules that encode AHAS proteins having mutations that result in the expression of AHAS proteins having an amino acid selected from arginine, glutamine, phenylalanine, tyrosine, tryptophan, lysine, glycine, histidine, serine, proline, glutamic acid, aspartic acid, cysteine, methionine, leucine, asparagine, isoleucine, and valine substituted at a position corresponding to position 122 and an amino acid selected from arginine, glutamine, phenylalanine, tyrosine, tryptophan, lysine, glycine, histidine, alanine, proline, glutamic acid, aspartic acid, cysteine, methionine, leucine, threonine, isoleucine, and valine substituted at a position corresponding to position 653.

Also provided are methods for controlling weeds using the transgenic and non-transgenic plants containing the mutated AHAS sequences as provided herein.

Further, the applicatiori provides herbicide resistant *Brassica* lines referred to herein as BnCL120C7, BnCL131A1, BnCL140B3, BnCL140C7, as well as seeds, progeny, and derivatives thereof, that was produced using the mutagenesis methods as described in detail below.

The present invention also provides for an enhanced herbicide-tolerance and methods for obtaining enhanced herbicide tolerance which is achieved when combining AHAS mutations in a single AHAS coding sequence. In one example, plants combining mutations corresponding to positions A122 and S653 of the Arabidopsis AHAS protein sequence (Figure 5; SEQ ID NO: 23) demonstrate such enhanced herbicide tolerance. The resulting herbicide tolerance is significantly enhanced, for example, having a synergistic effect, over that level of tolerance which is observed when the tolerance levels obtained in plants containing either mutations corresponding to positions A122 or S653, individually, are added together.

Prior to describing the invention in further detail, the following terms will first be defined.

### Definitions

An "herbicide-tolerant" or "herbicide-resistant" plant refers to a plant that is tolerant or resistant to at least one AHAS-inhibiting herbicide at a level that would normally kill, or inhibit the growth of, a normal or wild-type plant lacking a mutated AHAS nucleic acid molecule. By "herbicide-resistant AHAS nucleic acid molecule" is intended a nucleic acid molecule comprising one or more mutations that results in one or more amino acid substitutions relative to the non-mutated AHAS protein, where the mutations result in the expression of an herbicide-resistant AHAS protein. By "herbicide-tolerant AHAS protein" or "herbicide-resistant AHAS protein", it is intended that such an AHAS protein displays higher AHAS activity, relative to the AHAS activity of a wild-type AHAS protein, when in the presence of at least one herbicide that is known to interfere with AHAS activity and at a concentration or level of the herbicide that is to known to inhibit the AHAS activity of the wild-type AHAS protein. Furthermore, the AHAS activity of such an herbicide-tolerant or herbicide-resistant AHAS protein may be referred to herein as "herbicide-tolerant" or "herbicide-resistant" AHAS activity.

For the present invention, the terms "herbicide-tolerant" and "herbicide-resistant" are used interchangeably and are intended to have an equivalent meaning and an equivalent scope. Similarly, the terms "herbicide-tolerance" and "herbicide-resistance" are used interchangeably and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively. "Herbicide resistance" or "herbicide tolerance" can be measured by comparison of AHAS activity obtained from cell extracts from plants containing the mutagenized AHAS sequence and from plants lacking the mutagenized AHAS sequence in the presence of an AHAS inhibitor, such as imazamox, using the methods disclosed in Singh, et al. Anal. Biochem., (1988), 171: 173-179. In one embodiment, resistant or tolerant plants demonstrate greater than 25% uninhibition using the methods disclosed in Singh et al (1988) when assayed using 100 µM imazamox.

An "isolated" or "purified" nucleic acid molecule or protein, or biologically active portion thereof, is substantially or essentially free from components that normally accompany or interact with the nucleic acid molecule or protein as found in its naturally occurring environment. Thus, an isolated or purified nucleic acid molecule or protein is substantially free of other cellular material or culture medium when produced by recombinant techniques, or substantially free of chemical precursors or other chemicals when chemically synthesized. In one embodiment, an "isolated" nucleic acid is substantially free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. In some embodiments, the isolated nucleic acid molecule is flanked by its native genomic sequences that control its expression in the cell, for example, the native promoter, or native 3' untranslated region. A protein that is substantially free of cellular material includes preparations of protein having less than about 30%, 20%, 10%, 5%, or 1% (by dry weight) of contaminating protein. When the protein of the invention or biologically active portion thereof is recombinantly produced, preferably culture medium represents less than about 30%, 20%, 10%, 5%, or 1% (by dry weight) of chemical precursors or non-protein-of-interest chemicals.

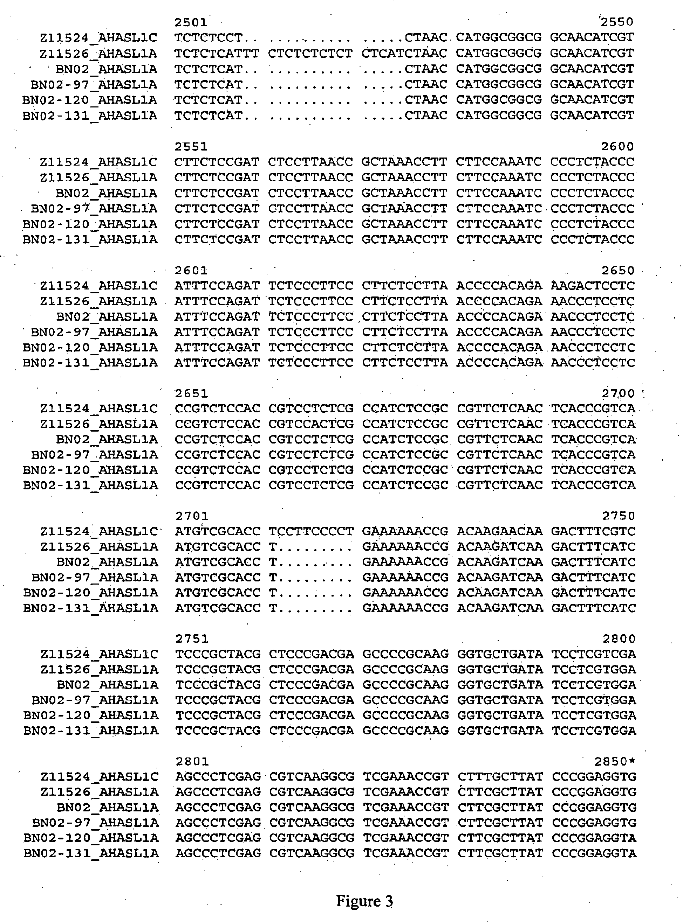

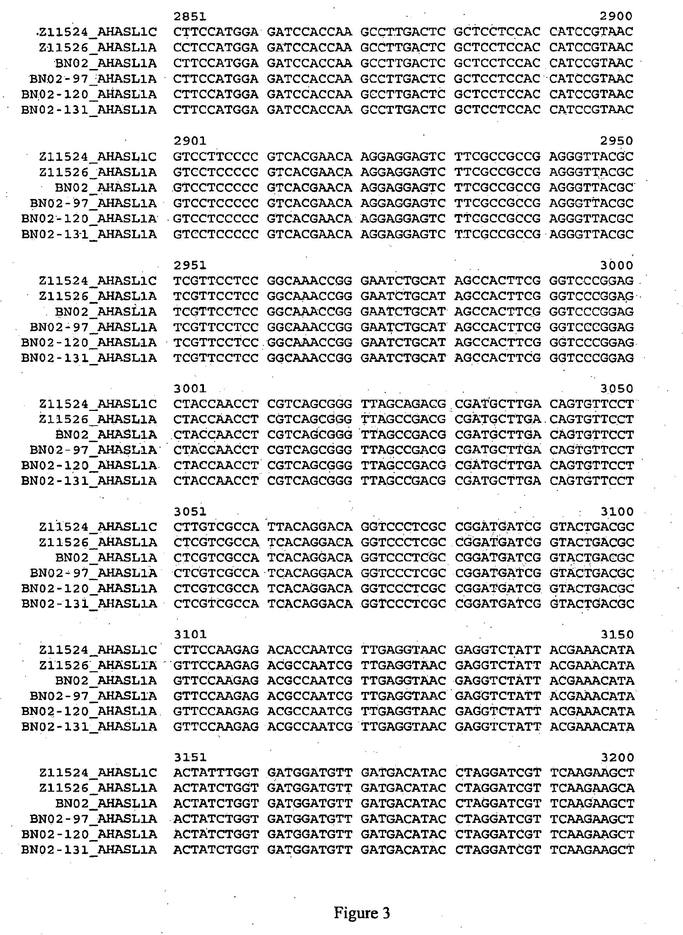

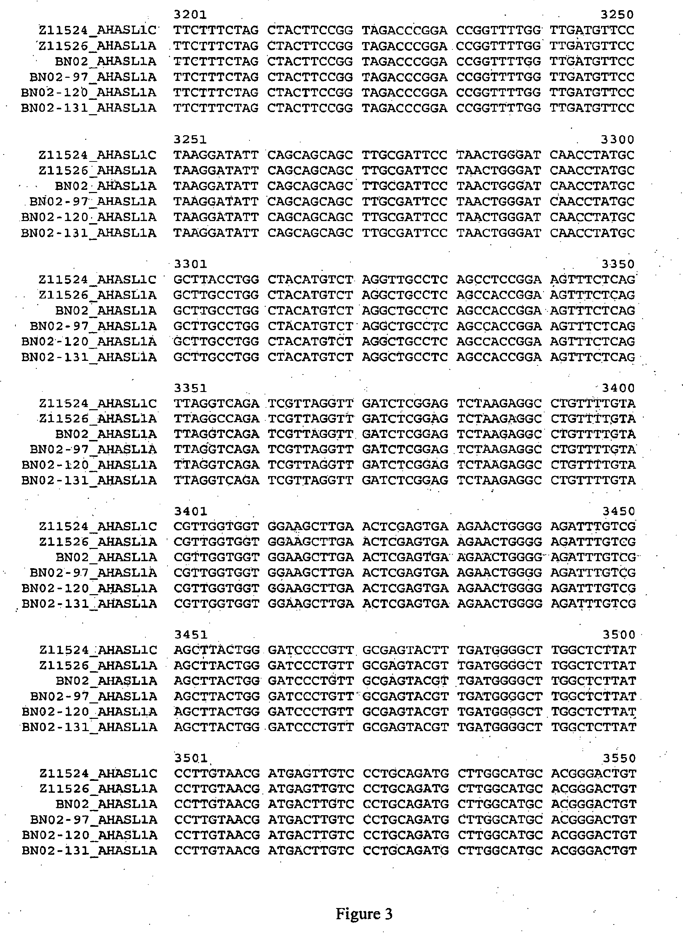

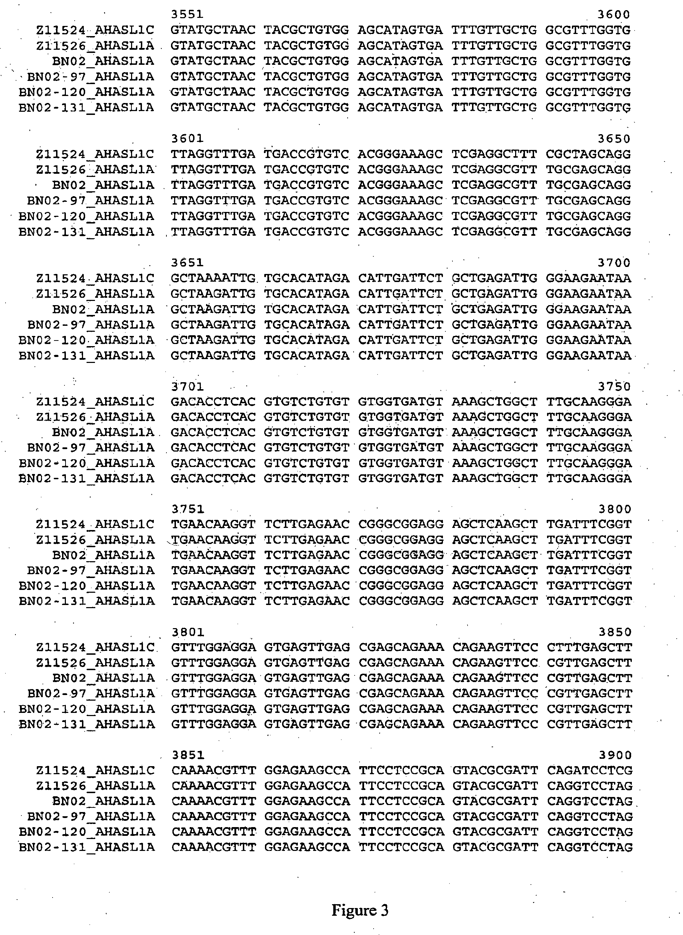

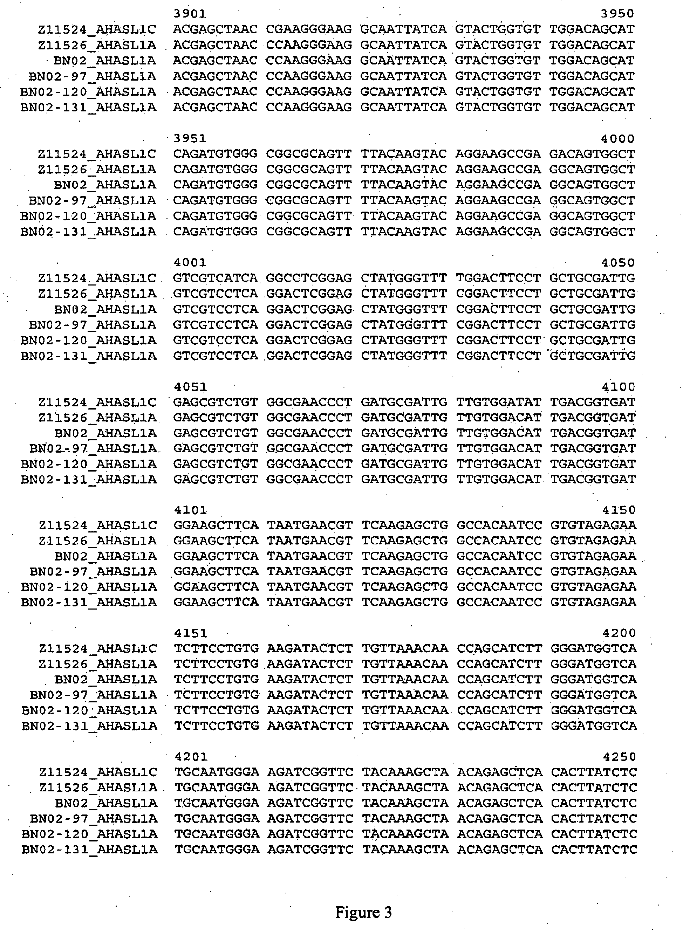

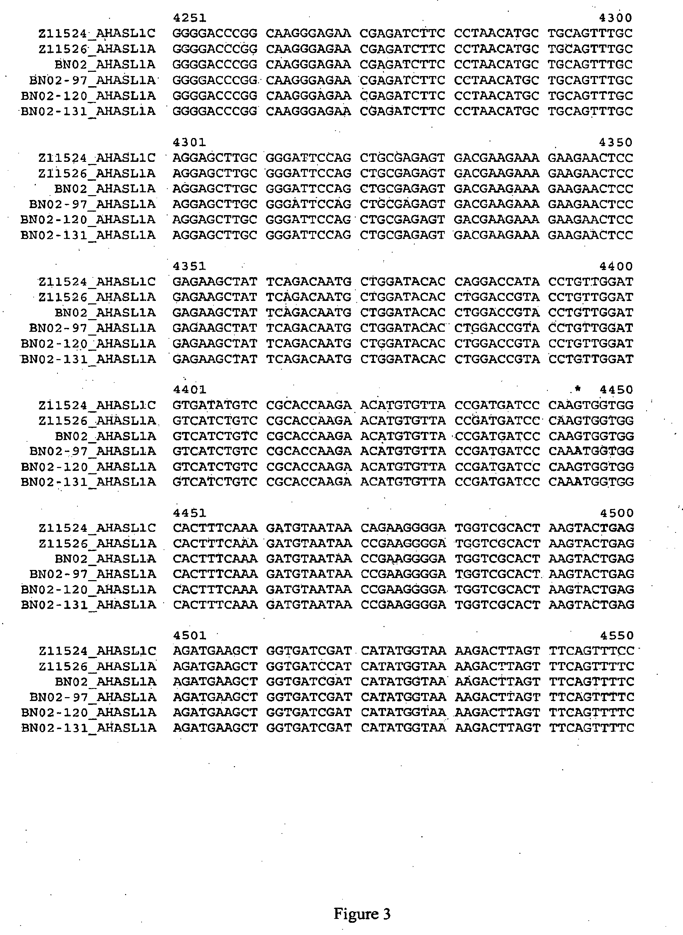

The term "wild-type" is used to refer to a nucleic acid molecule or protein that can be found in nature as distinct from being artificially produced or mutated by man. In one embodiment, a "wild-type" AHAS nucleic acid sequence refers to the nucleic acid sequence of BN-02 in Figure 3. Similarly, a wild-type *Brassica* plant refers to a *Brassica* plant having the nucleic acid sequence of BN-02 in Figure 3. The term is also used to refer to plants containing the AHAS nucleic acid sequence of BN-02 in Figure 3. The use of the term "wild-type" is not intended to necessarily imply that a plant, plant tissue, plant cell, or other host cell lacks recombinant DNA in its genome, and/or does not possess herbicide resistant characteristics that are different from those disclosed herein.

A "mutated" molecule or a "mutant molecule" or a "mutagenized" molecule, such as a nucleic acid molecule or an amino acid molecule, refers to a nucleic acid molecule or polypeptide having one or more nucleic acid or amino acid substitutions, deletions, or transversions compared to the nucleic acid or amino acid at an equivalent position a non-mutated, or wild-type, molecule. The terms refer to a nucleic acid molecule or polypeptide molecule that is modified from its native or wild type form as the result of deliberate human manipulation, and does not include natural sequences.

As used herein, the terms "synergy," "synergistic," and derivations thereof, such as in a "synergistic effect" refer to circumstances under which the biological activity of a combination of two or more mutated AHAS polypeptides, and/or a combination of mutations in a single coding sequence, such as an AHAS nucleic acid molecule encoding and AHAS polypeptide having mutations at positions corresponding to positions A122 and S653, is at least 10% greater than the sum of the biological activities of AHAS polypeptides having the individual mutations. Biological activity for AHAS polypeptides may be measured by comparing injury rates of plants containing the mutagenized molecules of the present invention to the combined injury rates of plants containing the respective mutations 15 days after treatment with an AHAS-inhibiting herbicide, such as imazamox.

An "equivalent position" or "corresponding position" refers a position that is within the same conserved region as a reference amino acid position. For example, with regard to an AHAS protein sequence, amino acid positions disclosed herein correspond to amino acid positions in the *Arabidopsis thaliana* AHASL protein having the amino acid sequence set forth in Figure 5 (Figure 5; SEQ ID NO: 23). Unless otherwise indicated herein, particular amino acid positions refer to the corresponding position of that amino acid in the full-length *A. thaliana* AHASL amino acid sequence set forth in Figure 5 (SEQ ID NO: 23). Furthermore, the actual amino acid positions can vary depending on whether amino acids are added to or removed from, for example, the N-terminal end of an amino acid sequence, however, the equivalent position could still be determined through alignment with a reference sequence, such as Figure 5 (SEQ ID NO: 23). Thus, the invention encompasses the amino substitutions at the recited position or equivalent position (e.g., "amino acid position 653 or equivalent position").

A "fragment" refers to a portion of the nucleotide sequence encoding an AHASL protein or a portion of the amino acid sequence of the AHAS protein of the invention. A fragment of an AHASL nucleotide sequence of the invention may encode a biologically active portion of an AHASL protein, or it may be a fragment that can be used as a hybridization probe or PCR primer using methods disclosed below. A fragment of an AHAS polypeptide may encompass a biologically active fragment of the AHAS protein.

The term "biologically active fragments and variants" refers to fragments and variants of the exemplified nucleic acid molecules and polypeptides that comprise or encode AHAS activity.

The term "regulatory element" as used herein refers to a nucleic acid that is capable of regulating the transcription and/or translation of an operably linked nucleic acid. Regulatory elements include, but are not limited to, promoters, enhancers, introns, 5' UTRs, and 3' UTRs. The term "operably linked" refers to a functional linkage between a regulatory element and a second nucleic acid, where the regulatory element sequence is involved in the control of the expression of the second nucleic acid sequence, for example initiates and/or mediates transcription and/or translation of the DNA sequence corresponding to the second sequence. Generally, operably linked means that the nucleic acid sequences being linked are contiguous and, where necessary to join two protein coding regions, contiguous and in the same reading frame.

As used herein, the term "transgenic" refers to any cell, tissue, plant, plant cell, callus, plant tissue, or plant part that contains all or part of at least one recombinant nucleic acid molecule.

### Nucleic Acid Molecules

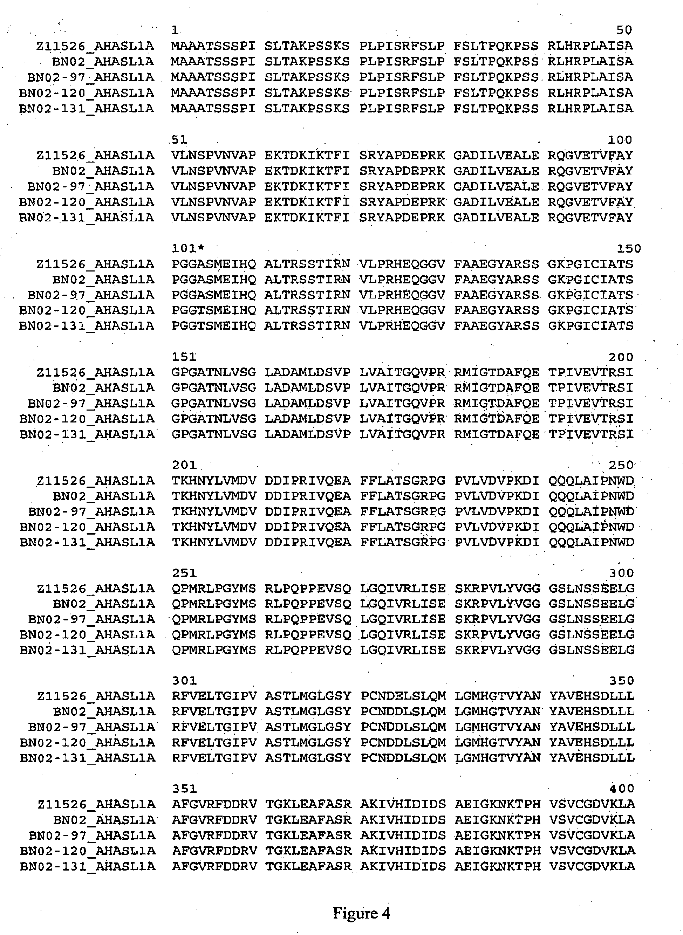

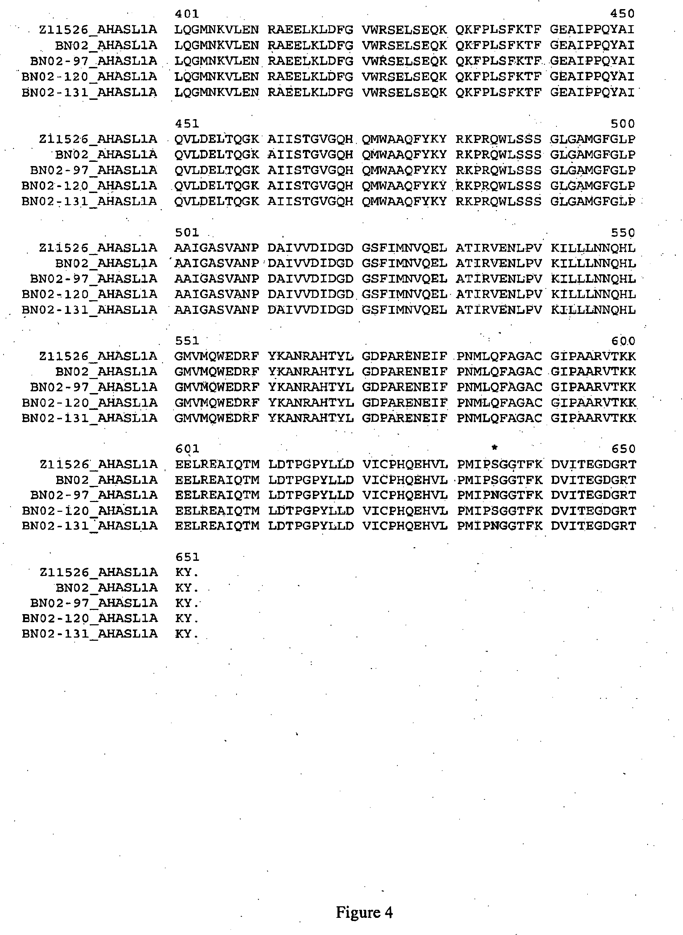

The invention relates, in one aspect, to isolated nucleic acid molecules related to acetohydroxyacid synthase large subunit (AHASL) sequences that comprise nucleic acid sequences having mutations relative to the wild type (i.e. natural) nucleic acid sequence from which it is derived, as well as to fragments of such sequences comprising such mutations. Such isolated nucleic acid sequences include those of Figures 3 and 4. In another aspect, the disclosure relates to nucleic acid molecules comprising nucleotide sequences that encode AHASL proteins having mutations in relation to the wild-type protein that are capable of providing tolerance, or enhanced levels of resistance, to AHAS-inhibiting herbicides and to such mutant AHASL proteins. In one embodiment, the AHASL coding sequence is a mutated *Brassica* AHASL coding sequence, for example the *Brassica napus* AHAS III coding sequence that contains two mutations relative to the wild type sequence. In another embodiment, the invention provides the isolation and nucleotide sequence of a nucleic acid molecule encoding an herbicide-resistant *Brassica* AHASL III protein from an herbicide-resistant *Brassica* plant that was produced by mutagenesis of wild-type *Brassica* plants.

The nucleic acid molecules can be derived from any source, such as plant or bacterial sources. In one embodiment, the nucleic acid molecules are derived from a plant source, such as a *Brassica* plant. Nucleic acid molecules derived from a *Brassica* plant can be derived from any *Brassica* species, including *B. napus, B. rapa,* or *B. juncea.* Such nucleic acid molecules can be derived from any AHAS coding sequences, such as AHAS I, AHAS II, or AHAS III. In one embodiment, the nucleic acid molecules comprise mutations in the nucleic acid sequence at positions that correspond to codon sequences at amino acid positions 122 (e.g. GCT -> ACT) and/or 653 (e.g. AGT -> AAT) from the ATG start codon of the AHASL coding sequence. However, any mutation can be produced at positions corresponding to amino acid positions 122 and 653 within the scope of the present disclosure.

Furthermore, the mutations at positions 122 and 653 of the AHAS sequence can result in the expression of an AHAS protein with any amino acid other than alanine at a position corresponding to position 122 or any amino acid other than serine at a position corresponding to position 653. In one embodiment, the mutations at positions corresponding to positions 122 and 653 can be conservative amino acid substitutions, while in other embodiments, the substitutions can be conservative substitutions of threonine or asparagine at positions 122 and 653, respectively. In another embodiment, the mutations at a position corresponding to position 122 result in the expression of an amino acid at that position selected from the group consisting of threonine and valine. In another embodiment, the mutations at a position corresponding to position 653 result in the expression of an asparagine amino acid. In one embodiment, the AHAS proteins encoded by the nucleic acid molecules of the invention comprise a serine-to-asparagine substitution at a position corresponding to position 653 (e.g. S653N) of the AHASL gene (based on the *Arabidopsis thaliana* numbering) and an alanine-to-threonine substitution at a position corresponding to position 122 (e.g. A122T). However, any amino acid substitution, including conservative amino acid substitutions, may be made at these positions within the scope of the present disclosure. The invention further discloses the isolation and nucleotide sequence of a nucleic acid molecule encoding a wild-type *Brassica* AHASL protein.

In some embodiments, the nucleic acid molecules of the present invention encode AHAS proteins having mutations that result in the expression of AHAS proteins having an alanine-to-threonine substitution at a position corresponding to position 122 and a serine-to-asparagine substitution at a position corresponding to position 653. In other embodiments, the nucleic acid molecules encode AHAS proteins having mutations that result in the expression of AHAS proteins having an amino acid selected from Arginine, Glutamine, Phenylalanine, Tyrosine, Tryptophan, Lysine, Glycine, Histidine, Serine, Proline, Glutamic Acid, Aspartic Acid, Cysteine, Methionine, Leucine, Asparagine, Isoleucine, and Valine substituted at a position corresponding to position 122 and an amino acid selected from Arginine, Glutamine, Phenylalanine, Tyrosine, Tryptophan, Lysine, Glycine, Histidine, Alanine, Proline, Glutamic Acid, Aspartic Acid, Cysteine, Methionine, Leucine, Threonine, Isoleucine, and Valine substituted at a position corresponding to position 653.

The mutagenized AHAS polypeptide sequences of the present invention may demonstrate resistance or tolerance to any AHAS-inhibiting herbicide. Such mutagenized AHAS polypeptide sequences may be resistant or tolerant to at least one AHAS-inhibiting herbicide. In one embodiment, the mutagenized AHAS polypeptide sequences of the present invention demonstrate resistance or tolerance to an AHAS-inhibiting herbicide selected from the group consisting of imidizolinone herbicides and sulfonurea herbicides. In another embodiment, the mutagenized AHAS polypeptide sequences of the present invention demonstrate resistance or tolerance to imidizolinone herbicides.

In some embodiments, the mutagenized AHAS sequences having mutations at positions corresponding to positions A 122 and S653 provide synergistic herbicide tolerance levels. The levels of herbicide tolerance obtained in plants containing a double mutant AHAS sequence of the present invention are at least 10%, 20%, 30%, 40%, 50%, 60%, 70%, 80% or greater, higher than the additive levels of tolerance observed in plants containing AHAS sequence having the respective individual mutations. Such synergistic herbicide tolerance levels may also be obtained in plants having the mutagenized AHAS sequences having mutations at amino acid positions corresponding to positions A122 and S653 in combination with one or more additional mutagenized or recombinant AHAS sequences encoding for an AHAS-inhibitor tolerance AHAS protein.

Also disclosed are mutated nucleic acid molecules that encode a *B. napus* AHAS III protein having an alanine-to-threonine substitution at a position corresponding to position 122 (A122T). In one embodiment, such nucleic acid molecules also contain a second mutation in the nucleic acid sequence, for example in the nucleic acid molecule encoding a serine-to-asparagine substitution at a position corresponding to position 653 (S653N).

The disclosure also provides isolated nucleic acid molecules having the nucleic acid sequences of BN02-120 or BN02-131 as set forth in Figures 3 and 4, as well as fragments and variants thereof.

The disclosure also provides isolated nucleic acid molecules that encode AHASL proteins from *Brassica,* having one or more nucleic acid substitutions that result in the expression of an AHAS protein having one or more amino acid substitutions. For example, the invention provides isolated nucleic acid molecules comprising: the nucleotide sequence of BN02-120 or BN02-131 as set forth in Figures 3 and 4, nucleotide sequences encoding the AHASL protein comprising the amino acid sequence as identified as BN02-102 or BN02-131 as set forth in Figure 4, the nucleotide sequences as set forth in Figure 3, nucleotide sequences encoding the AHASL protein comprising the amino acid sequence as set forth in BN02-120 or BN02-131 in Figure 4, and fragments and variants of such nucleotide sequences that encode functional AHASL proteins.

The mutagenized AHAS sequences of the present invention include those having any number of mutations in addition to those mutations corresponding to positions 122 and/or 653. For example, the mutagenized AHAS sequence may contain 1, 2, 3, 4, 5, 6, 7, 8, 9, 10 or more additional mutations that may be either silent mutations or provide resistance or tolerance to the same or other classes of AHAS-inhibiting herbicides.

In another embodiment, the present invention provides for isolated nucleic acid molecules comprising nucleotide sequences that encode the amino acid sequences shown in Figures 3 and 4, as well as fragments and variants thereof that encode polypeptides having AHAS activity. Further provided are polypeptides having an amino acid sequence encoded by a nucleic acid molecule described herein, for example the nucleotide sequences set forth in Figures 3 and 4 (identified as BN02-120 or BN02-131), and fragments and variants thereof that encode polypeptides comprising AHAS activity.

The present disclosure also provides AHASL proteins with amino acid substitutions at identified amino acid positions within conserved regions of the *Brassica* AHASL proteins disclosed herein.

The disclosure encompasses isolated or substantially purified nucleic acid or protein com]

The present disclosure provides isolated polypeptides comprising mutated AHAS proteins. The isolated polypeptides comprise an amino acid sequence selected from the group consisting of the amino acid sequence of BN02-120 or BN02-131 as set forth in Figure 4, the amino acid sequence encoded by the nucleotide sequence BN02-120 or BN02-131 as set forth in Figure 3, the amino acid sequence, and functional fragments and variants of said amino acid sequences that encode an AHAS polypeptide comprising AHAS activity.

The disclosed nucleic acid molecules can be used in nucleic acid constructs for the transformation of plants, for example, crop plants, such as *Brassica* plants. In one embodiment, such nucleic acid constructs containing the nucleic acid molecules of the present disclosure can be used to produce transgenic plants to provide for resistance to herbicides, such as herbicides that are known to inhibit AHAS activity, such as imidazolinone herbicides. The nucleic acid constructs can be used in expression cassettes, expression vectors, transformation vectors, plasmids and the like. The transgenic plants obtained following transformation with such constructs demonstrate increased resistance to AHAS-inhibiting herbicides such as, for example, imidazolinone and sulfonylurea herbicides.

Thus, the present disclosure encompasses AHASL nucleic acid molecules and fragments and variants thereof. Nucleic acid molecules that are fragments of these nucleotide sequences are also encompassed by the present invention.

The fragment comprises at least one mutated sequence in comparison to the corresponding sequence from the wild-type sequence. A biologically active portion of an AHAS protein can be prepared by isolating a portion of one of the AHAS nucleotide sequences of the invention, expressing the encoded portion of the AHAS protein (e.g., by recombinant expression *in vitro*), and assessing the activity of the encoded portion of the AHAS protein. Nucleic acid molecules that are fragments of an AHAS nucleotide sequence comprise at least about 15, 20, 50, 75, 100, 200, 300, 350,400,450, 500, 550, 600, 650, 700, 750, 800, 850, or 900 nucleotides, or up to the number of nucleotides present in a full-length nucleotide sequence disclosed herein depending upon the intended use.

A fragment of an AHAS nucleotide sequence that encodes a biologically active portion of an AHAS protein will encode at least about 15, 25, 30, 50, 75, 100, 125, 150, 175, 200, 225, or 250 contiguous amino acids, or up to the total number of amino acids present in a full-length AHAS protein of the invention. Fragments of an AHAS nucleotide sequence that are useful as hybridization probes for PCR primers generally need not encode a biologically active portion of an AHAS protein. Fragments of an AHAS sequence encompass one or more mutations disclosed herein.

Nucleic acid molecules that are variants of the nucleotide sequences are also disclosed "Variants" of the AHAS nucleotide sequences of the invention include those sequences that encode the AHAS proteins disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHAS protein disclosed in the present invention as discussed below. Generally, nucleotide sequence variants of the invention will have at least about 75%, 80%, 85%, 90%, 91%, 92%, 93%, 94%, 95%, 96%, 97%, 98%, or 99% identity to a particular nucleotide sequence disclosed herein. A variant AHAS nucleotide sequence will encode an AHAS protein, respectively, that has an amino acid sequence having at least about 75%, 80%, 85%, 90%, 91%, 92%, 93%, 94%, 95%, 96%, 97%, 98%, or 99% identity to the amino acid sequence of an AHAS protein disclosed herein.

In addition, the skilled artisan will further appreciate that changes can be introduced by mutation into the nucleotide sequences of the invention thereby leading to changes in the amino acid sequence of the encoded AHAS proteins without altering the biological activity of the AHAS proteins. Thus, an isolated nucleic acid molecule encoding an AHAS protein having a sequence that differs from that of BN02-120 or BN02-131 as set forth in Figures 3 can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis and those described herein. Such variant nucleotide sequences are also disclosed.

For example, in one embodiment, conservative amino acid substitutions may be made at one or more predicted, nonessential amino acid residues. A "nonessential" amino acid residue is a residue that can be altered from the wild-type sequence of an AHAS protein (e.g., the sequence of Figure 5 (SEQ ID NO: 23)) without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Such substitutions would not be made for conserved amino acid residues, or for amino acid residues residing within a conserved motif.

The proteins of the invention may be altered in various ways including amino acid substitutions, deletions, and insertions. Methods for such manipulations are generally known in the art. For example, amino acid sequence variants of the AHAS proteins can be prepared by mutations in the DNA. Methods for mutagenesis and nucleotide sequence alterations are well known in the art. See, for example, Kunkel (1985) Proc. Natl. Acad. Sci. USA 82:488-492; Kunkel et al. (1987) Methods in Enzymol. 154:367-382; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be preferable.

Alternatively, variant AHAS nucleotide sequences can be made by introducing mutations randomly along all or part of an AHAS coding sequence, such as by saturation mutagenesis, and the resultant mutants can be screened for AHAS activity to identify mutants that retain AHAS activity, including herbicide-resistant AHAS activity. Following mutagenesis, the encoded protein can be expressed recombinantly, and the activity of the protein can be determined using standard assay techniques.

Thus, the nucleotide sequences include the sequences disclosed herein as well as fragments and variants thereof. The AHASL nucleotide sequences of the invention, and fragments and variants thereof, can be used as probes and/or primers. Such probes can be used to detect transcripts or genomic sequences encoding the same or identical proteins.

In this manner, methods such as PCR, hybridization, and the like can be used to identify such sequences having substantial identity to the sequences of the invention. *See,* for example, Sambrook et al. (1989) Molecular Cloning: Laboratory Manual (2nd ed., Cold Spring Harbor Laboratory Press, Plainview, NY) and Innis, et al. (1990) PCR Protocols: A Guide to Methods and Applications (Academic Press, NY). *AHASL* nucleotide sequences isolated based on their sequence identity to the AHAS nucleotide sequences set forth herein or to fragments and variants thereof are encompassed by the present invention.

In hybridization methods, all or part of a known AHAS nucleotide sequence can be used to screen cDNA or genomic libraries. Methods for construction of such cDNA and genomic libraries are generally known in the art and are disclosed in Sambrook et al. (1989) Molecular Cloning: A Laboratory Manual (2nd ed., Cold Spring Harbor Laboratory Press, Plainview, NY). Hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known AHAS nucleotide sequence disclosed herein. Degenerate primers designed on the basis of conserved nucleotides or amino acid residues in a known AHAS nucleotide sequence or encoded amino acid sequence can additionally be used. The probe typically comprises a region of nucleotide sequence that hybridizes under stringent conditions to at least about 12, preferably about 25, more preferably about 50, 75, 100, 125, 150, 175, 200, 250, 300, 350, 400, 500, 600, 700, 800, or 900 consecutive nucleotides of an AHAS nucleotide sequence
or a fragment or variant thereof. Preparation of probes for hybridization is generally known in the art and is disclosed in Sambrook et al. (1989) Molecular Cloning: A Laboratory Manual (2d ed., Cold Spring Harbor Laboratory Press, Plainview, New York).

For example, the entire mutated AHAS nucleic acid sequence disclosed herein, or one or more portions thereof, may be used as a probe capable of specifically hybridizing to corresponding AHAS sequences and messenger RNAs. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances.

Stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1 % to about 1% SDS. The duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with >90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2nd ed., Cold Spring Harbor Laboratory Press, Plainview, New York).

The nucleic acid molecules and proteins encompass nucleic acid molecules and proteins comprising a nucleotide or an amino acid sequence that is sufficiently identical to the nucleotide sequence of BN02-120 or BN02-131 as set forth in Figures 3 and 4. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences that contain a common structural domain having at least about 45%, 55%, or 65% identity, preferably 75% identity, more preferably 85%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences (*i.e.,* percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the NBLAST and XBLAST programs of Altschul et al. (1990) J. Mol. Biol. 215:403. BLAST nucleotide searches can be performed with the NBLAST program, score = 100, wordlength = 12, to obtain nucleotide sequences homologous to the nucleic acid molecules of the invention. BLAST protein searches can be performed with the XBLAST program, score = 50, wordlength = 3, to obtain amino acid sequences homologous to protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. *See* Altschul *et al.* (1997) *supra.* When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarity values provided herein refer to the value obtained using the full-length sequences of the invention and using multiple alignment by mean of the algorithm Clustal W (Nucleic Acid Research, 22(22):4673-4680, 1994) using the program AlignX included in the software package Vector NTI Advance 10 (Invitrogen, Carlsbad, CA, USA) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Advance 10.

The mutant AHASL proteins encompass the disclosed proteins as well as variations and modified forms thereof. Such variants will continue to possess the desired AHAS mutant activity. Such mutations in the nucleic acid molecule encoding the variant protein does not place the sequence out of reading frame and preferably will not create complementary regions that could produce secondary mRNA structure. See, EP Patent Application Publication No. 75,444.

In one embodiment the sequences of the present invention encode proteins having AHAS activity. Such activity can be evaluated by AHAS activity screens, such as those disclosed in Singh et al. (1988) Anal. Biochem. 171:173-179

Variant nucleotide sequences and proteins also encompass sequences and proteins derived from a mutagenic and recombinogenic procedure such as DNA shuffling. With such a procedure, one or more different AHASL coding sequences can be manipulated to create a new AHASL protein possessing the desired properties. In this manner, libraries of recombinant nucleic acids are generated from a population of related sequence nucleic acids comprising sequence regions that have substantial sequence identity and can be homologously recombined *in vitro* or *in vivo.* For example, using this approach, sequence motifs encoding a domain of interest may be shuffled between the AHASL gene of the invention and other known *AHASL* genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

The nucleotide sequences of the invention can be used to isolate corresponding sequences from other organisms, particularly other plants, more particularly other dicots. In this manner, methods such as PCR, hybridization, and the like can be used to identify such sequences based on their sequence homology to the sequences set forth herein. Sequences isolated based on their sequence identity to the entire AHASL sequences set forth herein or to fragments thereof are encompassed by the present invention. Thus, isolated sequences that encode for an AHASL protein and which hybridize under stringent conditions to the sequence disclosed herein, or to fragments thereof, are encompassed by the present invention.

In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook et al. (1989) Molecular Cloning: A Laboratory Manual (2nd ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

### Constructs

The nucleic acid molecules of the present invention can be used in the production of recombinant nucleic acid constructs. In one embodiment, the nucleic acid molecules of the invention can be used in the preparation of nucleic acid constructs, for example, expression cassettes for expression in the plant of interest.

Expression cassettes may include regulatory sequences operably linked to the AHASL nucleic acid sequence of the invention. The cassette may additionally contain at least one additional gene to be co-transformed into the organism. Alternatively, the additional gene(s) can be provided on multiple expression cassettes.

The nucleic acid constructs may be provided with a plurality of restriction sites for insertion of the AHASL nucleic acid sequence to be under the transcriptional regulation of the regulatory regions. The nucleic acid constructs may additionally contain nucleic acid molecules encoding for selectable marker genes.

In one embodiment, the expression cassette includes in the 5'-3' direction of transcription, a transcriptional and translational initiation region (e.g., a promoter), an AHASL nucleic acid sequence of the invention, and a transcriptional and translational termination region (e.g., termination region) functional in plants.

Any promoter can be used in the production of the nucleic acid constructs. The promoter may be native or analogous, or foreign or heterologous, to the plant host and/or to the AHASL nucleic acid sequence of the invention. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the AHASL nucleic acid sequence of the invention, it is intended that the promoter is not the native or naturally occurring promoter for the operably linked AHASL nucleic acid sequence of the invention. As used herein, a chimeric gene comprises a coding sequence operably linked to a transcription initiation region that is heterologous to the coding sequence.

While it may be preferable to express the AHASL nucleic acid molecules of the invention using heterologous promoters, the native promoter sequences may be used in the preparation of the constructs. Such constructs would change expression levels of the AHASL protein in the plant or plant cell. Thus, the phenotype of the plant or plant cell is altered.

Any promoter can be used in the preparation of constructs to control the expression of the AHAS coding sequence, such as promoters providing for constitutive, tissue-preferred, inducible, or other promoters for expression in plants. Constitutive promoters include, for example, the core promoter of the Rsyn7 promoter and other constitutive promoters disclosed in WO 99/43 838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to direct AHASL expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 1 12(3):1331-1341; Van Camp et al. (1996) Plant Physiol. 1 12(2):525-535; Canevascini et al. (1996) Plant Physiol. 112(2): 513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586- 9590; and Guevara-Garcia et al. (1993) Plant J. 4(3):495-505.

The nucleic acid constructs may also comprise transcription termination regions. Where transcription terminations regions are used, any termination region may be used in the preparation of the nucleic acid constructs. For example, the termination region may be native to the transcriptional initiation region, may be native to the operably linked AHASL sequence of interest, may be native to the plant host, or may be derived from another source (i.e., foreign or heterologous to the promoter, the AHASL nucleic acid molecule of interest, the plant host, or any combination thereof). Examples of termination regions that are available for use in the constructs of the present invention include those from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

The nucleic acids may be optimized for increased expression in the transformed plant. That is, the nucleic acids encoding the mutant AHASL proteins can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

In addition, other sequence modifications can be made to the nucleic acid sequences disclosed herein. For example, additional sequence modifications are known to enhance gene expression in a cellular host. These include elimination of sequences encoding spurious polyadenylation signals, exon/intron splice site signals, transposon-like repeats, and other such well-characterized sequences that may be deleterious to gene expression. The G-C content of the sequence may also be adjusted to levels average for a target cellular host, as calculated by reference to known genes expressed in the host cell. In addition, the sequence can be modified to avoid predicted hairpin secondary mRNA structures.

Other nucleic acid sequences may also be used in the preparation of the constructs of the present invention, for example to enhance the expression of the AHAS coding sequence. Such nucleic acid sequences include the introns of the maize AdhI, intron1 gene (Callis et al. (1987) Genes and Development 1:1183-1200), and leader sequences, (W-sequence) from the Tobacco Mosaic virus (TMV), Maize Chlorotic Mottle Virus and Alfalfa Mosaic Virus (Gallie et al.(1987) Nucleic Acid Res. 15:8693-8711, and Skuzeski et al. (1990) Plant Mol. Biol. 15:65-79, 1990). The first intron from the shrunken-1 locus of maize has been shown to increase expression of genes in chimeric gene constructs. U.S. Pat. Nos. 5,424,412 and 5,593,874 disclose the use of specific introns in gene expression constructs, and Gallie et al. ((1994) Plant Physiol. 106:929-939) also have shown that introns are useful for regulating gene expression on a tissue specific basis. To further enhance or to optimize AHAS large subunit gene expression, the plant expression vectors of the invention may also contain DNA sequences containing matrix attachment regions (MARs). Plant cells transformed with such modified expression systems, then, may exhibit overexpression or constitutive expression of a nucleotide sequence of the invention.

The nucleic acid constructs of the present invention may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) (Virology 154:9-20), and human immunoglobulin heavy-chain binding protein (BiP) (Macejak et al. (1991)Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the nucleic acid constructs, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

The expression constructs of the present invention can also include nucleic acid sequences capable of directing the expression of the AHAS sequence to the chloroplast. Such nucleic acid sequences include chloroplast targeting sequences that encodes a chloroplast transit peptide to direct the gene product of interest to plant cell chloroplasts. Such transit peptides are known in the art. With respect to chloroplast-targeting sequences, "operably linked" means that the nucleic acid sequence encoding a transit peptide (*i.e.,* the chloroplast-targeting sequence) is linked to the AHASL nucleic acid molecule of the invention such that the two sequences are contiguous and in the same reading frame. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the AHASL proteins of the invention may include a native chloroplast transit peptide, any chloroplast transit peptide known in the art can be fused to the amino acid sequence of a mature AHASL protein of the invention by operably linking a choloroplast-targeting sequence to the 5'-end of a nucleotide sequence encoding a mature AHASL protein of the invention.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5- (enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081- 6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233 :478-481.

The nucleic acid constructs may be prepared to direct the expression of the mutant AHAS coding sequence from the plant cell chloroplast. Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

The nucleic acid constructs can be used to transform plant cells and regenerate transgenic plants comprising the mutant AHAS coding sequences. Numerous plant transformation vectors and methods for transforming plants are available. *See,* for example, U.S. Patent No. 6,753,458, An, G. et al. (1986) Plant Physiol., 81:301-305; Fry, J. et al. (1987) Plant Cell Rep. 6:321-325; Block, M. (1988) Theor. Appl Genet.76:767-774; Hinchee et al. (1990) Stadler. Genet. Symp.203212.203-212; Cousins et al. (1991) Aust. J. Plant Physiol. 18:481-494; Chee, P. P. and Slightom, J. L. (1992) Gene. 118:255-260; Christou et al. (1992) Trends. Biotechnol. 10:239-246; D'Halluin et al. (1992) Bio/Technol. 10:309-3 14; Dhir et al. (1992) Plant Physiol. 99:81-88; Casas et al. (1993) Proc. Nat. Acad Sci. USA 90:11212-11216; Christou, P. (1993) In Vitro Cell. Dev. Biol.-Plant; 29P:1 19-124; Davies, et al. (1993) Plant Cell Rep. 12:180-183; Dong, J. A. and Mc Hughen, A (1993) Plant Sci. 91:139-148; Franklin, C. I. and Trieu, T. N. (1993) Plant. Physiol. 102:167; Golovkin et al. (1993) Plant Sci. 90:41-52; Guo Chin Sci. Bull. 38:2072-2078; Asano, et al. (1994) Plant Cell Rep. 13; Ayeres N. M. and Park, W. D. (1994) Crit. Rev. Plant. Sci. 13:219-239; Barcelo et al. (1994) Plant. J. 5:583-592; Becker, et al. (1994) Plant. J. 5:299-307; Borkowska et al. (1994) Acta. Physiol Plant. 16:225- 230; Christou, P. (1994) Agro. Food. Ind. Hi Tech. 5: 17-27; Eapen et al. (1994) Plant Cell Rep. 13:582-586; Hartman et al. (1994) Bio-Technology 12: 919923; Ritala et al. (1994) Plant. Mol. Biol. 24:317-325; and Wan, Y. C. and Lemaux, P. G. (1994) Plant Physiol. 104:3748. The constructs may also be transformed into plant cells using homologous recombination.

The disclosed constructs comprising the AHASL sequences of the present invention can be used in various methods to produce transgenic host cells, such as bacteria, yeast, and to transform plant cells and in some cases regenerate transgenic plants. For example, methods of producing a transgenic crop plant containing the AHASL mutant coding nucleic acid of the present invention, where expression of the nucleic acid(s) in the plant results in herbicide tolerance as compared to wild-type plants or to known AHAS mutant type plants comprising: (a) introducing into a plant cell an expression vector comprising nucleic acid encoding a mutant AHASL of the present invention, and (b) generating from the plant cell a transgenic plant which is herbicide tolerant.

Plant cells for use in the methods of the present invention include, but are not limited to, protoplasts, gamete producing cells, and cells that regenerate into a whole plant. In many cases, all or part of the recombinant nucleic acid molecule is stably integrated into a chromosome or stable extra-chromosomal element, so that it is passed on to successive generations.

The present invention may be used for transformation of any plant species, including, but not limited to, monocots and dicots. Examples of plant species of interest include, but are not limited to, corn or maize (*Zea mays*)*, Brassica* sp. (e.g., *B. napus, B. rapa, B. juncea*), including those *Brassica* species useful as sources of seed oil, alfalfa (*Medicago sativa*), rice (*Oryza sativa*), rye *(Secale cereale),* sorghum (*Sorghum bicolor, Sorghum vulgare*), millet (e.g., pearl millet (*Pennisetum glaucum),* proso millet (*Panicum miliaceum*), foxtail millet (*Setaria italica),* finger millet (*Eleusine coracana*))*,* sunflower (*Helianthus annuus*), safflower (*Carthamus tinctorius*), wheat (*Triticum aestivum, T. Turgidum ssp. durum*)*,* soybean (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta),* coffee (*Coffea* spp.), coconut *(Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*)*,* sugar beets (*Beta vulgaris*), sugarcane (*Saccharum* spp.), oats (*Avena sativa*), barley (*Hordeum vulgare*), vegetables, ornamentals, and conifers. Preferably, plants of the present invention are crop plants (for example, sunflower, *Brassica sp.,* cotton, sugar, beet, soybean, peanut, alfalfa, safflower, tobacco, corn, rice, wheat, rye, barley triticale, sorghum, millet, etc.).

The mutant AHASL nucleic acid molecules encoding the AHASL polypeptides can also be used as selectable markers, for example, to identify transgenic cells containing introduced nucleic acid molecules encoding the mutant AHASL polypeptides. The disclosure provides a method of identifying or selecting a transformed plant cell, plant tissue, plant or part thereof comprising a) providing a transformed plant cell, plant tissue, plant or part thereof, wherein the transformed plant cell, plant tissue, plant or part thereof comprises an isolated nucleic acid encoding an AHASL mutant polypeptide as described above, where the polypeptide is used as a selection marker, and where the transformed plant cell, plant tissue, plant or part thereof may optionally comprise a further isolated nucleic acid of interest; b) contacting the transformed plant cell, plant tissue, plant or part thereof with at least one AHAS inhibitor or AHAS inhibiting compound; c) determining whether the plant cell, plant tissue, plant or part thereof is affected by the inhibitor or inhibiting compound; and d) identifying or selecting the transformed plant cell, plant tissue, plant or part thereof.

Also disclosed are purified AHASL proteins that contain the mutations described herein, which are useful in molecular modeling studies to design further improvements to herbicide tolerance. Methods of protein purification are well known, and can be readily accomplished using commercially available products or specially designed methods, as set forth for example, in Protein Biotechnology, Walsh and Headon (Wiley, 1994).

The present invention provides plants, plant tissues, plant cells, and host cells that are resistant or tolerant of at least one AHAS-inhibiting herbicide, such as imidazolinone or sulfonylurea herbicides. In some embodiments, the plants, plant tissues, plant cells, and host cells demonstrate enhanced resistance or enhanced tolerance to at least one AHAS-inhibiting herbicide. The term 'enhanced' refers to an increase in the amount of resistance or tolerance above that which is expected. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, microspore, or host cell, but that said amount does not kill or inhibit as severely the growth of the herbicide-resistant plants, plant tissues, plant cells, microspores, and host cells of the present invention. Typically, the effective amount of an herbicide is an amount that is routinely used in agricultural production systems to kill weeds of interest. Such an amount is known to those of ordinary skill in the art, or can be easily determined using methods known in the art. Furthermore, it is recognized that the effective amount of an herbicide in an agricultural production system might be substantially different than an effective amount of an herbicide for a plant culture system such as, for example, the microspore culture system.

The herbicides are those that interfere with the activity of the AHAS enzyme such that AHAS activity is reduced in the presence of the herbicide. Such herbicides may also be referred to herein as "AHAS-inhibiting herbicides" or simply "AHAS inhibitors." As used herein, an "AHAS-inhibiting herbicide" or an "AHAS inhibitor" is not meant to be limited to single herbicide that interferes with the activity of the AHAS enzyme. Thus, unless otherwise stated or evident from the context, an "AHAS-inhibiting herbicide" or an "AHAS inhibitor" can be a one herbicide or a mixture of two, three, four, or more herbicides, each of which interferes with the activity of the AHAS enzyme.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant including, plant calli, plant clumps, plant protoplasts and plant cell tissue cultures from which plants can be regenerated. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, an embryos, an ovule, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant part.

The plants of the present invention include both non-transgenic plants and transgenic plants. By "non-transgenic plant" is intended to mean a plant lacking recombinant DNA in its genome, but containing the mutant nucleic acid molecule in the plant cell genome which has been mutated using mutagenic techniques, such as chemical mutagenesis or by those methods provided herein. Non-transgenic plants do not encompass those plants having mutant sequences as a result of natural processes. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

The present invention also provides AHAS-herbicide resistant plants that contain the nucleic acid molecules disclosed herein. The AHAS-herbicide resistant plants can be non-transgenic or transgenic herbicide-tolerant plants comprising a nucleic acid molecule encoding an AHASL mutant polypeptide as described herein. AHAS-herbicide resistant plants can be produced by cross-pollinating a first plant with a second plant and allowing the pollen acceptor plant (can be either the first or second plant) to produce seed from this cross pollination. Seeds and progeny plants generated therefrom can have the mutation crossed into the genome of the seed and/or progeny plants. The pollen-acceptor plant can be either the first or second plant. The first plant comprises a first nucleic acid molecule encoding at least one AHASL mutant polypeptide as disclosed herein. The second plant can be any compatible plant and may comprise a second nucleic acid molecule encoding the same or different AHASL mutant polypeptide. The first and second AHASL mutant polypeptides may comprise the same or different amino acid substitution(s) relative to a wild-type AHASL polypeptide. Seeds or progeny plants arising from the cross which comprise one nucleic acid molecule encoding the AHASL mutant polypeptide or two nucleic acid molecules encoding the two AHASL mutant polypeptides can be selected.

When the first and second plants are homozygous for the first and second nucleic acid molecules, respectively, each of the resulting progeny plants comprises one copy of each of the first and second nucleic acid molecules and the selection step can be omitted. When at least one of the first and second plants is heterozygous, progeny plants comprising both nucleic acid molecules can be selected, for example, by analyzing the DNA of progeny plants to identify progeny plants comprising both the first and second nucleic acid molecules or by testing the progeny plants for increased herbicide tolerance.

Plants containing the nucleic acid sequences of the present invention can be produced using non-transgenic methods, such as site directed mutagenesis methods including those methods disclosed, for example, in International Publication No. WO 01/15740, or U.S. Patent Nos. 6,271,360, 6,479,292, and 7,094,606. In one embodiment, such methods may involve the use of oligonucleotide-directed gene repair to introduce point mutations into the host cell genome, and can involve the use of single-stranded oligonucleotides, such as gene repair oligonucleobases (See, U.S. Patent Nos. 6,870,075 and 5,565,350). An oligonucleobase is a polymer comrpises nucleobases, which polymer can hybridize by Watson-Crick base pairing to a DNA having the complementary sequence. Nucleobases comprise a base, which is a purine, pyrimidine, or a derivative or analog thereof. Nucleobases include peptide nucleobases, the subunits of peptide nucleic acids, and morpholine nucleobases as well as nucleosides and nucleotides. Nucleosides are nucleobases that contain a pentosefuranosyl moiety, e.g., an optionally substituted riboside or 2'-deoxyriboside. Nucleosides can be linked by one of several linkage moieties, which may or may not contain a phosphorus. Nucleosides that are linked by unsubstituted phosphodiester linkages are termed nucleotides. An oligonucleobase chain has a single 5' and 3' terminus, which are the ultimate nucleobases of the polymer. A particular oligonucleobase chain can contain nucleobases of all types. An oligonucleobase compound is a compound comprising one or more oligonucleobase chains that are complementary and hybridized by Watson-Crick base pairing. Nucleobases are either deoxyribo-type or ribo-type. Ribo-type nucleobases are pentosefuranosyl containing nucleobases wherein the 2' carbon is a methylene substituted with a hydroxyl, alkyloxy or halogen. Deoxyribo-type nucleobases are nucleobases other than ribo-type nucleobases and include all nucleobases that do not contain a pentosefuranosyl moiety.

Single-stranded oligonucleotide mutational vectors (SSMOVs) can be prepared to introduce mutations to the AHAS coding sequence. SSMOVs can be prepared in accordance with International Patent Publication No. WO 01/15740 and U.S. Patent Nos. 6,271,360; 6,479,292; and 7,094,606. The sequence of the SSOMV can be based on the same principles as the mutational vectors described in U.S. Pat. Nos. 5,565,350; 5,731,181, 5,756,325; 5,871,984; 5,760,012; 5,888,983; 5,795,972; 5,780,296; 5,945,339; 6,004,804; and 6,010,907 and in International Publication Nos. WO 98/49350; WO 99/07865; WO 99/58723; WO 99/58702; and WO 99/40789.

The sequence of the SSOMV contains two regions that are homologous with the target sequence separated by a region that contains the desired genetic alteration, termed the mutator region. The mutator region can have a sequence that is the same length as the sequence that separates the homologous regions in the target sequence, but having a different sequence. Such a mutator region can cause a substitution. Alternatively, the homologous regions in the SSOMV can be contiguous to each other, while the regions in the target gene having the same sequence are separated by one, two or more nucleotides. Such a SSOMV causes a deletion from the target gene of the nucleotides that are absent from the SSOMV. Lastly, the sequence of the target gene that is identical to the homologous regions may be adjacent in the target gene but separated by one two or more nucleotides in the sequence of the SSOMV. Such an SSOMV causes an insertion in the sequence of target gene.

The nucleotides of the SSOMV are deoxyribonucleotides that are linked by unmodified phosphodiester bonds except that the 5' terminal and/or 3' terminal internucleotide linkage or alternatively the two 5' terminal and/or 3' terminal internucleotide linkages can be a phosphorothioate or phosphoamidate. As used herein an internucleotide linkage is the linkage between nucleotides of the SSOMV and does not include the linkage between the 5' end nucleotide or 3' end nucleotide and a blocking substituent. In a one specific embodiment the length of the SSOMV is between 21 and 60 deoxynucleotides and the lengths of the homology regions are, accordingly, a total length of at least 20 deoxynucleotides and at least two homology regions should each have lengths of at least 8 deoxynucleotides.

The SSOMVs can be designed to be complementary to either the coding or the non-coding strand of the target gene. When the desired mutation is a substitution of a single base, it is preferred that both the mutator nucleotide be a pyrimidine. To the extent that is consistent with achieving the desired functional result it is preferred that both the mutator nucleotide and the targeted nucleotide in the complementary strand be pyrimidines. In one embodiment, the SSOMVs that encode transversion mutations, i.e., a C or T mutator nucleotide is mismatched, respectively, with a C or T nucleotide in the complementary strand.

In addition to the oligodeoxynucleotide, the SSOMV can contain a 5' blocking substituent that is attached to the 5' terminal carbons through a linker. The chemistry of the linker can be any chemistry and is at least 6 atoms long, and the linker is preferably flexible. A variety of non-toxic substituents such as biotin, cholesterol or other steroids or a non-intercalating cationic fluorescent dye can be used. Examples of reagents to make SSOMV include the reagents sold as Cy3™ and Cy5™ by Glen Research, Sterling Va. (now GE Healthcare), which are blocked phosphoroamidites that upon incorporation into an oligonucleotide yield 3,3,3',3'-tetramethyl N,N'-isopropyl substituted indomonocarbocyanine and indodicarbocyanine dyes, respectively. In one disclosure the reagent is Cy3. When the indocarbocyanine is N-oxyalkyl substituted it can be conveniently linked to the 5' terminal of the oligodeoxynucleotide through a phosphodiester bond with a 5' terminal phosphate. The chemistry of the dye linker between the dye and the oligodeoxynucleotide is not critical and is chosen for synthetic convenience. When the commercially available Cy3 phosphoramidite is used as directed the resulting 5' modification consists of a blocking substituent and linker together which are a N-hydroxypropyl, N'-phosphatidylpropyl 3,3,3',3'-tetramethyl indomonocarbocyanine.

In one disclosure the indocarbocyanine dye is tetra substituted at the 3 and 3' positions of the indole rings. Without limitations as to theory these substitutions prevent the dye from being an intercalating dye. The identity of the substituents at these positions is not critical. The SSOMV can, in addition, have a 3' blocking substituent. Again the chemistry of the 3' blocking substituent is not critical.

The change to be introduced into the AHAS gene can be in any region of the nucleic acid sequence, including coding and non-coding regions. In one disclosure the change to be introduced into the target (AHAS) gene is encoded by the heterologous region. The change to be introduced into the gene may be a change in one or more bases of the gene sequence (i.e. a substitution) or the addition or deletion of one or more bases.

The present disclosure also provides the herbicide-resistant *Brassica* line that is referred to herein as BnCL120C7. A deposit of at least 2500 seeds from *Brassica* line BnCL120C7 with the Patent Depository of the American Type Culture Collection (ATCC), Manassas, VA 20110 USA was made on June 23, 2008 and assigned ATCC Patent Deposit Number PTA-9278. The present invention also provides the herbicide-resistant *Brassica* line that is referred to herein as BnCL131A1. A deposit of at least 2500 seeds from *Brassica* line BnCL131A1 was made on June 23, 2008 and assigned ATCC Patent Deposit Number PTA-9279. The present invention also provides the herbicide-resistant *Brassica* line that is referred to herein as BnCL140B3. A deposit of at least 2500 seeds from *Brassica* line BnCL140B3 with the Patent Depository of the American Type Culture Collection (ATCC), Manassas, VA 20110 USA was made on August 27, 2008 and assigned ATCC Patent Deposit Number PTA-9402. The present disclosure also provides the herbicide-resistant *Brassica* line that is referred to herein as BnCL140C7. A deposit of at least 2500 seeds from *Brassica* line BnCL140C7 with the Patent Depository of the American Type Culture Collection (ATCC), Manassas, VA 20110 USA was made on August 27,2008 and assigned ATCC Patent Deposit Number PTA-9403. The present disclosure also provides the herbicide-resistant *Brassica* line that is referred to herein as PM1PM2/BnCL131A1. A deposit of at least 2500 seeds from *Brassica* line PM1PM2/BnCL131A1 with the Patent Depository of the American Type Culture Collection (ATCC), Manassas, VA 20110 USA was made on September 9, 2009 and assigned ATCC Patent Deposit Number PTA-10321. The deposits will be maintained under the terms of the Budapest Treaty on the International Recognition of the Deposit of Microorganisms for the Purposes of Patent Procedure. The deposit of *Brassica* lines BnCL120C7, BnCL131A1, BnCL140B3, BnCL140C7, and PM1PM2/BnCL131A1 was made for a term of at least 30 years and at least 5 years after the most recent request for the furnishing of a sample of the deposit is received by the ATCC. Additionally, Applicants have satisfied all the requirements of 37 C.F.R. §§ 1.801-1.809, including providing an indication of the viability of the sample.

The mutant herbicide-resistant *Brassica* lines BnCL120C7, BnCL131A1, BnCL140B3, and BnCL140C7 exemplified in the present disclosure were produced using site-directed mutagenesis techniques as disclosed in the Examples. However, the present invention is not limited to herbicide-resistant *Brassica* plants that are produced by such methods. Any mutagenesis method known in the art may be used to produce the herbicide-resistant *Brassica* plants of the present invention. Such mutagenesis methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 250 to 290 nm), and chemical mutagens such as ethyl methanesulfonate (EMS), base analogues (e.g., 5-bromo-uracil), related compounds (e.g., 8-ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348
Further details of mutation breeding can be found in "Principles of Cultivar Development" Fehr, 1993 Macmillan Publishing Company.

In addition, site directed mutagenesis can be used to produce the mutated AHASL sequences in a host plant cell as disclosed herein.

The *Brassica* plants of the invention further include plants that comprise, relative to the wild-type AHASL protein, an asparagine at amino acid position 653 (*A. thaliana* nomenclature), a threonine at amino acid position 122 (*A. thaliana* nomenclature) and one or more additional amino acid substitutions in the AHASL protein relative to the wild-type AHASL protein, wherein such a *Brassica* plant has increased resistance to at least one herbicide when compared to a wild-type *Brassica* plant.

### Plants and Breeding methods

The AHAS-herbicide resistant plants and progeny of such plants of the present invention, such as *Brassica* AHAS-herbicide resistant plants, can be used in methods for preparing resistant plants, plants having increased tolerance to AHAS-inhibiting herbicides, and seeds of such plants. Thus, for example, the *Brassica* plants exemplified herein may be used in breeding programs to develop additional herbicide resistant plants, such as commercial varieties of *B. napus* (e.g. canola). In accordance with such methods, a first parent plant may be used in crosses with a second parent plant, where at least one of the first or second parent plants contains at least one AHASL nucleic acid sequence of the present invention, for example a nucleic acid molecule that encodes for an AHASL polypeptide having an A122T mutation and an S653N mutation. One application of the process is in the production of F₁ hybrid plants. Another important aspect of this process is that the process can be used for the development of novel parent, dihaploid or inbred lines. For example, a plant line as described herein could be crossed to any second plant, and the resulting hybrid progeny each selfed and/or sibbed for about 5 to 7 or more generations, thereby providing a large number of distinct, parent lines. These parent lines could then be crossed with other lines and the resulting hybrid progeny analyzed for beneficial characteristics. In this way, novel lines conferring desirable characteristics could be identified. Various breeding methods may be used in the methods, including haploidy, pedigree breeding, single-seed descent, modified single seed descent, recurrent selection, and backcrossing.

In some embodiments, the plants and progeny thereof display a synergistic effect rather than additive effect of herbicide tolerance, whereby the level of herbicide tolerance in the plants and the progeny thereof comprising multiple mutations is greater than the combined herbicide tolerance of plants comprising AHASL single mutant protein.

Plant lines containing the nucleic acid molecules of the present invention can be crossed by either natural or mechanical techniques. Mechanical pollination can be effected either by controlling the types of pollen that can be transferred onto the stigma or by pollinating by hand.

Descendent and/or progeny plants may be evaluated for the nucleic acid molecules of the present invention by any method to determine the presence of a mutated AHASL nucleic acid or polypeptide. Such methods include phenotypic evaluations, genotypic evaluations, or combinations thereof. The progeny plants may be evaluated in subsequent generations for herbicide resistance, and other desirable traits. Resistance to AHAS-inhibitor herbicides may be evaluated by exposing plants to one or more appropriate AHAS-inhibitor herbicides and evaluating herbicide injury. Some traits, such as lodging resistance and plant height, may be evaluated through visual inspection of the plants, while earliness of maturity may be evaluated by a visual inspection of seeds within pods (siliques). Other traits, such as oil percentage, protein percentage, and total glucosinolates of seeds may be evaluated using techniques such as Near Infrared Spectroscopy and/or liquid chromatography and/or gas chromatography.

Plants of the present invention can also be identified using any genotypic analysis method. Genotypic evaluation of the plants includes using techniques such as Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), Allele-specific PCR (AS-PCR), DNA Amplification Fingerprinting (DAF), Sequence Characterized Amplified Regions (SCARs), Amplified Fragment Length Polymorphisms (AFLPs), Simple Sequence Repeats (SSRs) which are also referred to as "Microsatellites." Additional compositions and methods for analyzing the genotype of the plants provided herein include those methods disclosed in U.S. Publication No. 2004/0171027, U.S. Publication No. 2005/02080506, and U.S. Publication No. 2005/0283858.

Evaluation and manipulation (through exposure to one or more appropriate AHAS-inhibitor herbicides) may occur over several generations. The performance of the new lines may be evaluated using objective criteria in comparison to check varieties. Lines showing the desired combinations of traits are either crossed to another line or self-pollinated to produce seed. Self-pollination refers to the transfer of pollen from one flower to the same flower or another flower of the same plant. Plants that have been self-pollinated and selected for type for many generations become homozygous at almost all gene loci and produce a uniform population of true breeding progeny.

Any breeding method may be used in the methods of the present disclosure. In one example, the herbicide-resistant plants of the present invention may be bred using a haploid method. In such methods, parents having the genetic basis for the desired complement of characteristics are crossed in a simple or complex cross. Crossing (or cross-pollination) refers to the transfer of pollen from one plant to a different plant. Progeny of the cross are grown and microspores (immature pollen grains) are separated and filtered, using techniques known to those skilled in the art [(e.g. Swanson, E. B. et al., (1987) Plant Cell Reports, 6: 94-97, "Efficient isolation of microspores and the production of microspore-derived embryos in Brassica napus, L.; and Swanson, E. B., (1990) Microspore culture in Brassica, pp. 159-169 in Methods in Molecular Biology, vol. 6, Plant Cell and Tissue Culture, Humana Press]. These microspores exhibit segregation of genes. The microspores are cultured in the presence of an appropriate AHAS-inhibitor herbicide, such as imazethapyr (e.g. PURSUIT™) or imazamox (e.g. SOLO™, BEYOND™, and RAPTOR™) or a 50/50 mix of imazethapyr and imazamox (e.g. ODYSSEY™), which kills microspores lacking the mutations responsible for resistance to the herbicide. Microspores carrying the genes responsible for resistance to the herbicide survive and produce embryos, which form haploid plants. Their chromosomes are then doubled to produce doubled haploids.

Other breeding methods may also be used.

For example, pedigree breeding may be used for the improvement of largely self-pollinating crops such as *Brassica* and canola. Pedigree breeding starts with the crossing of two genotypes, each of which may have one or more desirable characteristics that is lacking in the other or which complements the other. If the two original parents do not provide all of the desired characteristics, additional parents can be included in the crossing plan.

These parents may be crossed in a simple or complex manner to produce a simple or complex F₁. An F₂ population is produced from the F₁ by selfing one or several F₁ plants, or by intercrossing two F₁'s (i.e., sib mating). Selection of the best individuals may begin in the F₂ generation, and beginning in the F₃ the best families, and the best individuals within the best families are selected. Replicated testing of families can begin in the F₄ generation to improve the effectiveness of selection for traits with low heritability. At an advanced stage of inbreeding (i.e., F₆ and F₇), the best lines or mixtures of phenotypically similar lines may be tested for potential release as new cultivars. However, the pedigree method is more time-consuming than the haploidy method for developing improved AHAS-herbicide resistant plants, because the plants exhibit segregation for multiple generations, and the recovery of desirable traits is relatively low.

The single seed descent (SSD) procedure may also be used to breed improved varieties. The SSD procedure in the strict sense refers to planting a segregating population, harvesting a sample of one seed per plant, and using the population of single seeds to plant the next generation. When the population has been advanced from the F₂ to the desired level of inbreeding, the plants from which lines are derived will each trace to different F₂ individuals. The number of plants in a population declines each generation due to failure of some seeds to germinate or some plants to produce at least one seed. As a result, not all of the plants originally sampled in the F₂ population will be represented by a progeny when generation advance is completed.

In a multiple-seed procedure, canola breeders commonly harvest one or more pods from each plant in a population and thresh them together to form a bulk. Part of the bulk is used to plant the next generation and part is put in reserve. The procedure has been referred to as modified single-seed descent or the pod-bulk technique. The multiple-seed procedure has been used to save labor at harvest. It is considerably faster to thresh pods with a machine than to remove one seed from each by hand for the single-seed procedure. The multiple-seed procedure also makes it possible to plant the same number of seeds of a population each generation of inbreeding. Enough seeds are harvested to make up for those plants that did not germinate or produce seed.

Backcross breeding can be used to transfer a gene or genes for a simply inherited, highly heritable trait from a source variety or line (the donor parent) into another desirable cultivar or inbred line (the recurrent parent). After the initial cross, individuals possessing the phenotype of the donor parent are selected and are repeatedly crossed (backcrossed) to the recurrent parent. When backcrossing is complete, the resulting plant is expected to have the attributes of the recurrent parent and the desirable trait transferred from the donor parent.

Improved varieties may also be developed through recurrent selection. In this method, genetically variable population of heterozygous individuals is either identified or created by intercrossing several different parents. The best plants are selected based on individual superiority, outstanding progeny, or excellent combining ability. The selected plants are intercrossed to produce a new population in which further cycles of selection are continued.

The present disclosure provides a method of producing a plant having resistance to AHAS-inhibiting herbicides comprising: (a) crossing a first plant line with a second plant line to form a segregating population, where the first or the second plant line is an AHAS-inhibiting herbicide resistant plant comprising a nucleic acid molecule of the present invention; (b) screening the population for increased AHAS-inhibiting herbicide resistance, the presence of a nucleic acid molecule of the present invention, or both; and (c) selecting one or more members of the population having increased AHAS resistance relative to a wild-type plant or contains a nucleic acid molecule of the present invention. The first or second plant contains an AHASL nucleic acid molecule of the present invention. In one embodiment, the plants for use in the method are *Brassica* plants.

The present disclosure provides a method of introgressing an AHAS-inhibiting herbicide resistance trait into a plant comprising: (a) crossing at least a first AHAS-inhibiting herbicide resistant plant line with a second plant line to form a segregating population; (b) screening the population for increased AHAS-inhibiting herbicide resistance; and (c) selecting at least one member of the population having increased AHAS-inhibiting herbicide resistance. In one embodiment, the plants for use in the method are *Brassica* plants.

Alternatively, both first and second parent *Brassica* plants can be an AHAS-inhibiting herbicide resistant *Brassica* plant as described herein. Thus, any *Brassica* plant produced using a *Brassica* plant having increased AHAS-inhibiting herbicide resistance as described herein forms a part of the invention. As used herein, crossing can mean selfing, sibbing, backcrossing, crossing to another or the same parent line, crossing to populations, and the like.

The present disclosure also provides methods for producing an herbicide-resistant plant, such as an herbicide-resistant *Brassica* plant, through conventional plant breeding involving sexual reproduction. The methods comprise crossing a first plant that is resistant to an herbicide to a second plant that is not resistant to the herbicide. The first plant can be any of the herbicide resistant plants of the present invention including, for example, *Brassica* plants comprising at least one of the mutant nucleic acid molecules of the present invention that encode an herbicide resistant AHASL and non-transgenic *Brassica* plants that comprise the herbicide-resistance characteristics of the *Brassica* plant of BnCL120C7, BnCL131A1, BnCL140B3, or BnCL140C7. The second plant can be any plant that is capable of producing viable progeny plants (i.e., seeds) when crossed with the first plant. Typically, but not necessarily, the first and second plants are of the same species. For example, the mutant AHASL sequence on the A genome in a *B. napus* plant can be bred into other *Brassica* plants that also have the A genome by crossing a *B. napus* plant with, for example, a *B. juncea* plant. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the invention can additionally involve selecting plants that comprise the herbicide resistance characteristics of the first plant.

The present disclosure further provides methods for increasing the herbicide-resistance of a plant, particularly an herbicide-resistant *Brassica* plant, through conventional plant breeding involving sexual reproduction. The methods comprise crossing a first plant that is resistant to an herbicide to a second plant that may or may not be resistant to the herbicide or may be resistant to different herbicide or herbicides than the first plant. The first plant can be any of the herbicide resistant plants of the present invention including, for example, transgenic or non-trangenic plants comprising at least one of the nucleic acid molecules of the present invention that encode an herbicide resistant AHASL and non-transgenic *Brassica* plants that comprise the herbicide-resistance characteristics of the *Brassica* plant of BnCL120C7, BnCL131A1, BnCL140B3, or BnCL140C7. The second plant can be any plant that is capable of producing viable progeny plants (i.e., seeds) when crossed with the first plant. Typically, but not necessarily, the first and second plants are of the same species; as well, the first and second plants can be from different species but within the same genus (example: *Brassica juncea x Brassica napus, Brassica juncea x Brassica rapa, Brassica napus x Brassica oleracea, Brassica juncea x Brassica nigra,* etc.), and also, the first and second plants are of different genera (example: *Brassica x Sinapis).* The progeny plants produced by this method have increased resistance to an herbicide when compared to either the first or second plant or both. When the first and second plants are resistant to different herbicides, the progeny plants will have the combined herbicide resistance characteristics of the first and second plants. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods can additionally involve selecting plants that comprise the herbicide resistance characteristics of the first plant, the second plant, or both the first and the second plant.

The plants of the present invention can be transgenic or non-transgenic. An example of a non-transgenic *Brassica* plant having increased resistance to imidazolinone and/or sulfonylurea herbicides includes the *Brassica* plant of BnCL131A1, BnCL120C7, BnCL140B3, BnCL140C7, or PM1PM2/BnCL131A1; or a mutant, a recombinant, or a genetically engineered derivative of the plant of BnCL131A1, BnCL120C7, BnCL140B3, BnCL140C7, or PM1PM2/BnCL131A1; or of any progeny of the plant of BnCL131A1, BnCL120C7, BnCL140B3, BnCL140C7, or PM1PM2/BnCL131A1; or a plant that is a progeny of any of these plants; or a plant that comprises the herbicide resistance characteristics of the plant of BnCL131A1, BnCL120C7, BnCL140B3, BnCL140C7, PM1PM2/BnCL131A1.

The present invention also provides plants, plant organs, plant tissues, plant cells, seeds, and non-human host cells that are transformed with at least one nucleic acid molecule, expression cassette, or transformation vector of the invention. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. In one embodiment, the transformed plants, plant tissues, plant cells, and seeds of the invention are *Brassica* and crop plants.

The present invention also provides a seed of an AHAS-inhibiting herbicide tolerant plant capable of producing a plant having AHAS-inhibiting herbicide resistance obtained from plants produced by the methods of the present invention.

The present disclosure also provides for a plant grown from the seed of a plant having AHAS-inhibiting herbicide resistance obtained from plants grown from the seed having the herbicide resistance trait, as well as plant parts and tissue cultures from such plants.

Also provided herein is a container of plant seeds, where the seeds are capable of producing an AHAS-inhibiting herbicide resistant plant. The container of seeds may contain any number, weight or volume of seeds. For example, a container can contain at least, or greater than, about 10, 25, 50, 75, 100,200, 300, 400, 500, 600, 700, 800, 900, 1000, 1500, 2000, 2500, 3000, 3500, 4000, 4500, 5000 or more seeds. Alternatively, the container can contain at least, or greater than, about 1 ounce, 5 ounces, 10, ounces, 1 pound, 2 pounds, 3 pounds, 4 pounds, 5 pounds or more seeds.

Containers of plant seeds may be any container available in the art. By way of non-limiting example, a container may be a box, a bag, a packet, a pouch, a tape roll, a pail, a foil, or a tube.

The seeds contained in the containers of seeds can be treated or untreated seeds. In one aspect, the seeds can be treated to improve germination, for example, by priming the seeds, or by disinfection to protect against seed-born pathogens. In another aspect, seeds can be coated with any available coating to improve, for example, plantability, seed emergence, and protection against seed-born pathogens. Seed coating can be any form of seed coating including, but not limited to pelleting, film coating, and encrustments.

In addition, the *Brassica* plants may be used in breeding methods to produce plants having additional traits of interest combined with the AHAS-inhibitor tolerance (also referred to as "stacked traits" or "trait stacking"), such as combinations of resistance to additional herbicides, such as glyphosate, glufosinate, and/or dicamba. In addition, the *Brassica* plants may be used in breeding methods to produce *Brassica* plants having multiple AHAS-inhibiting herbicide resistance coding sequences. Also, the disclosed plants may be used in breeding methods to produce plants having the AHAS-inhibiting herbicide tolerance trait combined with other agronomically important traits, such as disease and pathogen resistance, such as those conferred by the Bt gene, black leg resistance.

The disclosed methods of breeding include methods of breeding AHAS-inhibiting herbicide resistant *Brassica* plants, where the method comprises the steps of (a) crossing a *Brassica* plant containing a mutagenized nucleic acid molecule encoding an herbicide tolerant AHAS protein having a mutation at a position corresponding to position A122 of SEQ ID NO: 23 and a mutation at a position corresponding to position S653 of SEQ ID NO: 23 with a second *Brassica* plant; and (b) obtaining seed from the cross. The obtained seeds may be screened to identify seeds that contain a mutagenized nucleic acid molecule. Such methods may further involve obtaining a DNA sample from the seed of the cross and assaying the sample for the presence or absence of the mutagenized nucleic acid molecule. Alternatively, the seeds may be screened for AHAS-inhibiting herbicide tolerance to identify seeds or progeny that express the herbicide tolerant AHAS nucleic acid.

The present disclosure provides a method of producing a *Brassica* plant having resistance to AHAS-inhibiting herbicides comprising: (a) crossing a first *Brassica* line with a second *Brassica* line to form a segregating population, where the first *Brassica* line is an AHAS-inhibiting herbicide resistant *Brassica* plant comprising the mutagenized AHAS nucleic acid molecule of the present invention; (b) screening the population for increased AHAS-inhibiting herbicide resistance; and (c) selecting one or more members of the population having increased AHAS resistance relative to a wild-type *Brassica* plant.

The present disclosure provides a method of introgressing an AHAS-inhibiting herbicide resistance trait into a *Brassica* plant comprising: (a) crossing at least a first AHAS-inhibiting herbicide resistant *Brassica* line with a second *Brassica* line to form a segregating population; (b) screening the population for increased AHAS-inhibiting herbicide resistance; and (c) selecting at least one member of the population having increased AHAS-inhibiting herbicide resistance.

Alternatively, both first and second parent *Brassica* plants can be an AHAS-inhibiting herbicide resistant *Brassica* plant as described herein. Thus, any *Brassica* plant produced using a *Brassica* plant having the mutagenized AHAS nucleic acid molecule as described herein forms a part of the disclosure. As used herein, crossing can mean selfing, sibbing, backcrossing, crossing to another or the same parent line, crossing to populations, and the like.

The mutagenized AHAS nucleic acid molecule of the invention can be engineered into a molecular stack with a nucleic acid molecule that confers resistance to a second herbicide, such as glyphosate or glufosinate. The molecular stack may further comprise at least one additional nucleic acid molecule that confers tolerance to a third herbicide. In one disclosure the sequence confers tolerance to glufosinate, and in a specific embodiment, the sequence comprises pat.

The *Brassica* plant may comprise one or more traits of interest, e.g. the mutagenized AHAS nucleic acid molecule is stacked with any combination of nucleic acid molecule sequences of interest in order to create plants with a desired combination of traits. A trait, as used herein, refers to the phenotype derived from a particular sequence or groups of sequences. For example, herbicide-tolerance nucleic acid molecules may be stacked with any other nucleic acid molecules encoding polypeptides having pesticidal and/or insecticidal activity, such as Bacillus thuringiensis toxic proteins (described in U.S. Pat. Nos. 5,366,892; 5,747,450; 5,737,514; 5,723,756; 5,593,881; Geiser et al. (1986) Gene 48: 109-118; Lee et al. (2003) Appl. Environ. Microbiol. 69: 4648-4657 (Vip3A); Galitzky et al. (2001) Acta Crystallogr. D. Biol. Crystallogr. 57: 1101-1109 (Cry3Bb1); and Herman et al. (2004) J. Agric. Food Chem. 52: 2726-2734 (CrylF)), lectins (Van Damme et al. (1994) Plant Mol. Biol. 24: 825-830, pentin (described in U.S. Pat. No. 5,981,722), and the like. The combinations generated can also include multiple copies of any one of the nucleic acid molecules of interest.

Mutagenized AHAS nucleic acid molecule can be stacked with other herbicide-tolerance traits to create a *Brassica* plant with additional improved properties. Other herbicide-tolerance nucleic acid molecules that could be used in such embodiments include those conferring tolerance to glyphosate or to AHAS inhibitors by other modes of action, such as, for example, a gene that encodes a glyphosate oxido-reductase enzyme as described more fully in U.S. Pat. Nos. 5,776,760 and 5,463,175. Other traits that could be combined with the mutagenized AHAS nucleic acid molecule include those derived from nucleic acid molecules that confer on the plant the capacity to produce a higher level of 5-enolpyruvylshikimate-3-phosphate synthase (EPSPS), for example, as more fully described in U.S. Pat. Nos. 6,248,876 B1; 5,627,061; 5,804,425; 5,633,435; 5,145,783; 4,971,908; 5,312,910; 5,188,642; 4,940,835; 5,866,775; 6,225,114 B1; 6,130,366; 5,310,667; 4,535,060; 4,769,061; 5,633,448; 5,510,471;
and 5,491,288; and international publications WO 97/04103; WO 00/66746; WO 01/66704; and WO 00/66747. Other traits that could be combined with the mutagenized AHAS nucleic acid molecules include those conferring tolerance to sulfonylurea and/or imidazolinone, for example, as described more fully in U.S. Pat. Nos. 5,605,011; 5,013,659; 5,141,870; 5,767,361; 5,731,180; 5,304,732; 4,761,373; 5,331,107; 5,928,937; and 5,378,824; and international publication WO 96/33270.

Thus, *Brassica* plants are provided that contain the mutagenized AHAS nucleic acid molecules of the present invention in combination (i.e. "stacked") with other mutant or recombinant nucleic acids encoding genes of interest, such as AHAS-inhibitor tolerant nucleic acid molecules. In one example, the additional AHAS-inhibitor tolerance nucleic acid molecules that may be used in such combinations may contain any mutation in comparison to the wild-type AHAS sequence, such as, for example, AHAS proteins having mutations P197S, A205V, S653N, W574L, and/or A122T, and combinations thereof (e.g., a double or triple mutant AHAS sequence). In one example, *Brassica* plants are provided that combine an herbicide tolerant AHASL protein comprising amino acid mutations at positions A122 and S653 with another herbicide tolerant AHASL protein comprising an amino acid mutation at position W574 and an herbicide tolerant AHASL protein comprising an amino acid mutation at position S653. Also provided are seeds of such mutant *Brassica* plant lines designated PM1PM2/BnCL131A1, a sample of said seed having been deposited under ATCC Accession No. PTA-10321.

In addition, the mutated or recombinant nucleic acids to be combined with the AHAS-inhibitor tolerant nucleic acids may be located on any of the genomes of *Brassica* and produce heterozygous or homozygous stacks. Thus, the nucleic acids of interest for use in stacking may be found on the same or different genomes as the double mutation AHAS-inhibitor tolerant nucleic acid of the present invention. For example, *Brassica napus* plants (containing the AACC genome) are provided containing the mutagenized AHAS of the present invention in combination with imidazolinone tolerance nucleic acid molecules located, for example, on the "A" genome to produce a heterozygous stack or on the "C" genome to produce a homozygous stack. In another example, *Brassica juncea* plants (containing the AABB genome) are provided containing the mutagenized AHAS of the present invention in combination with imidazolinone tolerance nucleic acid molecules located, for example, on the "A" genome to produce a heterozygous stack or on the "B" genome to produce a homozygous stack. In still another example, *Brassica rapa* plants (containing the AA genome) are provided containing the mutagenized AHAS of the present invention in combinations with imidazolinone tolerance nucleic acid molecules located, for example, on the "A" genome to produce a heterozygous stack.

Examples of such *Brassica* plants include *Brassica napus* plants that contain the mutagenized AHAS nucleic acid molecule of the present invention in combination with PM2, which is an AHASL nucleic acid molecule from the A genome containing a single mutation, W574L (See, e.g., WO 2004/040011), and in further combination with PM1, which is an AHASL nucleic acid molecule from the C genome sequence containing a single mutation S653N (See, e.g., WO 2004/040011). Another example includes *Brassica juncea* plants containing the mutagenized AHAS nucleic acid molecule of the present invention in combination with bR, which is an AHASL nucleic acid molecule from the B genome containing a single mutation S653N (e.g., U.S. Patent Publication Nos. 2005/0283858 and 2009/0013424). Other examples include *Brassica* plants containing the mutagenized AHAS nucleic acid molecules of the present invention in combination with PM1.

In some embodiments, combinations of AHAS-inhibitor tolerant nucleic acids may provide any level of tolerance to an AHAS-inhibitor, such as additive effects or synergistic effects.

The mutagenized AHAS nucleic acid molecules may be stacked with, for example, hydroxyphenyl-pyruvatedioxygenases which are enzymes that catalyze the reaction in which para-hydroxyphenylpyruvate (HPP) is transformed into homogentisate. Molecules which inhibit this enzyme and which bind to the enzyme in order to inhibit transformation of the HPP into homogentisate are useful as herbicides. Traits conferring tolerance to such herbicides in plants are described in U.S. Pat. Nos. 6,245,968 B1; 6,268,549; and 6,069,115; and international publication WO 99/23886. Other examples of suitable herbicide-tolerance traits that could be stacked with the mutagenized AHAS sequences include aryloxyalkanoate dioxygenase nucleic acid molecules (which reportedly confer tolerance to 2,4-D and other phenoxy auxin herbicides as well as to aryloxyphenoxypropionate herbicides as described, for example, in WO2005/107437) and dicamba-tolerance nucleic acid molecules as described, for example, in Herman et al. (2005) J. Biol. Chem. 280: 24759-24767.

Other examples of herbicide-tolerance traits that could be combined with the mutagenized AHAS nucleic acid molecules of the present invention include those conferred by nucleic acid molecules encoding an exogenous phosphinothricin acetyltransferase, as described in U.S. Pat. Nos. 5,969,213; 5,489,520; 5,550,318; 5,874,265; 5,919,675; 5,561,236; 5,648,477; 5,646,024; 6,177,616; and 5,879,903. Plants containing an exogenous phosphinothricin acetyltransferase can exhibit improved tolerance to glufosinate herbicides, which inhibit the enzyme glutamine synthase. Other examples of herbicide-tolerance traits that could be combined with the mutagenized AHAS sequences include those conferred by nucleic acid molecules conferring altered protoporphyrinogen oxidase (protox) activity, as described in U.S. Pat. Nos. 6,288,306 B1; 6,282,837 B1; and 5,767,373; and international publication WO 01/12825. Plants containing such nucleic acid molecules can exhibit improved tolerance to any of a variety of herbicides which target the protox enzyme (also referred to as "protox inhibitors").

Still other examples of herbicide tolerance genes that can be combined with the mutagenized AHAS nucleic acid molecules include those genes conferring tolerance to dicamba herbicides. Such genes are known in the art, and are described, for example, in U.S. Patent No. 7,022,896 and U.S. Publication No. 2008/0015110.

Other examples of herbicide-tolerance traits that could be combined with the mutagenized AHAS nucleic acid molecules of the present invention include those conferring tolerance to at least one herbicide in a plant such as, for example, a *Brassica* plant. Herbicide-tolerant *Brassica* plants are known in the art, as are plants that vary in their tolerance to particular herbicides. See, e.g., U.S. Pat. No. 5,627,061. The trait(s) responsible for these tolerances can be combined by breeding or via other methods, such as transgenic, with the mutagenized AHAS nucleic acid molecules to provide a plant as well as methods of use thereof.

The mutagenized AHAS nucleic acid molecules can also be combined with at least one other trait to produce plants of the present invention that further comprise a variety of desired trait combinations including, but not limited to, traits desirable for animal feed such as high oil content (e.g., U.S. Pat. Nos. 7,238,856; 7,268,276); amino acid composition, protein content, improved digestibility, or altered fatty acid compositions.

The mutagenized AHAS nucleic acid molecules can also be combined with other desirable traits such as, for example, avirulence and disease resistance genes (Jones et al. (1994) Science 266: 789-793; Martin et al. (1993) Science 262: 1432-1436; Mindrinos et al. (1994) Cell 78: 1089-1099), and traits desirable for processing or process products such as modified oils (e.g., fatty acid desaturase genes (U.S. Pat. No. 5,952,544; WO 94/11516)); and polymers or bioplastics (e.g., U.S. Pat. No. 5,602,321; beta-ketothiolase, polyhydroxybutyrate synthase, and acetoacetyl-CoA reductase (Schubert et al. (1988) J. Bacteriol. 170:5837-5847) facilitate expression of polyhydroxyalkanoates (PHAs)). One could also combine herbicide-tolerant nucleic acid molecules with nucleic acid molecules providing any agronomic traits.

The stacked combinations can be created by any method including, but not limited to, breeding plants by any known methodology, or genetic transformation, or combinations. If the sequences are stacked by genetically transforming the plants, the nucleic acid molecule sequences of interest can be combined at any time and in any order. The traits can be introduced simultaneously in a co-transformation protocol with the nucleic acid molecules of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the nucleic acid molecule of interest. This may be combined with any combination of other suppression cassettes or overexpression cassettes to generate the desired combination of traits in the plant. It is further recognized that nucleic acid molecules can be stacked at a desired genomic location using a site-specific recombination system. See, for example, WO99/25821, WO99/25854, WO99/25840, WO99/25855, and WO99/25853.

### Detection Methods

Methods and compositions for identifying a plant comprising the mutagenized AHAS nucleic acid molecules and polypeptides, including progeny and derivatives, are also provided. Such methods find use in identifying and/or detecting plants containing such mutagenized sequences in any biological material. Such methods include, for example, methods to confirm seed purity and methods for screening seeds in a seed lot for the mutagenized sequences of the present invention. In one embodiment, a method for identifying the mutagenized AHAS sequences in a biological sample is provided and comprises forming a mixture of a biological sample and a first and a second nucleic acid primer capable of amplifying a mutagenized AHAS nucleic acid molecule; reacting the mixture under conditions that allow the first and second nucleic acid primers to amplify the mutagenized AHAS nucleic acid molecule; and, detecting the presence or absence of an amplified mutagenized AHAS sequence nucleic acid molecule.

The amplified nucleic acid molecule (amplicon) can be of any length that allows for the detection of a mutagenized AHAS sequence. For example, the amplicon can be about 10, 50, 100, 200, 300, 500, 700, 100, 2000, 3000, 4000, 5000 nucleotides in length or longer.

Also provided are methods for identifying or detecting a mutagenized AHAS sequence in a biological sample comprising forming a mixture containing a biological sample having *Brassica* DNA and a nucleic acid molecule probe that is capable of hybridizing to a mutagenized nucleic acid molecule, reacting the mixture under conditions that allow the nucleic acid molecule probe to hybridize to a mutagenized AHAS nucleic acid molecule, and detecting whether the nucleic acid molecule probe hybridizes to a mutagenized AHAS nucleic acid molecule in the sample, where the presence of hybridization indicates the presence of a mutagenized AHAS nucleic acid molecule.

### Transgenics

The present disclosure also provides methods for increasing AHAS activity in a plant comprising transforming a plant with a nucleic acid construct comprising a promoter operably linked to an AHASL nucleotide of the invention. The methods involve introducing a nucleic acid construct of the invention into at least one plant cell and regenerating a transformed plant therefrom. The nucleic acid construct comprises at least one nucleotide that encodes an herbicide-resistant AHASL protein of the invention, particularly the nucleotide sequences of BN02-120 or BN02-131 as set forth in Figures 3 and 4, and fragments and variants thereof. The methods further involve the use of a promoter that is capable of driving gene expression in a plant cell. In one embodiment, such a promoter is a constitutive promoter or a tissue-preferred promoter. A plant produced by this method comprises increased AHAS activity, particularly herbicide-tolerant AHAS activity, when compared to an untransformed plant. Thus, the methods find use in enhancing or increasing the resistance of a plant to at least one herbicide that interferes with the catalytic activity of the AHAS enzyme, particularly an imidazolinone herbicide.

The methods for producing an herbicide-resistant plant comprise transforming a plant cell with a nucleic acid construct comprising a nucleotide sequence operably linked to a promoter that drives expression in a plant cell and regenerating a transformed plant from said transformed plant cell. The nucleotide sequence is selected from those nucleotide sequences that encode the herbicide-resistant AHASL proteins of the invention, particularly the nucleotide sequences of BN02-120 or BN02-131 as set forth in Figures 3 and 4, and fragments and variants thereof. An herbicide-resistant plant produced by this method comprises enhanced resistance, compared to an untransformed plant, to at least one herbicide, particularly an herbicide that interferes with the activity of the AHAS enzyme such as, for example, an imidazolinone herbicide or a sulfonylurea herbicide.

### Herbicide resistance and weed control

The AHAS-inhibiting herbicide resistant plants of the invention find use in methods for controlling weeds. Thus, the present invention further provides a method for controlling weeds in the vicinity of an herbicide-resistant plant, such as a *Brassica* plant, comprising an AHASL nucleic acid molecule of the invention. The method comprises applying an effective amount of an AHAS-inhibiting herbicide to the weeds and to the herbicide-resistant plant, wherein the plant has resistance to at least one AHAS-inhibiting herbicide, such as an imidazolinone or sulfonylurea herbicide, when compared to a wild-type plant. Any plants having the nucleic acid sequences of the present invention can be used in such methods. In one embodiment the herbicide-resistant plants of the invention are *Brassica* crop plants, including, but not limited to, *Brassica napus, Brassica rapa,* and *Brassica juncea.*

By providing plants having increased resistance to AHAS-inhibiting herbicides, such as imidazolinone and sulfonylurea herbicides, a wide variety of formulations can be employed for protecting plants from weeds, so as to enhance plant growth and reduce competition for nutrients. A herbicide can be used by itself for pre-emergence, post-emergence, pre-planting and at planting control of weeds in areas surrounding the plants described herein or an AHAS-inhibiting herbicide formulation can be used that contains other additives. The herbicide can also be used as a seed treatment. An effective concentration or an effective amount of the herbicide, or a composition comprising an effective concentration or an effective amount of the herbicide can be applied directly to the seeds prior to or during the sowing of the seeds. Additives found in an AHAS-inhibiting herbicide formulation or composition include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irrigation, dusting, coating, and the like.

The AHAS-inhibiting herbicide for use in the methods provided herein can be applied by any method known in the art including, but not limited to, seed treatment, soil treatment, and foliar treatment.

The present disclosure provides methods for enhancing the tolerance or resistance of a plant, plant tissue, plant cell, or other host cell to at least one herbicide that interferes with the activity of the AHAS enzyme. Preferably, such an AHAS-inhibiting herbicide is an imidazolinone herbicide, a sulfonylurea herbicide, a triazolopyrimidine herbicide, a pyrimidinyloxybenzoate herbicide, a sulfonylaminocarbonyltriazolinone herbicide, or mixture thereof. More preferably, such an herbicide is an imidazolinone herbicide, a sulfonylurea herbicide, or mixture thereof. For the present invention, the imidazolinone herbicides include, but are not limited to, PURSUIT® (imazethapyr), CADRE® (imazapic), RAPTOR® (imazamox), SCEPTER@ (imazaquin), ASSERT® (imazethabenz), ARSENAL® (imazapyr), a derivative of any of the aforementioned herbicides, and a mixture of two or more of the aforementioned herbicides, for example, imazapyr/imazamox (ODYSSEY®). More specifically, the imidazolinone herbicide can be selected from, but is not limited to, 2- (4-isopropyl-4-methyl-5-oxo-2-imidiazolin-2-yl) -nicotinic acid, [2- (4-isopropyl)-4-][methyl-5-oxo-2-imidazolin-2-yl)-3-quinolinecarboxylic] acid, [5-ethyl-2-(4-isopropyl-]4-methyl-5-oxo-2-imidazolin-2-yl)-nicotinic acid, 2- (4-isopropyl-4-methyl-5-oxo-2- imidazolin-2-yl)-5- (methoxymethyl)-nicotinic acid, [2-(4-isopropyl-4-methyl-5-oxo-2-]imidazolin-2-yl)-5-methylnicotinic acid, and a mixture of methyl [6-(4-isopropyl-4-] methyl-5-oxo-2-imidazolin-2-yl) -m-toluate and methyl [2-(4-isopropyl-4-methyl-5-] oxo-2-imidazolin-2-yl) -p-toluate. The use of 5-ethyl-2- (4-isopropyl-4-methyl-5-oxo- 2-imidazolin-2-yl) -nicotinic acid and [2-(4-isopropyl-4-methyl-5-oxo-2-imidazolin-2-] yl)-5- (methoxymethyl)-nicotinic acid is preferred. The use of [2-(4-isopropyl-4-] methyl-5-oxo-2-imidazolin-2-yl)-5-(methoxymethyl)-nicotinic acid is particularly preferred.

The sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl, halosulfuron, azimsulfuron, cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfuron, a derivative of any of the aforementioned herbicides, and a mixture of two or more of the aforementioned herbicides. The triazolopyrimidine herbicides of the invention include, but are not limited to, cloransulam, diclosulam, florasulam, flumetsulam, metosulam, and penoxsulam. The pyrimidinyloxybenzoate herbicides of the invention include, but are not limited to, bispyribac, pyrithiobac, pyriminobac, pyribenzoxim and pyriftalid. The sulfonylamino-carbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Accordingly, the herbicides further include pyrimidinylthiobenzoate herbicides, including, but not limited to, the pyrimidinyloxybenzoate herbicides described above.

The present disclosure also provides agricultural compositions for application to the disclosed *Brassica* plants. Such compositions may include herbicides, fungicides, bacteriacides, fertilizers, and the like. In one embodiment, the agricultural compositions are herbicidal compositions comprising one or more herbicides or combinations of one or more herbicides with another agricultural composition, such as a fungicide, bacteriacide, fertilizer, and the like.

Any herbicide can be applied to a crop, crop part, or the area of cultivation containing a *Brassica* plant containing an AHAS-inhibitor tolerant nucleic acid sequence of the present invention. Classification of herbicides (i.e., the grouping of herbicides into classes and subclasses) is well-known in the art and includes classifications by HRAC (Herbicide Resistance Action Committee) and WSSA (the Weed Science Society of America). The HRAC classification is available, for example, on the worldwide at the website hracglobal.com/Publications/Classificationof HerbicideModeofAction/tabid/222/Default.aspx.

The present invention provides methods that involve the use of at least one AHAS inhibiting herbicide selected from the group consisting of imidazolinone herbicides, sulfonylurea herbicides, triazolopyrimidine herbicides, pyrimidinyloxybenzoate herbicides, sulfonylaminocarbonyltriazolinone herbicides, and mixtures thereof. In these methods, the AHAS-inhibiting herbicide can be applied by any method known in the art including, but not limited to, seed treatment, soil treatment, and foliar treatment.

The AHAS-inhibiting herbicide can be combined with one or more additional agricultural compositions, such as additional herbicides, fungicides, bacteriocides, anti-viral compositions, or combinations thereof. The additional herbicides for use in the combinations include any herbicide, including sulfamide herbicides, organophosphate herbicides, and benzothiadiazinone herbicides. Sulfamide herbicides include, but are not limited to saflufenacil. Organophosphate herbicides include, but are not limited to glyphosate and glufosinate. Benzothiadiazinone herbicides include, but are not limited to bentazon. Fungicides for use in such combinations include, but are not limited to pyraclostrobin.

Protoporphyrinogen [IX] oxidase (PPO) inhibiting herbicides also find use in the compositions of the present invention. PPO inhibiting herbicides are known in the art and include, but are not limited to diphenylether herbicides (including nitrophenyl ether herbicides), such as acifluorfen (5-[2-chloro-4-(trifluoromethyl)phenoxy]-2-nitrobenzoic acid), bifenox (methyl 5-(2,4-dichlorophenoxy)-2-nitrobenzoate), DPEI (5-[2-chloro-4-(trifluoromethyl)phenoxy]-2-nitroacetophenone oxime-o-(acetic acid, methyl ester)), DPEII (5-[2-chloro-4-(trifluoromethyl)phenoxy]-3-methoxyphthalide), ethoxyfen ((1S)-1-carboxyethyl 2-chloro-5-[2-chloro-4-(trifluoromethyl)phenoxy]benzoate), fomesafen (5-[2-chloro-4-(trifluoromethyl)phenoxy]-N-(methylsulfonyl)-2-nitrobenzamide), lactofen (ethyl O-[5-(2-chloro-α,α,α-trifluoro-p-tolyloxy)-2-nitrobenzoyl]-DL-lactate), and oxyfluorfen (2-chloro-1-(3-ethoxy-4-nitrophenoxy)-4-(trifluoromethyl)benzene). PPO-inhibiting herbicides also include dicaboximide herbicides such as N-phenyl-phthalimides flumiclorac ([2-chloro-5-(cyclohex-1-ene-1,2-dicarboximido)-4-fluororophenoxy]acetic acid), flumioxazin (N-(7-fluoro-3,4-dihydro-3-oxo-4-prop-2-ynyl-2H-1,4-benzoxazin-6-yl)cyclohex-1-ene-1,2-dicarboxamide). PPO-inhibiting herbicides further include triazolinone herbicides such as carfentrazone (α,2-dichloro-5-[4-(difluoromethyl)-4,5-dihydro-3-methyl-5-oxo-1H-1,2,4-triazol-1-yl]-4-fluorobenzenepropanoic acid), and sulfentrazone (N-[2,4-dichloro-5-[4-(difluoromethyl)-4,5-dihydro-3-methyl-5-oxo-1H-1,2,4-triazol-1-yl]phenyl]methanesulfonamide). PPO-inhibiting herbicides also include phenylpyrazole herbicides, including, but not limited to nipyraclofen (1-[2,6-dichloro-4-(trifluoromethyl)phenyl]-4-nitro-1H-pyrazol-5-amine) and pyraflufen (2-chloro-5-(4-chloro-5-difluoromethoxy-1-methylpyrazol-3-yl)-4-fluorophenoxyacetic acid). PPO-inhibiting herbicides also include oxadiazolone herbicides such as oxadiazon (3-[2,4-dichloro-5-(1-methylethoxy)phenyl]-5-(1,1-dimethylethyl)-1,3,4-oxadiazol-2(3H)-one) and oxadiargyl (5-*tert*-butyl-3-[2,4-dichloro-5-(prop-2-ynyloxy)phenyl]-1,3,4-oxadiazol-2(3*H*)-one). PPO-inhibiting herbicides further include thiadiazolone herbicides such as fluthiacet ([[2-chloro-4-fluoro-5-[(tetrahydro-3-oxo-1H,3H-[1,3,4]thiadiazolo[3,4-a]pyridazin-1-ylidene)amino]phenyl] thio]acetic acid); as well as those described in section III.4) of US2008254985 to Zagar and Sievernich.

Auxinic herbicides also find use in the compositions.

Auxinic herbicides include those comprising herbicidal active ingredients whose mode of action is as auxin mimics or auxin inhibitors (antiauxins). Examples of auxinic herbicides include, but are not limited to, picloram (4-amino-3,5,6-trichloropicolinic acid); dicamba (3,6-dichloro-2-methoxybenzoic acid); clofibric acid ((p-chlorophenoxy)isobutyric acid); 2-(4-chlorophenoxy)-2-methylpropanoic acid); benazolin (4-chloro-2-oxo-3-benzothiazoleacetic acid; 4-chloro-2-oxobenzothiazolin-3-yl-acetic acid); TIBA (2,3,5-triiodobenozic acid); 2,3,6-TBA (2,3,6-trichlorobenzoic acid); triclopyr (3,5,6-trichloro-2-pyridyloxyacetic acid); quinclorac (3,7-dichloroquinoline-8-carboxylic acid); and the auxin-mimicking or auxin-blocking phenoxy herbicides, for example, phenoxyacetic, phenoxypropionic, and phenoxybutyric herbicides, including: 2,4-D ((2,4-dichlorophenoxy)acetic acid), MCPA ((4-chloro-2-methylphenoxy)acetic acid), 2,4-DB (4-(2,4-dichlorophenoxy)butyric acid), 2,4-DEP (tris[2-(2,4-dichlorophenoxy)ethyl] phosphate), 4-CPA (4-chlorophenoxyacetic acid), 2,4,5-T ((2,4,5-trichlorophenoxy)acetic acid), dichlorprop (2-(2,4-dichlorophenoxy)propanoic acid), fenoprop (2-(2,4,5-trichlorophenoxy)propanoic acid), and mecoprop (2-(2-methyl-4-chloro-phenoxy)propionic acid).

Examples of combinations of agricultural compositions include: imazapyr and imazapic; imazapyr and bentazon; imazapyr, imazapic, and bentazon; imazapyr and pyraclostrobin; imazapyr, imazapic, and pyraclostrobin; imazapyr and saflufenacil; imazapyr, imazapic, and saflufenacil; imazapic, saflufenacil, and glyphosate; imazapyr, imazapic, saflufenacil, and glyphosate; imazapic and glyphosate; imazapyr and glyphosate; imazapyr, saflufenacil, and glyphosate; and saflufenacil and glyphosate.

Prior to application, the AHAS-inhibiting herbicide can be converted into the customary formulations, for example solutions, emulsions, suspensions, dusts, powders, pastes and granules. The use form depends on the particular intended purpose; in each case, it should ensure a fine and even distribution of the compound according to the invention.

The formulations can be prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents for use in the formulations include water, aromatic solvents (for example Solvesso products, xylene), paraffins (for example mineral oil fractions), alcohols (for example methanol; butanol, pentanol, benzyl alcohol), ketones (for example cyclohexanone, gamma-butyrolactone), pyrrolidones (NMP, NOP), acetates (glycol diacetate), glycols, fatty acid diethylamides, fatty acids and fatty acid esters. Solvent mixtures can also be used.

Examples of suitable carriers for use in the formulations of the present invention include ground natural minerals (for example kaolins, clays, talc, chalk) and ground synthetic minerals (for example highly disperse silica, silicates).

Suitable emulsifiers for use in the formulations of the present invention include nonionic and anionic emulsifiers (for example polyoxyethylene fatty alcohol ethers, alkylsulfonates and arylsulfonates).

Examples of dispersants for use in the formulations of the present invention include lignin-sulfite waste liquors and methylcellulose.

Suitable surfactants for use in the formulations of the present invention include alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents for use in the formulations of the present invention include for example antifoaming agents based on silicon or magnesium stearate.

Suitable preservatives for use in the formulations of the present invention include, for example, dichlorophenol and benzylalcoholhemiformaldehyde.

Seed Treatment formulations of the present invention can additionally include binders and optionally colorants.

Binders can be added to the disclosed seed formuations to improve the adhesion of the active materials on the seeds after treatment. Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An example of a suitable gelling agent is carrageen (Satiagel®).

Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0:01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS-inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG)
      Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   I) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds.

In one embodiment a FS formulation is used for seed treatment. Typically, a FS formulation may comprise 1-800 g/L of active ingredient, 1-200 g/L Surfactant, 0 to 200 g/L antifreezing agent, 0 to 400 g/L of binder, 0 to 200 g/L of a pigment and up to 1 liter of a solvent, preferably water.

The present invention provides non-transgenic and transgenic seeds of the herbicide-resistant *Brassica* plants of the present invention. Such seeds include, for example, non-transgenic *Brassica* seeds comprising the herbicide-resistance characteristics of the plant of BnCL120C7 or BnCL131A1, and transgenic seeds comprising a nucleic acid molecule of the invention that encodes an herbicide-resistant AHASL protein.

For seed treatment, seeds of the herbicide resistant plants according to the present invention are treated with herbicides, preferably herbicides selected from the group consisting of AHAS-inhibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide.

The term seed treatment comprises all suitable seed treatment techniques known in the art, such as seed dressing, seed coating, seed dusting, seed soaking, and seed pelleting.

Further disclosed is a method of treating soil by the application, in particular into the seed drill: either of a granular formulation containing the AHAS-inhibiting herbicide as a composition/formulation, e.g., a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

The present invention also comprises seeds coated with or containing with a seed treatment formulation comprising at least one AHAS inhibitor selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid and pyrithiobac.

The term "seed" embraces seeds and plant propagules of all kinds including but not limited to true seeds, seed pieces, suckers, corms, bulbs, fruit, tubers, grains, cuttings, cut shoots and the like.

The term "coated with and/or containing" generally signifies that the active ingredient is for the most part on the surface of the propagation product at the time of application, although a greater or lesser part of the ingredient may penetrate into the propagation product, depending on the method of application. When the propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.1 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

Any herbicide formulation applied over the *Brassica* plants of the present invention can be prepared as a "tank-mix" composition. Each ingredient or a combination of ingredients can be stored separately from one another. The ingredients can then be mixed with one another prior to application. Typically, such mixing occurs shortly before application. In a tank-mix process, each ingredient, before mixing, typically is present in water or a suitable organic solvent. Methods and guidance for the preparation of such formulations are known in the art.

The methods further allow for the development of herbicide combinations to be used with the *Brassica* plants of the present invention. In such methods, the environmental conditions in an area of cultivation are evaluated. Environmental conditions that can be evaluated include, but are not limited to, ground and surface water pollution concerns, intended use of the crop, crop tolerance, soil residuals, weeds present in area of cultivation, soil texture, pH of soil, amount of organic matter in soil, application equipment, and tillage practices. Upon the evaluation of the environmental conditions, an effective amount of a combination of herbicides can be applied to the crop, crop part, seed of the crop or area of cultivation.

The herbicide applied to the *Brassica* plants of the present invention serves to prevent the initiation of growth of susceptible weeds or undesired plants and/or serve to cause damage to weeds or undesired plants that are growing in the area of interest. The herbicide or herbicide mixture exert these effects on weeds or undesired plants affecting crops that are subsequently planted in the area of interest (i.e., field or area of cultivation). In the methods, the application of the herbicide combination need not occur at the same time. So long as the field in which the crop is planted contains detectable amounts of the first herbicide and the second herbicide is applied at some time during the period in which the crop is in the area of cultivation, the crop is considered to have been treated with a mixture of herbicides according to the invention. Thus, the provided methods encompass applications of herbicide which are "preemergent," "postemergent," "preplant incorporation" and/or which involve seed treatment prior to planting.

In addition, methods are provided for coating *Brassica* seeds of the present invention. The methods comprise coating a seed with an effective amount of an herbicide or a combination of herbicides (as disclosed elsewhere herein). The seeds can then be planted in an area of cultivation. Further provided are *Brassica* seeds having a coating comprising an effective amount of an herbicide or a combination of herbicides (as disclosed elsewhere herein).

"Preemergent" refers to an herbicide which is applied to an area of interest (e.g., a field or area of cultivation) before a plant emerges visibly from the soil and/or before germination of seed. "Postemergent" refers to an herbicide which is applied to an area after a plant emerges visibly from the soil. In some instances, the terms "preemergent" and "postemergent" are used with reference to a weed or undesired plant in an area of interest, and in some instances these terms are used with reference to a crop plant in an area of interest. When used with reference to a weed or undesired plant, these terms may apply to only a particular type of weed or species of weed or undesired plant that is present or believed to be present in the area of interest. While any herbicide may be applied in a preemergent and/or postemergent treatment, some herbicides are known to be more effective in controlling a weed or weeds or undesired plants when applied either preemergence or postemergence. For example, rimsulfuron has both preemergence and postemergence activity, while other herbicides have predominately preemergence (metolachlor) or postemergence (glyphosate) activity. These properties of particular herbicides are known in the art and are readily determined by one of skill in the art. Further, one of skill in the art would readily be able to select appropriate herbicides and application times for use with the transgenic plants of the invention and/or on areas in which transgenic plants of the invention are to be planted. "Preplant incorporation" involves the incorporation of compounds into the soil prior to planting.

Thus, improved methods.of growing a crop and/or controlling weeds or undesired plants are provided such as, for example, "pre-planting burn down," where an area is treated with one or more herbicides prior to planting the crop of interest in order to better control weeds or undesired plants. Further provided are methods of growing a crop and/or controlling weeds or undesired plants which are "no-till" or "low-till" (also referred to as "reduced tillage"). In such methods, the soil is not cultivated or is cultivated less frequently during the growing cycle in comparison to traditional methods; these methods can save costs that would otherwise be incurred due to additional cultivation, including labor and fuel costs.

The methods encompass the use of simultaneous and/or sequential applications of multiple classes of herbicides. In some embodiments, the methods involve treating a plant of the invention and/or an area of interest (e.g., a field or area of cultivation) and/or weed and/or undesired plant with just one herbicide or other chemical such as, for example, an imidazolinone herbicide.

The time at which an herbicide is applied to an area of interest (and any plants therein) may be important in optimizing weed or undesired plant control. The time at which an herbicide is applied may be determined with reference to the size of plants and/or the stage of growth and/or development of plants in the area of interest, e.g., crop plants or weeds or undesired plants growing in the area. The stages of growth and/or development of plants are known in the art. Thus, for example, the time at which an herbicide or other chemical is applied to an area of interest in which plants are growing may be the time at which some or all of the plants in a particular area have reached at least a particular size and/or stage of growth and/or development, or the time at which some or all of the plants in a particular area have not yet reached a particular size and/or stage of growth and/or development.

The *Brassica* plants of the present invention show improved tolerance to postemergence herbicide treatments. For example, the *Brassica* plants of the present invention may be tolerant to higher doses of herbicide, tolerant to a broader range of herbicides (i.e., tolerance to more AHAS inhibitor chemistries), and/or may be tolerant to doses of herbicide applied at earlier or later times of development in comparison to an appropriate control plant.

Different chemicals such as herbicides have different "residual" effects, i.e., different amounts of time for which treatment with the chemical or herbicide continues to have an effect on plants growing in the treated area. Such effects may be desirable or undesirable, depending on the desired future purpose of the treated area (e.g., field or area of cultivation). Thus, a crop rotation scheme may be chosen based on residual effects from treatments that will be used for each crop and their effect on the crop that will subsequently be grown in the same area. One of skill in the art is familiar with techniques that can be used to evaluate the residual effect of an herbicide; for example, herbicides that act to inhibit AHAS vary in their residual activity levels. Residual activities for various herbicides are known in the art, and are also known to vary with various environmental factors such as, for example, soil moisture levels, temperature, pH, and soil composition (texture and organic matter). The *Brassica* plants of the present invention find particular use in methods of growing a crop where improved tolerance to residual activity of an herbicide is beneficial.

In addition, the *Brassica* plants of the present invention provide improved tolerance to treatment with additional chemicals used on crops in conjunction with herbicide treatments, such as safeners, adjuvants such as ammonium sulfonate, and crop oil concentrate, and the like.

In addition, the disclosed methods can comprise the use of an AHAS-inhibiting herbicide or a mixture of herbicides, as well as, one or more other insecticides, fungicides, nematocides, bactericides, acaricides, growth regulators, chemosterilants, semiochemicals, repellents, attractants, pheromones, feeding stimulants or other biologically active compounds or entomopathogenic bacteria, virus, or fungi to form a multi-component mixture giving an even broader spectrum of agricultural protection. Examples of such agricultural protectants that can be used in methods include: insecticides such as abamectin, acephate, acetamiprid, amidoflumet (S-1955), avermectin, azadirachtin, azinphos-methyl, bifenthrin, bifenazate, buprofezin, carbofuran, cartap, chlorfenapyr, chlorfluazuron, chlorpyrifos, chlorpyrifos-methyl, chromafenozide, clothianidin, cyflumetofen, cyfluthrin, beta-cyfluthrin, cyhalothrin, lambda-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim (UR-50701), flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, monocrotophos, methoxyfenozide, nitenpyram, nithiazine, novaluron, noviflumuron (XDE-007), oxamyl, parathion, parathion-methyl, permethrin, phorate, phosalone, phosmet, phosphamidon, pirimicarb, profenofos, profluthrin, pymetrozine, pyrafluprole, pyrethrin, pyridalyl, pyriprole, pyriproxyfen, rotenone, ryanodine, spinosad, spirodiclofen, spiromesifen (BSN 2060), spirotetramat, sulprofos, tebufenozide, teflubenzuron, tefluthrin, terbufos, tetrachlorvinphos, thiacloprid, thiamethoxam, thiodicarb, thiosultap-sodium, tralomethrin, triazamate, trichlorfon and triflumuron; fungicides such as acibenzolar, aldimorph, amisulbrom, azaconazole, azoxystrobin, benalaxyl, benomyl, benthiavalicarb, benthiavalicarb-isopropyl, binomial, biphenyl, bitertanol, blasticidin-S, Bordeaux mixture (Tribasic copper sulfate), boscalid/nicobifen, bromuconazole, bupirimate, buthiobate, carboxin, carpropamid, captafol, captan, carbendazim, chloroneb, chlorothalonil, chlozolinate, clotrimazole, copper oxychloride, copper salts such as copper sulfate and copper hydroxide, cyazofamid, cyflunamid, cymoxanil, cyproconazole, cyprodinil, dichlofluanid, diclocymet, diclomezine, dicloran, diethofencarb, difenoconazole, dimethomorph, dimoxystrobin, diniconazole, diniconazole-M, dinocap, discostrobin, dithianon, dodemorph, dodine, econazole, etaconazole, edifenphos, epoxiconazole, ethaboxam, ethirimol, ethridiazole, famoxadone, fenamidone, fenarimol, fenbuconazole, fencaramid, fenfuram, fenhexamide, fenoxanil, fenpiclonil, fenpropidin, fenpropimorph, fentin acetate, fentin hydroxide, ferbam, ferfurazoate, ferimzone, fluazinam, fludioxonil, flumetover, fluopicolide, fluoxastrobin, fluquinconazole, fluquinconazole, flusilazole, flusulfamide, flutolanil, flutriafol, folpet, fosetyl-aluminum, fuberidazole, furalaxyl, furametapyr, hexaconazole, hymexazole, guazatine, imazalil, imibenconazole, iminoctadine, iodicarb, ipconazole, iprobenfos, iprodione, iprovalicarb, isoconazole, isoprothiolane, kasugamycin, kresoxim-methyl, mancozeb, mandipropamid, maneb, mapanipyrin, mefenoxam, mepronil, metalaxyl, metconazole, methasulfocarb, metiram, metominostrobin/fenominostrobin, mepanipyrim, metrafenone, miconazole, myclobutanil, neo-asozin (ferric methanearsonate), nuarimol, octhilinone, ofurace, orysastrobin, oxadixyl, oxolinic acid, oxpoconazole, oxycarboxin, paclobutrazol, penconazole, pencycuron, penthiopyrad, perfurazoate, phosphonic acid, phthalide, picobenzamid, picoxystrobin, polyoxin, probenazole, prochloraz, procymidone, propamocarb, propamocarb-hydrochloride, propiconazole, propineb, proquinazid, prothioconazole, pyraclostrobin, pryazophos, pyrifenox, pyrimethanil, pyrifenox, pyroinitrine, pyroquilon, quinconazole, quinoxyfen, quintozene, silthiofam, simeconazole, spiroxamine, streptomycin, sulfur, tebuconazole, techrazene, tecloftalam, tecnazene, tetraconazole, thiabendazole, thifluzamide, thiophanate, thiophanate-methyl, thiram, tiadinil, tolclofos-methyl, tolyfluanid, triadimefon, triadimenol, triarimol, triazoxide, tridemorph, trimoprhamide tricyclazole, trifloxystrobin, triforine, triticonazole, uniconazole, validamycin, vinclozolin, zineb, ziram, and zoxamide; nematocides such as aldicarb, oxamyl and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological-agents including entomopathogenic bacteria, such as Bacillus thuringiensis subsp. Aizawai, Bacillus thuringiensis subsp. Kurstaki, and the encapsulated delta-endotoxins of Bacillus thuringiensis (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV. The weight ratios of these various mixing partners to other compositions (e.g., herbicides) used in the methods typically are between 100:1 and 1:100, or between 30:1 and 1:30, between 10:1 and 1:10, or between 4:1 and 1:4.

Further provided are compositions comprising a biologically effective amount of an AHAS-inhibiting herbicide of interest or a mixture of herbicides, and an effective amount of at least one additional biologically active compound or agent and can further comprise at least one of a surfactant, a solid diluent or a liquid diluent. Examples of such biologically active compounds or agents are: insecticides such as abamectin, acephate, acetamiprid, amidoflumet (S-1955), avermectin, azadirachtin, azinphos-methyl, bifenthrin, binfenazate, buprofezin, carbofuran, chlorfenapyr, chlorfluazuron, chlorpyrifos, chlorpyrifos-methyl, chromafenozide, clothianidin, cyfluthrin, beta-cyfluthrin, cyhalothrin, lambda-cyhalothrin, cypermethrin, cyromazine, deltamethrin, diafenthiuron, diazinon, diflubenzuron, dimethoate, diofenolan, emamectin, endosulfan, esfenvalerate, ethiprole, fenothicarb, fenoxycarb, fenpropathrin, fenvalerate, fipronil, flonicamid, flucythrinate, tau-fluvalinate, flufenerim (UR-50701), flufenoxuron, fonophos, halofenozide, hexaflumuron, imidacloprid, indoxacarb, isofenphos, lufenuron, malathion, metaldehyde, methamidophos, methidathion, methomyl, methoprene, methoxychlor, monocrotophos, methoxyfenozide, nithiazin, novaluron, noviflumuron (XDE-007), oxamyl, parathion, parathion-methyl, permethrin, phorate, phosalone, phosmet, phosphamidon, pirimicarb, profenofos, pymetrozine, pyridalyl, pyriproxyfen, rotenone, spinosad, spiromesifin (BSN 2060), sulprofos, tebufenozide, teflubenzuron, tefluthrin, terbufos, tetrachlorvinphos, thiacloprid, thiamethoxam, thiodicarb, thiosultap-sodium, tralomethrin, trichlorfon and triflumuron; fungicides such as acibenzolar, azoxystrobin, benomyl, blasticidin-S, Bordeaux mixture (tribasic copper sulfate), bromuconazole, carpropamid, captafol, captan, carbendazim, chloroneb, chlorothalonil, copper oxychloride, copper salts, cyflufenamid, cymoxanil, cyproconazole, cyprodinil, (S)-3,5-dichloro-N-(3-chloro-1-ethyl-1-methyl-2-oxopropyl)-4-methylbenzam- ide (RH 7281), diclocymet (S-2900), diclomezine, dicloran, difenoconazole, (S)-3,5-dihydro-5-methyl-2-(methylthio)-5-phenyl-3-(phenyl-amino)-4H-imid- azol-4-one (RP 407213), dimethomorph, dimoxystrobin, diniconazole, diniconazole-M, dodine, edifenphos, epoxiconazole, famoxadone, fenamidone, fenarimol, fenbuconazole, fencaramid (SZX0722), fenpiclonil, fenpropidin, fenpropimorph, fentin acetate, fentin hydroxide, fluazinam, fludioxonil, flumetover (RPA 403397), flumorf/flumorlin (SYP-L190), fluoxastrobin (HEC 5725), fluquinconazole, flusilazole, flutolanil, flutriafol, folpet, fosetyl-aluminum, furalaxyl, furametapyr (S-82658), hexaconazole, ipconazole, iprobenfos, iprodione, isoprothiolane, kasugamycin, kresoxim-methyl, mancozeb, maneb, mefenoxam, mepronil, metalaxyl, metconazole, metomino-strobin/fenominostrobin (SSF-126), metrafenone (AC375839), myclobutanil, neo-asozin (ferric methane-arsonate), nicobifen (BAS 510), orysastrobin, oxadixyl, penconazole, pencycuron, probenazole, prochloraz, propamocarb, propiconazole, proquinazid (DPX-KQ926), prothioconazole (JAU 6476), pyrifenox, pyraclostrobin, pyrimethanil, pyroquilon, quinoxyfen, spiroxamine, sulfur, tebuconazole, tetraconazole, thiabendazole, thifluzamide, thiophanate-methyl, thiram, tiadinil, triadimefon, triadimenol, tricyclazole, trifloxystrobin, triticonazole, validamycin and vinclozolin; nematocides such as aldicarb, oxamyl and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents including entomopathogenic bacteria, such as Bacillus thuringiensis subsp. Aizawai, Bacillus thuringiensis subsp. Kurstaki, and the encapsulated delta-endotoxins of Bacillus thuringiensis (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV. Methods may also comprise the use of plants genetically transformed to express proteins toxic to invertebrate pests (such as Bacillus thuringiensis delta-endotoxins). In such embodiments, the effect of exogenously applied invertebrate pest control compounds may be synergistic with the expressed toxin proteins.

Thus, the methods can employ an AHAS-inhibiting herbicide or AHAS-inhibiting herbicide combination and may further comprise the use of insecticides and/or fungicides, and/or other agricultural chemicals such as fertilizers. The use of such combined treatments can broaden the spectrum of activity against additional weed species and suppress the proliferation of any resistant biotypes.

The present disclosure provides a method for combating undesired vegetation or controlling weeds comprising contacting the seeds of the resistant plants according to the present invention before sowing and/or after pregermination with an AHAS-inhibiting herbicide. The method can further comprise sowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds include, for example, dicotyledonous and monocotyledonous weeds. Dicotyledonous weeds include, but are not limited to, weeds of the genera: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus,* and *Taraxacum.* Monocotyledonous weeds include, but are not limited to, weeds of the genera: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus,* and *Apera.*

In addition, the weeds can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element" means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation.

### Example 1 - Preparation of Mutant AHAS Sequences

Prior to creating the mutant sequences, the full coding sequences for *Brassica napus* AHAS I and III were amplified using BnALS3(5' CTAACCATGGCGGCGGCAAC (SEQ ID NO: 1)) and BnALSOR2 (5'-AGTCTGGGAACAAACCAAAAGCAGTACA (SEQ ID NO: 2)) and BnALSOR3 (5'-CGTCTGGGAACAACCAAAAGTAGTACAA (SEQ ID NO: 3)) respectively, cloned and sequenced from lines BN-02 (Figures 3 and 4), line 97 (BN-02 with the S653N in AHAS III, Figures 3 and 3) and BN-11 to serve as reference sequences for comparative purposes.

### 1A. Gene Repair Oligonucleotide (GRON) design

Based on the AHAS III nucleic acid sequence obtained, a series of gene repair oligonucleotides (GRONs, shown in Table 1) were designed to specifically introduce a nucleic acid mutation in the AHAS coding region that results in an alanine to threonine amino acid substitution corresponding to position 122 in the encoded AHAS III protein, based on the amino acid numbering for the *Arabidopsis* AHAS. The GRONs were designed to introduce a GCT to ACT nucleotide change in AHAS III. Only the AHAS III nucleic acid sequence is targeted for this change using these GRON sequences.

**Table 1. GRON sequences.**

| **GRON** | **Sequence** | **SEQ ID NO:** |
|---|---|---|
| BnALS1122/C/42/5'Cy3/ 3'idC | | 4 |
| BnALS1122/NC/42/5'Cy3/ 3'idC | | 5 |

The converting base is shown in bold. V=CY3; H=3'DMT dC CPG

### 1B. Cell Culture Work Description

### 1B.1 Gene Repair Oligonucleotide (GRON) introduction experiments targeting the A122T mutation in the AHAS III gene of spring Canola Line 97

Canola Line 97 was derived from previous Rapid Trait Development System™ (Cibus LLC, San Diego, CA, *"RTDS^{™}"*) experiments using leaf protoplasts from haploid, microspore-derived plantlets of the *Brassica* cultivar known as BN-02. Line 97 contains a mutation at a position corresponding to amino acid position 653 in the AHAS III protein. The mutation is encoded by the codon change AGT to AAT and results in a serine to asparagine substitution, which provides tolerance to imazethapyr. The addition of the A122T mutation in the same gene resulted in greatly enhanced Imi-tolerance compared to the single mutation.

*RTDS^{™}* experiments were performed aimed at introducing the additional mutation in the AHAS III gene of Line 97. Line 97 was regenerated from protoplast-derived callus that had been selected with 0.5 and 0.2 µM Imi. The A122T;S653N double mutation was selected at the concentration of 10 µM Imi. The protocol that was used for the experiments is described in Sections 1B.2-1B.6.

### 1B.2 Propagation of material for isolation of leaf protoplasts

Haploid shoots derived from microspore-derived embryos were propagated under sterile conditions *in vitro.* Cuttings were subcultured every 2 - 4 weeks and cultured in petri dishes (90 mm x 20 mm) that contain a volume of 40 - 45 mL RS medium (Dovzhenko A., 2001). The dishes were sealed with Micropore tape (3M Company). Young leaves were excised and used for protoplast isolation.

### 1B.3 Protoplast isolation and purification

About 600 mg of leaf tissue from 2 - 3 week-old *in vitro* shoots was cut into small strips with a scalpel in a petri dish with 5 mL medium B (Pelletier et al., 1983), pH adjusted to 5.8. After 1 h, medium B was replaced with 12 mL of a filter-sterilized enzyme solution, consisting of medium B in which 0.5% Cellulase YC and 0.75% Macerozyme R10 (both from Karlan Research Products, Cottonwood, Arizona), 1 g/L bovine serum albumin, and 1 g/L 2-morpholinoethanesulfonic acid are dissolved. The enzyme solution was vacuum-infiltrated, and subsequently the dish with leaf pieces in enzyme solution was incubated for 16 - 18 h at 25°C in darkness. Protoplast purification was performed using an iodixanol density gradient. After the density gradient centrifugation, the band with purified protoplasts was removed together with about 5 mL W5 medium. Protoplast yield was determined using a hemocytometer, and the protoplasts were stored for 2 h at 4°C.

### 1B.4 Gene Repair Oligonucleotide (GRON) introduction

The protoplast suspension was mixed with an equal volume of cold medium W5, transferred to a 50 mL centrifuge tube, and centrifuged for 10 min at the lowest setting of a clinical centrifuge (about 50 x g). The supernatant was removed and replaced with TM medium (Klaus, S. 2003), adjusting the protoplast density to 5 x 10⁶/mL. Aliquots of 500 µL containing 2.5 x 10⁶ protoplasts each were distributed into 50 mL centrifuge tubes. The GRON (e.g. BnALS1122/C/41/5'Cy3/3'idC; see Table 1) was delivered to the protoplasts by mixing it with polyethylene glycol (PEG) and mixing it on a rotator on ice. Approximately 12 - 300 µg of GRON is used per 500 µL treatment volume. The protoplasts were then collected using centrifugation and stored overnight at 4°C in the dark.

### 1B.5 Embedding of protoplasts in calcium alginate

The protoplasts were then embedded in a gel (e.g., agarose, alignate) based on the method described in Dovzhenko (2001). The embedding of protoplasts in gel substrates has been shown to enhance protoplast survival and to increase division frequencies of protoplast-derived cells. One day after the PEG-GRON treatment, the protoplasts derived from one treated sample were transferred to a 50 mL centrifuge tube and allowed to reach room temperature. The tube was centrifuged for 10 min at the lowest setting (about 50 x g) with a clinical centrifuge. The pellet was resuspended in 2.75 mL medium B (calcium chloride dihydrate concentration reduced to 131 mg/L) and mixed with 2.75 mL of a 2.8% (w/v) solution of sodium alginate in the same reduced-calcium medium B in a V-shaped 60 mL reagent basin. The protoplast-alginate suspension was taken up with a multi-channel pipette (using precut tips to provide a wider opening), and 40 µL aliquots were dropped into 50 mL Ca-A medium.

### 1B.6 Protoplast culture and selection of Imi-tolerant calli

Imidazolinone-tolerant calli were selected using sequential subcultures of the alginate beads in media similar to those described by Pelletier et al. (1983) Mol Gen. Genet. 191:244-250 (see Table 2). Selection was started one week after the PEG/GRON treatment at a concentration of 10 µM Imi. The addition of herbicide to the culture medium did not have an immediate effect. Initially, all microcolonies that had formed during the early culture phase without imidazolinone continued to grow, but slower than controls without added herbicide. One to two weeks after the onset of selection, growth of the majority of colonies slowed down or stopped.

Cells and microcolonies were released from the alginate three weeks after the embedding of protoplasts by treating them for 30 - 45 min with culture medium containing 50 mM sodium citrate. Upon release, most colonies were either dead, or consist of a greenish center, covered with outer layers of dead cells. After transfer to solidified selection medium E, the majority of microcalli that still contained living cells stopped growing and turned brownish. Limited growth of individual calli continued, but all non-tolerant calli eventually turned brown. Two to three weeks after the transfer to solidified selection medium (occasionally earlier), actively growing calli appeared among a background of brownish cells and microcalli.

**Table 2. Protocol for the selection of imazethapyr-tolerant calli, starting with one PEG/GRON-treated sample of 2.5 x 10⁶ protoplasts.**

| **Day** | **Step** |
|---|---|
| -1 | Leaves to enzyme solution. |
| 0 | Purification of protoplasts and PEG/GRON treatment, overnight in medium B at 4°C. |
| 1 | Embedding of protoplasts in alginate; culture about 5 mL of 40 µL beads in 20 mL medium B in 20 mm x 150 mm petri dish. |
| 8 | Replace half of the liquid medium with medium C; add Imi at 10 µM. |
| 15 | Replace liquid medium with fresh medium C containing Imi at 10 µM. |
| 22 | Release cells from alginate; culture in fresh medium C containing Imi at 10 µM. |
| 29 | Replace liquid medium with fresh medium C, containing Imi at 10 µM. |
| 36 | Transfer cells/microcalli to agar-solidified medium E, Imi 10 µM, in a 145 x 20 mm petri dish. |
| 50 - 57 | Identify growing calli, transfer again to agar-solidified medium E, Imi 10 µM. |
| 3 - 4 wks later | Identify growing calli for subculture and analysis; discard non-growing calli. |

### 1B.7 Regeneration of plants from protoplast-derived, Imi-tolerant calli containing the A122T;S653N double mutation in the AHAS III gene

Imi-tolerant calli that had developed on solidified selection medium and that had been confirmed to have the targeted mutation (see Example 1C, below) were transferred to herbicide-free medium E to accelerate development. Individual callus lines varied in their growth rates and morphologies. In general, the development towards shoot regeneration follows the steps of:

Undifferentiated, green callus -> callus with dark green areas -> development of roots -> development of shoot initials -> development of stunted shoots with hyperhydric (vitrified) leaves.

The development of an individual callus line was variable, but through continuous subculture and multiplication on medium E or modifications of medium E with lower concentrations of NAA the chances of obtaining shoot formation events were increased.

Once shoots had formed on medium E and had formed three to four leaves, they were transferred to RS medium (Dovzhenko, 2001). On this medium, over time newly formed shoot and leaf tissue developed that was morphologically 'normal' (i.e., non-hyperhydric). Subsequently, standard protocols were used for hardening of plantlets and for transfer to the greenhouse.

### 1C. Molecular Screening

Genomic DNA from callus showing tolerance to imazethapyr was screened for the presence of the introduced A122T mutation. Genomic DNA was extracted using the procedure of Edwards et al. (1991) Nucleic Acids Research 19(6): 1349*.* Since AHAS I and III do not contain any introns, PCR from genomic DNA was possible. Gene specific (GS) primers BnALS3 (5'-CTAACCATGGCGGCGGCAAC (SEQ ID NO: 1)) and BnALS9 (5'-CCCATCAACGTACTCGCACCA (SEQ ID NO: 6)), were used to amplify the region containing the A122 position in AHAS III. Following purification of the resulting amplicon and quantification, the resulting amplicon was used as a template for an allele-specific (AS) PCR reaction. A three-primer mix (BnALSOF5 (5'-GTCAATGTCGCACCTGAAAAAACCGACA (SEQ ID NO: 7)), BnALSOR8 (5'-GCTAACCCGCTGACGAGGTTGGTAGCT (SEQ ID NO: 8)) and BnALSIR2A (5'-AAGGCTTGGTGGATCTCCATGGACGT (SEQ ID NO: 9)) - Master-mix 40) was used to detect the presence of the A122T mutation. The inner primer (BnALS1R2A) was designed such that it will only anneal and form a product, pairing with BnALSOF5 if the ACT (threonine) codon is present.

The gene-specific PCR was repeated for all calli showing the A122T change, using the same genomic DNA template as was used in the AS-PCR screen. This time, the amplicon was gel-purified from a 1% Tris-Acetate EDTA gel and sequenced over the A122 region, to confirm the change. Upon confirmation, an independent genomic DNA sample was prepared from callus tissue and the A 122 region was sequenced once again.

Once two independent samples from a given callus line had been confirmed for the presence of the A122T mutation, AHAS genes I and III were sequenced in their entirety to check for any other unintended single nucleotide polymorphisms (SNP's). The full length genes were amplified using BnALS3 (5'-CTAACCATGGCGGCGGCAAC (SEQ ID NO: 1)) with BnALSOR2 (5'-AGTCTGGGAACAAACCAAAAGCAGTACA (SEQ ID NO: 2)) (for gene III) and BnALSOR3 (5'- CGTCTGGGAACAACCAAAAGTAGTACAA (SEQ ID NO: 3)) (for gene I) and cloned into TopoPCR2.1 (Invitrogen). Four clones of each gene were sequenced to distinguish PCR error from genuine SNP's.

Genomic DNA was extracted using a Bio-sprint machine (Qiagen) from plant tissue regenerated from the screened calli and was screened again to confirm the presence of the mutations. Gene-specific PCR (GS-PCR) was performed using primers to amplify the regions containing the A122T (BnALS3 (5'-CTAACCATGGCGGCGGCAAC (SEQ ID NO: 1)) and BnALS9 (5'-CCCATCAACGTACTCGCACCA (SEQ ID NO: 6)) and the S653N (BnALS10 (5'-GAGGCTTTCGCTAGCAGGGCTCAA (SEQ ID NO: 10)) and BnALSOR2 (5'-AGTCTGGGAACAAACCAAAAGCAGTACA (SEQ ID NO: 2))) sequences with the line 97 background plants, in gene III, to confirm their presence.

A total of 18 callus lines were identified in which the presence of both the A122T mutation and the S653N mutation were confirmed by sequencing of the AHAS III gene. Twelve of these callus lines regenerated shoots. Excepting one line, the double mutant lines were homozygous (i.e., haploid or homozygous diploid) for both the A122T and S653N mutations in AHAS III. The most advanced line, BnCL131A1, produced several grams of seeds. The yield per plant was comparable to that of a diploid plant, which means that at some point during the selection/regeneration spontaneous diploidization had occurred. Pollen of this line was used to fertilize ovaries of two winter oilseed rape (WOSR) cultivars, BN-11 and BN-19. In addition, seeds from the BnCL131A1 line were deposited with the ATCC as Accession Number PTA-9279. Two additional lines, BnCL140B3 and BnCL140C7, both containing the mutagenized AHAS nucleic acid molecule encoding the A122T and S653N mutations in AHAS III were also deposited as Accession Nos: PTA-9402 and PTA-9403, respectively.

Another *Brassica* line containing only the A122T mutation in the AHAS III sequence was also identified. Seeds of this line, BnCL120C7, were also deposited with the ATCC as Accession No. PTA-9278.

### Example 2: AHAS Enzymatic Activity from B. napus Plant Extracts and Whole Plants

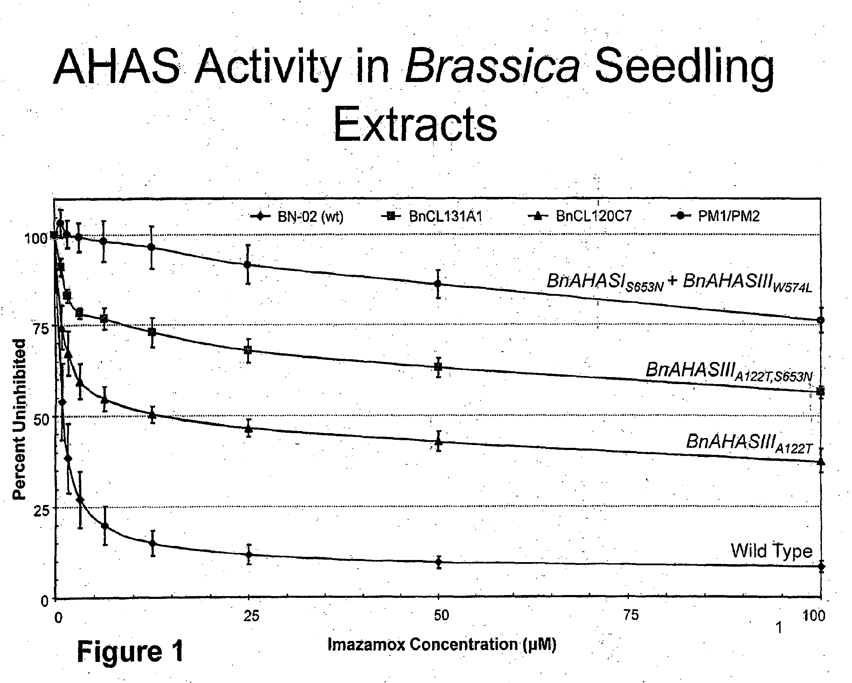

Plants for lines BN02 (wild type), PM1/PM2 (two mutant genes, See, e.g. U.S. Patent No. 7,355,098), BnCL131A1 (AHAS III A122T S653N double mutant) and BnCL120C7 (AHAS III A122T single mutant) were grown from seed until the 3-4 leaf stage before being harvested. Cell extracts were assayed in triplicate for AHAS activity according to Singh et al., (1988 Assay of Acetohydroxyacid Synthase. Analytical Biochemistry 171, 173-179.) in the absence or presence of 8 serial dilutions of imazamox ranging from 100-0.78 µM. Activity is expressed as the percentage of the uninhibited (no imazamox) activity (Figure 1). The graph shown in Figure 1 depicts the average percent uninhibited results for each imazamox concentration from three planting dates each with 2-3 replicates for a total of 7 independent extractive assays. Error bars represent ± two standard error of the mean.

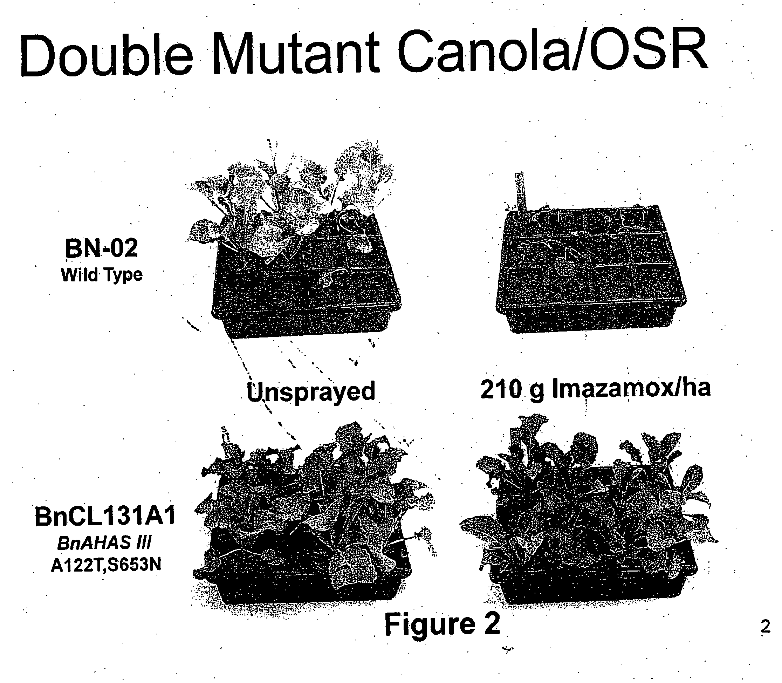

In addition, *B. napus* plants (3-4 leaf stage) containing the double mutant were analyzed for response to treatment with imazamox compared to wild type plants. Lines BN02 (wild-type) and BnCL131A1 (AHAS III A122T:S653N, double mutant) were treated with either 210 g ai/ha imazamox supplemented with 0.5% v/v Merge by foliar spray or left unsprayed as checks. The treated plants were evaluated three (3) weeks after treatment, and the results are presented in Figure 2. Figure 2 provides the results for plants BN02 (top row) and BnCL131A1 (bottom row). As can be seen in Figure 2, wild type BN-02 plants were nearly non-tolerant of the 210 g ai/ha application of imazamox, while the BnCL131A1 line was almost completely tolerant to such application.

*B. napus* plants containing the double mutant were also tested for plant injury due to imidazolinone herbicide application. *B. napus* plants for lines BN02 (wild type), BnCL131A1 (AHAS III A122T:S653N double mutant), BnCL120C7 (AHAS III A122T single mutant), BnCL97 (AHAS III S653N single mutant), PM1/PM2 (commercial two gene check), PM1 (AHAS I S653N single mutant) and PM2 (AHAS III W574L single mutant) were grown from seed until 3-4 leaf stage then either left unsprayed or sprayed with 35, 70 or 210 g ai/ha imazamox supplemented with 0.5% v/v Merge. Herbicide injury was evaluated 2 weeks after treatment (DAT) on 8-24 plants per line using a 0 to 9 scale where 0 is no injury and 9 is plant death. The results are presented as mean injury ± one standard error of the mean in Table 3 below. The results presented cover two independent plantings and sprays for all lines except BnCL97. The table also presents values for ± one standard error of the mean.

**Table 3**

| | | Spray Rate [g ai/ha] | | | |
|---|---|---|---|---|---|
| Line | | 0 | 35 | 70 | 210 |
| BN-02 (wild type) | A | 0.06 ±0.06 | 9.00 ±0.00 | 9.00 ±0.00 | 9.00 ±0.00 |
| BnCL131A1 (BnAHAS III A122T:S653) | B | 0.00 ±0.00 | 0.76 ±0.33 | 2.53 ±0.24 | 3.22 ±0.33 |
| BnCL120C7 (BnAHASIII A122T) | C | 0.17 ±0.08 | 4.19 ±0.39 | 5.43 ±0.24 | 6.65 ±0.30 |
| BnCL97 (BnAHASIII S653N) | D | 0.17 ±0.11 | 4.08 ±0.15 | 8.58 ±0.15 | 8.00 ±0.23 |
| PM1/PM2 | E | 0.07 ±0.07 | 1.21 ±033 | 4.13 ±0.36 | 4.93 ±0.53. |
| PM2 (BnAHASIII W574L) | F | 0.30 ±0.15 | 1.81 ±0.25 | 4.14 ±0.23 | 6.17 ±0.22 |
| PM1 (BnAHASI S653N) | G | 0.11 ±0.07 | 7.22 ±0.18 | 8.61 ±0.12. | 9.00 ±0.00 |

### Example 3: Herbicide Injury Rating for Brassica napus Plants Treated with Imazamox

Herbicide Injury Rating for *Brassica napus* Plants Treated with Imazamox. Plants for lines BN02 (wild type), two lines of BnCL131A1 (AHAS III A122T S653N double mutant; BnCL131A1.0 and BnCL131A1.18), PM1/PM2 (commercial two gene check: AHAS I S653N & AHAS III W574L) were grown from seed until 3-4 leaf stage then either left unsprayed or sprayed with 35, 70 or 210 g ai/ha imazamox supplemented with 0.5% v/v Merge. Herbicide injury was evaluated 2 weeks post spray on 12 plants per line. The results are presented as mean injury ± one standard error of the mean in Table 4 below. The results cover one planting and spray for all lines and the values for ± two standard error of the mean are also provided.

**Table 4**

| | | Spray Rate [g ai/ha] | | | | |
|---|---|---|---|---|---|---|
| Line | | 0 | 35 | 70 | 210 | 420 |
| BN-02 (wild type) | A | 0.17 ±0.11 | 8.92 ±0.08 | 9.00 ±0.00 | 9_{:}00 ±0.00 | 9.00 ±0.00 |
| PM1/PM2 | B | 0.17 ±0.17 | 0.50 ±0.19 | 1.83 ±0.21 | 2.00 ±0.23 | 3.83 ±0.21 |
| BnCL131A1.0 | C | 0.00 ±0.00 | 0.90 ±0.28 | 1.20 ±0.29 | 1.45 ±0.28 | 3.20 ±0.20 |
| BnCL131A1.18 | D | 0.08 ±0.08 | 0.64 ±0.20 | 0.91 ±0.21 | 1.89 ±0.20 | 2.27 ±0.14 |

### Example 4 Imazamox Herbicide Injury Ratings of Field Tested Brassica napus Plants

*B. napus* plants from the following spring oilseed rape lines: BN-02 (wild type), BN-T (wild type), anCL131A1 in a BN-02 background genotype (AHAS III A122T S653N double mutant), BnCL120C7 in a BN-02 background genotype (BnAHASIII A122T), BnCL97 in a BN-02 background genotype (BnAHASIII S653N), PM1/PM2 in a BN-T background (two gene check: AHAS I S653N & AHAS III W574L), PM1 in a BN-T background (one gene check: AHAS I S653N) and PM2 in a BN-T background (one gene check: AHAS III W574L) were field tested at a site near Minot, North Dakota. Lines were planted in 5 m long single row plots and arranged in a two factorial split plot design with 3 repetitions and 4 treatments (0 g ai/ha imazamox, 50 g ai/ha imazamox, 100 g ai/ha imazamox and 200 g ai/ha imazamox). Plants were sprayed at the 2-4 leaf stage with the above mentioned imazamox treatments. The imazamox product (Beyond 120 g/L LC + 0.25% (v/v) non-ionic surfactant) was diluted in 20 gallons per acre (or 200 liters/ha) and applied using a tractor mounted boom.

Herbicide injury was evaluated 15 days after treatment (DAT) on a per plot basis using a scale from 0 to 100%, where 0 was no injury and 100% was complete plant death. The injury ratings were analyzed statistically using an ANOVA and the results are presented in Table 5.

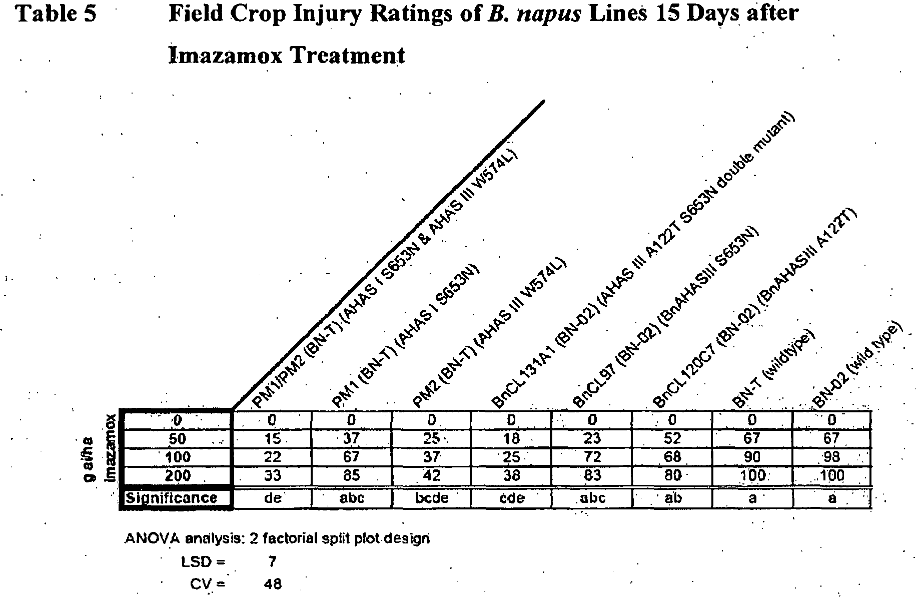

The double mutant line, BnCL131A1 (BN-02) (AHAS III A122T, S653N), demonstrated a much greater herbicide tolerance than the sum of the individual mutant lines BnCL97 (BN-02) (AHASIII S653N) and BNCL120C7 (BN-02) (AHASIII A122T). The amount of tolerance demonstrated by the double mutant line (A122T + S653N) at 200 g ai/ha of imazamox is 62% (100% - 38% crop injury). The amount of tolerance demonstrated by the A122T single mutant at 200 g ai/ha is 20% (100% - 80% crop injury). The amount of tolerance demonstrated by the S653N single mutant at 200 g ai/ha is 17% (100% - 83% crop injury). If the tolerance were additive, then the expected tolerance of combining the A122T mutation together with the S653N mutation would be 37%. Instead, the observed tolerance of the double mutant was 62%. The level of herbicide tolerance observed in the double mutant line, BNCL131A1(BN-02), is therefore synergistic to that observed in the two single mutant lines, BNCL97 (BN-02) and BNCL120C7 (BN-02).

### Example 5 Brassica plants containing stacked AHAS Traits and Imazamox Herbicide Injury Ratings

*Brassica napus* plants containing stacked AHAS-inhibitor resistant nucleic acid molecules were obtained.by conventional breeding methodologies, by crossing an AHAS-inhibitor resistant line homozygous for CLB-1 (*AHASL1A-*A122(*At*)T-S653(*At*)N) with an AHAS-inhibitor resistant line homozygous for *AHASLIA-*W574(*At*)L (PM2) + *AHASL1C*- S653(*At*)N (PM1).

Hand-crossed hybrids were produced in the greenhouse between two IMI-tolerant spring *Brassica napus* parental lines: "BnCL131A1" (homozygous for the A genome IMI-tolerant mutation *AHASL1A*-A122(*At*)T-S653*(At)*N) and "PM1PM2", (homozygous for the A genome AHAS-inhibitor tolerant mutation *AHASLIA-*W574(*At*)L (PM2) plus the C genome AHAS-inhibitor tolerant mutation *AHASLIC-*S653(*At*)N (PM1)). The resulting heterozygous hand-crossed hybrid line, "PM1PM2/BnCL131A1" (heterozygous for the stacked mutations: *AHASL1A-*A122(*At*)T-S653(*At*)N, *AHASL1A*-W574(*At*)L(PM2), *AHASL1C-* S653*(At*)N (PM1)) was field tested together with lines each containing only one AHAS-inhibitor tolerant-mutation (homozygous or heterozygous) and several spring *Brassica napus* checks.

The three repetition trial was conducted in Simcoe, North Dakota, and used a randomized split block design. The trial was designed to measure the percent phytotoxicity (% crop injury) of the different entries under 5 different imidazolinone herbicide treatment rates. Plants were sprayed at the 2-4 leaf stage at a volume of 100 liters/ha. The imidazolinone herbicide rates used were as follows (Imazamox = BEYOND™ 120 g/I LC (BAS 72001H); NIS = INDUCE™ 90 (90% non-ionic surfactant; Helena Chemical Co., Collierville, TN):

| **Trtmnt No.** | **Treatment Rate** |
|---|---|
| **1** | **0** |
| **2** | **35 g ai/ha imazamox + 0.25% (v/v) NIS** |
| **3** | **70 g ai/ha imazamox + 0.25% (v/v) NIS** |
| **4** | **140 g ai/ha imazamox + 0.25% (v/v) NIS** |
| **5** | **210ai/ha imazamox + 0.25% (v/v) NIS** |

The hand-crossed hybrid line, PM1PM2/BnCL131A1 contained 3 heterozygous AHAS-inhibitor tolerant mutant alleles:
*AHASL1A*-A122(*At*)*T-S653(At)*N
AHASL1A-W574(At)L
AHASLIC-S653(At)N

the comparator line BnCL131A1 (He) contained 1 heterozygous AHAS-inhibitor tolerant mutant allele:
*AHASLIA-*A122(*At*)T-S653(*At*)N

the comparator line PM2 (AHASL1A-PM2) contained 1 homozygous AHAS-inhibitor tolerant mutant allele:
AHASL1A-W574(At)L

and the comparator line PM1 (AHASL1C-PM1) contained 1 homozygous AHAS-inhibitor tolerant mutant allele:
*AHASL1C*-S6S3(*At*)N.

Crop Injury Ratings were determined in the field by assessing each plot in the trial for the level of phytotoxicity (percent crop injury) on a scale from 0 - 100% at 18 days after treatment. Mean values for each entry across the three replications along with their statistical significance (ANOVA) are presented in Table 6 (Ho = homozygous; He = heterozygous). Each value presented in Table 6 is the mean of three replications.

**Table 6. The Mean Percent Crop Phytotoxicity (% Crop Injury) Observed at 18 Days After Treatment.**

| | | | | **Imazamox g ai/ha** | | | | | | |
|---|---|---|---|---|---|---|---|---|---|---|
| **Line ID** | **Line Description** | **Mutation (Zygosity)** | **0** | **35** | **70** | **140** | **210** | **Mean** | **N** | **Sig.** |
| ID-13257 | PM1PM2 / BnCL131A1 | S653N (He),W574L (He)/ A122T-S653N (He) | 0 | 8.33 | 15 | 21.67 | 15 | 12 | 15 | G |
| ID-13248 | BnCL131A1 (Ho) | A122T-S653N (Ho) | 0 | 16.67 | 31.67 | 43.33 | 55 | 29.33 | 15 | F |
| ID-13249 | BnCL131A1 (He) | A122T-S653N (He) | 0 | 33.33 | 60 | 80 | 73.33 | 49.33 | 15 | DE |
| ID-13278 | PM2 (AHASL1A-PM2) | W574L (Ho) | 0 | 36.67 | 46.67 | 83.33 | 80 | 49.33 | 15 | DE |
| ID-13264 | AHASL1A-A122T | A122T(Ho) | 0 | 70 | 78.33 | 66.67 | 70 | 57 | 15 | CD |
| ID-13263 | AHASL1A-S653N | S653N (Ho) | 0 | 66.67 | 78.33 | 86.67 | 90 | 64.33 | 15 | C |
| ID-12786 | PM1 (AHASL1C-PM1) | S653N (Ho) | 0 | 66.67 | 86.67 | 91.67 | 91.67 | 67.33 | 15 | BC |
| ID-13162 | Bn WT1 | - | 0 | 85 | 96.67 | 100 | 98.33 | 76 | 15 | AB |
| ID-13254 | Bn WT2 | - | 0 | 95 | 100 | 100 | 100 | 79 | 15 | A |
| | | | | | | | | | | |
| | | **Mean** | | 43.93 | 53.33 | 62.38 | 62.86 | 44.5 | 210 | |
| | | **N** | | 42 | 42 | 42 | 42 | 210 | | |
| | | **Sig.** | | C | B | AB | A | | | |

### Results

The percent phytotoxicity (% crop injury) ratings were transposed into Herbicide Tolerance ratings by subtracting the percent crop injury rating (Table 6) for each line from 100% to obtain the percent herbicide tolerance (Table 7) (Ho = homozygous; He = heterozygous).

**Table 7. The Mean Percent Herbicide Tolerance Observed at 18 Days After Treatment (Transposed from Table 1: Percent Phytotoxicity, Table 1).**

| | | | **Beyond g ai/ha** | | | | | | |
|---|---|---|---|---|---|---|---|---|---|
| **Variety** | **Line Description** | **Mutation + Zygosity** | 0 | 35 | 70 | 140 | 210 | **N** | **Sig.** |
| ID-13257 | PM1PM2 / BnCL131A1 | S653N (He),W574L (He) /A122T-S653N (He) | 100 | 91.7 | 85.0 | 78.3 | 85.0 | 15 | G |
| ID-13248 | BnCL131A1 (Ho) | A122T-S653N (Ho) | 100 | 83.3 | 68.3 | 56.7 | 45.0 | 15 | F |
| ID-13249 | BnCL131A1 (He) | A122T-S653N (He) | 100 | 66.7 | 40.0 | 20.0 | 26.7 | 15 | DE |
| ID-13278 | PM2 (AHASL1A -PM2) | W574L (Ho) | 100 | 63.3 | 53.3 | 16.7 | 20.0 | 15 | DE |
| ID-13264 | AHASL1A-A122T | A122T (Ho) | 100 | 30.0 | 21.7 | 33.3 | 30.0 | 15 | CD |
| ID-13263 | AHASL1A-S653N | S653N (Ho) | 100 | 33.3 | 21.7 | 13.3 | 10.0 | 15 | C |
| ID-12786 | PM1 (AHASL1C-PM1) | S653N (Ho) | 100 | 33.3 | 13.3 | 8.3 | 8.3 | 15 | BC |
| ID-13162 | Bn WT1 | - | 100 | 15.0 | 3.3 | 0.0 | 1.7 | 15 | AB |
| ID-13254 | Bn WT 2 | - | 100 | 5.0 | 0.0 | 0.0 | 0.0 | 15 | A |
| | | | | | | | Beyond g ai/ha | | |
| | | | | | | | 140 | 210 | |
| Expected Additive % Herbicide Tolerance: | | | | | | | | | |
| ID-13249 | BnCL131A1 (He) | A122T-S653N (He) | | | | | 20.0 | 26.7 | |
| ID-13278 | PM2 (AHASL1A -PM2) | W574L (Ho) | | | | | 16.7 | 20.0 | |
| ID-12786 | PM1 (AHASL1C-PM1) | S653N (Ho) | | | | | 8.3 | 8.3 | |
| | | | | | Additive %Herbicide Tolerance = | | **45.0** | **55.0** | |
| Observed Additive % Herbicide Tolerance: | | | | | | | | | |
| ID-13257 | PM1PM2/ BnCL131A1 | S653N (He) W574L (He) / A122T-S653N (He) | Observed % Herbicide Tolerance= | | | | | | |
| | | | | | | | **78**.**3** | **85.0** | |

To understand whether stacking the three heterozygous mutations in PM1PM2/BNCL131A1 resulted in an additive or synergistic herbicide tolerance effect, the observed mean herbicide tolerances for BnCL131A1 (He), PM1 (AHASL1C-PM1) and PM2 (AHASL1A-PM2) were added to obtain an expected percent herbicide tolerance (Table 7). It is noted that the PM1 and PM2 lines used in this experiment were homozygous for the AHAS-inhibitor tolerant mutation. Another check line would have been a heterozygous IMI-tolerant PM1 line and a heterozygous IMI-tolerant PM2 line, but since this material was not available, their homozygous counterparts were used. From previous studies it has been shown that lines containing a heterozygous IMI-tolerant mutation were always less tolerant to imidazolinone herbicide treatments than lines containing a homozygous AHAS-inhibitor tolerant mutation (data not shown). The expected additive percent herbicide tolerance for the three separate lines (BnCL131A1 (He) + PM1 (AHASL1C-PM1) + PM2 (AHASL1A-PM2)) at 140 g ai/ha of imazamox was 45%, whereas the observed percent herbicide tolerance of the PM1PM1/BnCL131A1 line (containing all 3 mutations in the heterozygous state) was 78.3%. A similar result was observed for the higher treatment rate of 210 g ai/ha where the expected additive percent herbicide tolerance was 55%, whereas the observed percent herbicide tolerance for PM1PM2/BnCL131A1 was 85% (Table 7).

In conclusion, the stacked line, PM1PM2/BnCL131A1, with the heterozygous AHAS-inhibitor tolerant mutations, demonstrated a synergistic herbicide tolerance effect resulting in an unexpected high level of tolerance to imidizolinone herbicides resulting in a tolerance significantly greater than that observed with the single homozygous AHAS-inhibitor tolerant mutations.

Although the foregoing invention has been described in some detail by way of illustration and example for purposes of clarity of understanding, it will be obvious that certain changes and modifications may be practiced within the scope of the appended claims.

## Claims

1. A recombinant or mutagenized nucleic acid molecule encoding a *Brassica* acetohydroxyacid synthase large subunit (AHASL) protein comprising an alanine to threonine substitution at a position corresponding to position A122 of SEQ ID NO:23, and a serine to asparagine substitution at a position corresponding to position S653 of SEQ ID NO:23, wherein the nucleic acid molecule encodes an AHASL protein that is resistant to inhibition by an AHAS-inhibiting herbicide, provided that the nucleic acid molecule is not SEQ ID NO:29 of WO2008124495 containing mutation A90T and fused at base 160 of that sequence to the vector tag and translational start site of pTrcHisA; and
provided that the nucleic acid molecule is not SEQ ID NO:29 of WO2008124495 containing mutation A90T, comprised in a plant transformation vector; and
provided that the nucleic acid molecule is not SEQ ID NO:33 of WO2008124495 containing mutation A122T, comprised in the bacterial expression vector pKK233-2 (Genbank Accession X70478); and
provided that the nucleic acid molecule is not SEQ ID NO:34 of WO2008124495 containing mutation A122T, comprised in a plant transformation vector; and
provided that the nucleic acid molecule is not pZm UBI + I::c-ZmAHAS L2::t-ZmAHAS L2 of WO2008124495 containing mutations A122T and S653N.

2. A recombinant or mutagenized nucleic acid molecule as claimed in claim 1, wherein the nucleic acid molecule encodes an AHASL protein having the amino acid sequence of BN02-131 (SEQ ID NO:21).

3. A recombinant or mutagenized nucleic acid molecule as claimed in claim 1, wherein the nucleic acid molecule has the nucleic acid sequence of BN02-131 (SEQ ID NO:16).

4. A recombinant or mutagenized nucleic acid molecule as claimed in any preceding claim, wherein the nucleic acid molecule encodes a *Brassica napus* AHASL III protein.

5. An expression vector comprising a recombinant or mutagenized nucleic acid molecule of any of claims 1 to 4.

6. A *Brassica* plant comprising a mutagenized nucleic acid molecule of any of claims 1 to 4, wherein the *Brassica* plant is resistant to at least one AHAS-inhibiting herbicide.

7. *ABrassica* plant as claimed in claim 6, wherein the *Brassica* plant is of a *Brassica* species selected from the group consisting of *B. napus, B. rapa,* and *B. juncea.*

8. A *Brassica* plant as claimed in claim 6 or claim 7, wherein the *Brassica* plant comprises a second mutagenized AHASL protein.

9. A *Brassica* plant as claimed in any of claims 6 to 8, wherein the mutagenized nucleic acid molecule is part of a recombinant expression vector.

10. A *Brassica* plant as claimed in any of claims 6 to 9, wherein the *Brassica* plant is resistant to at least one AHAS-inhibiting herbicide selected from the group consisting of imidazolinone herbicides, sulfonylurea herbicides, triazolopyrimidine herbicides, pyrimidinyloxybenzoate herbicides, sulfonylamino-carbonyltriazolinone herbicides, and mixtures thereof.

11. A *Brassica* plant as claimed in any of claims 6 to 9, wherein the plant is non-transgenic.

12. A *Brassica* plant as claimed in any of claims 6 to 10, further comprising at least one additional nucleic acid molecule encoding a protein of interest, wherein the at least one additional nucleic acid molecule encodes a protein of interest providing for a trait of interest selected from the group consisting of AHAS-tolerance, glyphosate tolerance, dicamba tolerance, PPO-inhibitor tolerance, insect resistance, glufosinate tolerance, HPPD-inhibitor tolerance, auxinic herbicide tolerance, and disease resistance.

13. A *Brassica* plant as claimed in claim 12, wherein the protein of interest provides for AHAS-inhibitor tolerance.

14. A *Brassica* plant as claimed in claim 13, wherein the protein of interest is an AHASL protein comprising an amino acid molecule having at least one substitution at an amino acid position selected from the group consisting of A122, P197, R199, T203, A205, W574, S653, G654, and combinations thereof.

15. A *Brassica* plant as claimed in claim 13, wherein the AHAS inhibitor tolerance is encoded by a gene selected from the group consisting of PM1, PM2, and BR1, and combinations thereof.

16. A *Brassica* seed of, or capable of producing a *Brassica* plant comprising a mutagenized nucleic acid molecule of any of claims 1 to 4.

17. A container of *Brassica* seeds wherein the container comprises at least 10% seeds capable of producing *Brassica* plants comprising a mutagenized nucleic acid molecule of any of claims 1 to 4.

18. A method of controlling weeds in a *Brassica* plant field, said method comprising:
(a) growing in said field *Brassica* plants comprising in their genome at least one mutagenized acetohydroxyacid synthase large subunit (AHASL)-encoding nucleic acid molecule of any of claims 1 to 5 that encodes an herbicide resistant AHASL protein; and
(b) applying an effective amount of at least one AHAS-inhibiting herbicide to weeds and to the *Brassica* plants in the *Brassica* plant field, wherein the effective amount of herbicide is sufficient to kill or inhibit the growth of a wild-type *Brassica* plant.

19. A method as claimed in claim 18, wherein the *Brassica* plant is selected from the group consisting of *B. juncea, B. napus, B. rapa, B. carinata, B. oleracea,* and *B. nigra.*

20. A method as claimed in claim 18, wherein the AHAS-inhibiting herbicide is selected from the group consisting of imidazolinone herbicides, sulfonylurea herbicides, triazolopyrimidine herbicides, pyrimidinyloxybenzoate herbicides, and mixtures thereof.

21. A method as claimed in claim 18, wherein the *Brassica* plant is non-transgenic.

22. A *Brassica* seed as claimed in claim 16 coated with a seed treatment formulation comprising at least one AHAS-inhibiting herbicide.

23. A plant part from a plant of any of claims 6 to 15 comprising a mutagenized nucleic acid of any of claims 1 to 4.

24. A method as claimed in claim 20, wherein the herbicide is an imidazolinone herbicide.

25. A method as claimed in claim 24, wherein the imidazolinone herbicide is imazamox.

## Patentansprüche

1. Rekombinantes oder mutagenisiertes Nukleinsäuremolekül, das für ein Protein der großen Untereinheit der Acetohydroxysäuresynthase (AHASL) aus *Brassica* codiert, und das eine Alanin-zu-Threonin-Substitution in einer Position entsprechend Position A122 von SEQ ID No: 23 und eine Serin-zu-Asparagin-Substitution in einer Position entsprechend Position S653 von SEQ ID No: 23 umfasst, wobei das Nukleinsäuremolekül für ein AHASL-Protein codiert, das gegen Hemmung durch AHAS-hemmendes Herbizid resistent ist, mit der Maßgabe, dass es sich bei dem Nukleinsäuremolekül nicht um SEQ ID No: 29 von WO2008124495 mit der Mutation A90T, das an der Base 160 jener Sequenz mit dem Tag des Vektors und der Translationsstartstelle von pTrcHisA fusioniert ist, handelt; und
mit der Maßgabe, dass es sich bei dem Nukleinsäuremolekül nicht um SEQ ID No: 29 von WO2008124495 mit der Mutation A90T, das in einem Pflanzentransformationsvektor umfasst ist, handelt; und
mit der Maßgabe, dass es sich bei dem Nukleinsäuremolekül nicht um SEQ ID No: 33 von WO2008124495 mit der Mutation A122T, das in dem bakteriellen Expressionsvektor pKK233-2 umfasst ist (Genbank-Zugang X70478), handelt; und
mit der Maßgabe, dass es sich bei dem Nukleinsäuremolekül nicht um SEQ ID No: 34 von WO2008124495 mit der Mutation A122T, das in einem Pflanzentransformationsvektor umfasst ist, handelt; und
mit der Maßgabe, dass es sich bei dem Nukleinsäuremolekül nicht um pZm UBI + I::c-ZmAHAS L2::t-ZmAHAS L2 von WO2008124495 mit den Mutationen A122T und S653N handelt.

2. Rekombinantes oder mutagenisiertes Nukleinsäuremolekül nach Anspruch 1, wobei das Nukleinsäuremolekül für ein AHASL-Protein mit der Aminosäuresequenz von BN02-131 (SEQ ID No: 21) codiert.

3. Rekombinantes oder mutagenisiertes Nukleinsäuremolekül nach Anspruch 1, wobei das Nukleinsäuremolekül die Nukleinsäuresequenz von von BN02-131 (SEQ ID No: 16) aufweist.

4. Rekombinantes oder mutagenisiertes Nukleinsäuremolekül nach einem vorhergehenden Anspruch, wobei das Nukleinsäuremolekül für ein *Brassica napus-*AHASL-III-Protein codiert.

5. Expressionsvektor, umfassend ein rekombinantes oder mutagenisiertes Nukleinsäuremolekül nach einem der Ansprüche 1 bis 4.

6. *Brassica*-Pflanze, die ein mutagenisiertes Nukleinsäuremolekül nach einem der Ansprüche 1 bis 4 umfasst, wobei die *Brassica*-Pflanze gegen mindestens ein AHAS-hemmendes Herbizid resistent ist.

7. *Brassica*-Pflanze nach Anspruch 6, wobei es sich bei der *Brassica*-Pflanze um eine einer *Brassica-*Art, ausgewählt aus der Gruppe bestehend aus *B*. *napus, B. rapa* und *B. juncea* handelt.

8. Brassica-Pflanze nach Anspruch 6 oder Anspruch 7, wobei die *Brassica*-Pflanze ein zweites mutagenisiertes AHASL-Protein umfasst.

9. *Brassica*-Pflanze nach einem der Ansprüche 6 bis 8, wobei das mutagenisierte Nukleinsäuremolekül Bestandteil eines rekombinanten Expressionsvektors ist.

10. *Brassica*-Pflanze nach einem der Ansprüche 6 bis 9, wobei die *Brassica*-Pflanze gegen mindestens ein AHAS-hemmendes Herbizid, ausgewählt aus der Gruppe bestehend aus Imidazolinonherbiziden, Sulfonylharnstoffherbiziden, Triazolopyrimidinherbiziden, Pyrimidinyloxybenzoatherbiziden, Sulfonylaminocarbonyltriazolinonherbiziden und Mischungen davon, resistent ist.

11. *Brassica*-Pflanze nach einem der Ansprüche 6 bis 9, wobei die Pflanze nicht transgen ist.

12. *Brassica*-Pflanze nach einem der Ansprüche 6 bis 10, die weiterhin mindestens ein zusätzliches Nukleinsäuremolekül, das für ein Protein von Interesse codiert, umfasst, wobei das mindestens eine zusätzliche Nukleinsäuremolekül für ein Protein von Interesse codiert, das ein Merkmal von Interesse, ausgewählt aus der Gruppe bestehend aus AHAS-Toleranz, Glyphosat-Toleranz, Dicamba-Toleranz, PPO-Hemmer-Toleranz, Insektenresistenz, Glufosinat-Toleranz, HPPD-Hemmer-Toleranz, Auxinherbizidtoleranz und Krankheitsresistenz vermittelt.

13. *Brassica*-Pflanze nach Anspruch 12, wobei das Protein von Interesse AHAS-Hemmer-Toleranz vermittelt.

14. *Brassica*-Pflanze nach Anspruch 13, wobei das Protein von Interesse ein AHASL-Protein ist, das ein Aminosäuremolekül mit mindestens einer Substitution an einer Aminosäureposition, ausgewählt aus der Gruppe bestehend aus A122, P197, R199, T203, A205, W574, S653, G654 und Kombinationen davon aufweist.

15. *Brassica*-Pflanze nach Anspruch 13, wobei die AHAS-Hemmer-Toleranz von einem Gen, ausgewählt aus der Gruppe bestehend aus PM1, PM2 und BR1 und Kombinationen davon codiert wird.

16. *Brassica*-Samen von einer *Brassica-Pflanze,* umfassend ein mutagenisiertes Nukleinsäuremolekül nach einem der Ansprüche 1 bis 4, oder *Brassica-*Samen, der fähig ist, eine *Brassica*-Pflanze umfassend ein mutagenisiertes Nukleinsäuremolekül nach einem der Ansprüche 1 bis 4 zu produzieren.

17. Behältnis von *Brassica*-Samen, wobei das Behältnis mindestens 10% Samen enthält, die fähig sind, *Brassica*-Pflanzen umfassend ein mutagenisiertes Nukleinsäuremolekül nach einem der Ansprüche 1 bis 4 zu produzieren.

18. Verfahren zum Bekämpfen von Unkräutern in einem *Brassica*-Pflanzenfeld, wobei das Verfahren Folgendes umfasst:
(a) Heranziehen von *Brassica*-Pflanzen in dem Feld, die in ihren Genom mindestens ein mutagenisiertes Nukleinsäuremolekül, das für die große Untereinheit der Acetohydroxysäuresynthase (AHASL) codiert, nach einem der Ansprüche 1 bis 5, das für ein herbizidresistentes AHASL-Protein codiert, umfasst, und
(b) Applizieren einer wirksamen Menge an mindestens einem AHAS-hemmenden Herbizid auf Unkräuter und auf die *Brassica*-Pflanzen in dem *Brassica*-Pflanzenfeld, wobei die wirksame Herbizidmenge ausreicht, um eine Wildtyp-*Brassica*-Pflanze abzutöten oder ihr Wachstum zu hemmen.

19. Verfahren nach Anspruch 18, wobei die *Brassica-*Pflanze aus der Gruppe bestehend aus *B. juncea, B. napus, B. rapa, B. carinata, B. oleracea* und *B. nigra* ausgewählt ist.

20. Verfahren nach Anspruch 18, wobei das AHAS-hemmende Herbizid aus der Gruppe bestehend aus Imidazolinonherbiziden, Sulfonylharnstoffherbiziden, Triazolopyrimidinherbiziden, Pyrimidinyloxybenzoatherbiziden und Mischungen davon ausgewählt ist.

21. Verfahren nach Anspruch 18, wobei die *Brassica-*Pflanze nicht transgen ist.

22. *Brassica*-Samen nach Anspruch 16, der mit einer Samenbehandlungsformulierung, die mindestens ein AHAS-hemmendes Herbizid umfasst, beschichtet ist.

23. Pflanzenteil von einer Pflanze nach einem der Ansprüche 6 bis 15, umfassend eine mutagenisierte Nukleinsäure nach einem der Ansprüche 1 bis 4.

24. Verfahren nach Anspruch 20, wobei es sich bei dem Herbizid um ein Imidazolinonherbizid handelt.

25. Verfahren nach Anspruch 24, wobei es sich bei dem Imidazolinonherbizid um Imazamox handelt.

## Revendications

1. Molécule d'acide nucléique recombinée ou mutée, codant pour une protéine de la grande sous-unité d'acétohydroxyacide synthase (AHASL) de *Brassica,* comprenant une substitution alanine en thréonine au niveau d'une position correspondant à la position A122 de SEQ ID n° 23, et une substitution sérine en asparagine au niveau d'une position correspondant à la position S653 de SEQ ID n° 23, **caractérisée en ce que** la molécule d'acide nucléique code pour une protéine AHASL qui est résistante à une inhibition par un herbicide d'inhibition d'AHAS, à condition que la molécule d'acide nucléique ne soit pas SEQ ID n° 29 de WO 2008/124495 contenant la mutation A90T et fusionné au niveau de la base 160 de cette séquence à l'étiquette de vecteur et au site de début de traduction de pTrcHisA ; et
à condition que la molécule d'acide nucléique ne soit pas SEQ ID n° 29 de WO 2008/124495 contenant la mutation A90T, comprise dans un vecteur de transformation végétale ; et
à condition que la molécule d'acide nucléique ne soit pas SEQ ID n° 33 de WO 2008/124495 contenant la mutation A122T, comprise dans le vecteur d'expression bactérien pKK233-2 (accès GenBank X70478) ; et
à condition que la molécule d'acide nucléique ne soit pas SEQ ID n° 34 de WO 2008/124495 contenant la mutation A122T, comprise dans un vecteur de transformation végétale ; et
à condition que la molécule d'acide nucléique ne soit pas pZm UBI + I::c-ZmAHAS L2::t-ZmAHAS L2 de WO 2008/124495 contenant les mutations A122T et S653N.

2. Molécule d'acide nucléique recombinée ou mutée selon la revendication 1, **caractérisée en ce que** la molécule d'acide nucléique code pour une protéine AHASL ayant la séquence d'acides aminés de BN02-131 (SEQ ID n° 21).

3. Molécule d'acide nucléique recombinée ou mutée selon la revendication 1, **caractérisée en ce que** la molécule d'acide nucléique possède la séquence d'acide nucléique de BN02-131 (SEQ ID n° 16).

4. Molécule d'acide nucléique recombinée ou mutée selon l'une quelconque des revendications précédentes, **caractérisée en ce que** la molécule d'acide nucléique code pour une protéine AHASL III de *Brassica napus.*

5. Vecteur d'expression comprenant une molécule d'acide nucléique recombinée ou mutée selon l'une quelconque des revendications 1 à 4.

6. Plante de *Brassica* comprenant une molécule d'acide nucléique mutée selon l'une quelconque des revendications 1 à 4, **caractérisée en ce que** la plante de *Brassica* est résistante à au moins un herbicide d'inhibition d'AHAS.

7. Plante de *Brassica* selon la revendication 6, **caractérisée en ce que** la plante de *Brassica* appartient à une espèce de *Brassica* choisie dans le groupe constitué par *B. napus, B. rapa* et *B. juncea.*

8. Plante de *Brassica* selon la revendication 6 ou la revendication 7, **caractérisée en ce que** la plante de *Brassica* comprend une deuxième protéine AHASL mutée.

9. Plante de *Brassica* selon l'une quelconque des revendications 6 à 8, **caractérisée en ce que** la molécule d'acide nucléique mutée fait partie d'un vecteur d'expression recombiné.

10. Plante de *Brassica* selon l'une quelconque des revendications 6 à 9, **caractérisée en ce que** la plante de *Brassica* est résistante à au moins un herbicide d'inhibition d'AHAS choisi dans le groupe constitué par les herbicides à base d'imidazolinone, les herbicides à base de sulfonylurées, les herbicides à base de triazolopyrimidine, les herbicides à base d'oxybenzoate de pyrimidinyle, les herbicides à base de sulfonylaminocarbonyltriazolinone, et des mélanges de ceux-ci.

11. Plante de *Brassica* selon l'une quelconque des revendications 6 à 9, **caractérisée en ce que** la plante n'est pas transgénique.

12. Plante de *Brassica* selon l'une quelconque des revendications 6 à 10, comprenant en outre au moins une autre molécule d'acide nucléique codant pour une protéine d'intérêt, **caractérisée en ce que** la au moins une autre molécule d'acide nucléique code pour une protéine d'intérêt fournissant un caractère d'intérêt choisi dans le groupe constitué par la tolérance à l'AHAS, la tolérance au glyphosate, la tolérance au dicamba, la tolérance à l'inhibiteur de PPO, la résistance aux insectes, la tolérance au glufosinate, la tolérance à l'inhibiteur de HPPD, la tolérance aux herbicides auxiniques, et la résistance aux maladies.

13. Plante de *Brassica* selon la revendication 12, **caractérisée en ce que** la protéine d'intérêt fournit une tolérance aux inhibiteurs d'AHAS.

14. Plante de *Brassica* selon la revendication 13, **caractérisée en ce que** la protéine d'intérêt est une protéine AHASL comprenant une molécule d'acides aminés ayant au moins une substitution au niveau d'une position d'acide aminé choisie dans le groupe constitué par A122, P197, R199, T203, A205, W574, S653, G654, et des combinaisons de celles-ci.

15. Plante de *Brassica* selon la revendication 13, **caractérisée en ce que** la tolérance aux inhibiteurs d'AHAS est codée par un gène choisi dans le groupe constitué par PM1, PM2 et BR1, et des combinaisons de ceux-ci.

16. Graine de *Brassica* de, ou capable de produire une plante de *Brassica* comprenant une molécule d'acide nucléique mutée selon l'une quelconque des revendications 1 à 4.

17. Conteneur de graines de *Brassica,* **caractérisé en ce que** le conteneur comprend au moins 10% de graines capables de produire des plantes de *Brassica* comprenant une molécule d'acide nucléique mutée selon l'une quelconque des revendications 1 à 4.

18. Méthode de contrôle d'adventices dans une parcelle de plantes de *Brassica,* ladite méthode comprenant :
(a) la culture dans ladite parcelle de plantes de *Brassica* comprenant dans leur génome au moins une molécule d'acide nucléique mutée codant pour la grande sous-unité d'acétohydroxyacide synthase (AHASL) selon l'une quelconque des revendications 1 à 5, qui code pour une protéine AHASL résistante aux herbicides ; et
(b) l'application d'une quantité efficace d'au moins un herbicide d'inhibition d'AHAS aux adventices et aux plantes de *Brassica* dans la parcelle de plantes de *Brassica,* où la quantité efficace d'herbicide est suffisante pour tuer ou inhiber la croissance d'une plante de *Brassica* de type sauvage.

19. Méthode selon la revendication 18, **caractérisée en ce que** la plante de *Brassica* est choisie dans le groupe constitué par *B. juncea, B. napus, B. rapa, B. carinata, B. oleracea* et *B. nigra.*

20. Méthode selon la revendication 18, **caractérisée en ce que** l'herbicide d'inhibition d'AHAS est choisi dans le groupe constitué par les herbicides à base d'imidazolinone, les herbicides à base de sulfonylurées, les herbicides à base de triazolopyrimidine, les herbicides à base d'oxybenzoate de pyrimidinyle, et des mélanges de ceux-ci.

21. Méthode selon la revendication 18, **caractérisée en ce que** la plante de *Brassica* n'est pas transgénique.

22. Semence de *Brassica* selon la revendication 16, enrobée par une formulation de traitement de semences comprenant au moins un herbicide d'inhibition d'AHAS.

23. Partie de plante issue d'une plante selon l'une quelconque des revendications 6 à 15, comprenant un acide nucléique muté selon l'une quelconque des revendications 1 à 4.

24. Méthode selon la revendication 20, **caractérisée en ce que** l'herbicide est un herbicide à base d'imidazolinone.

25. Méthode selon la revendication 24, **caractérisée en ce que** l'herbicide à base d'imidazolinone est l'imazamox.


### SEQUENCE LISTING

<110> BASF AGROCHEMICAL PRODUCTS B.V.
<120> HERBICIDE-RESISTANT AHAS-MUTANTS AND METHODS OF USE
<130> 13783-49
<140>
   <141>
<150> 61/100,541 <151> 2008-09-26
<160> 23
<170> PatentIn version 3.5
<210> 1
   <211> 20
   <212> DNA
   <213> Artificial Sequence
<220>
   <223> Description of Artificial Sequence: Synthetic primer
<400> 1
   ctaaccatgg cggcggcaac 20
<210> 2
   <211> 28
   <212> DNA
   <213> Artificial Sequence
<220>
   <223> Description of Artificial Sequence: Synthetic primer
<400> 2.
   agtctgggaa caaaccaaaa gcagtaca 28
<210> 3
   <211> 28
   <212> DNA
   <213> Artificial Sequence
<220>
   <223> Description of Artificial Sequence: Synthetic primer
<400> 3
   cgtctgggaa caaccaaaag tagtacaa 28
<210> 4
   <211> 43
   <212> DNA
   <213> Artificial Sequence
<220>
   <223> Description of Artificial Sequence: Synthetic oligonucleotide
<400> 4
   cttcgcttat cccggaggta cttccatgga gatccaccaa gcc 43
<210> 5
   <211> 43
   <212> DNA
   <213> Artificial Sequence
<220>
   <223> Description of Artificial Sequence: Synthetic oligonucleotide
<400> 5
   gcttggtgga tctccatgga agtacctccg ggataagcga agc 43
<210> 6
   <211> 21
   <212> DNA
   <213> Artificial Sequence
<220>
   <223> Description of Artificial Sequence: Synthetic primer
<400> 6
   cccatcaacg tactcgcacc a 21
<210> 7
   <211> 28
   <212> DNA
   <213> Artificial Sequence
<220>
   <223> Description of Artificial Sequence: Synthetic primer
<400> 7
   gtcaatgtcg cacctgaaaa aaccgaca 28
<210> 8
   <211> 27
   <212> DNA
   <213> Artificial Sequence
<220>
   <223> Description of Artificial Sequence: Synthetic primer
<400> 8
   gctaacccgc tgacgaggtt ggtagct 27
<210> 9
   <211> 26
   <212> DNA
   <213> Artificial Sequence
<220>
   <223> Description of Artificial Sequence: Synthetic primer
<400> 9
   aaggcttggt ggatctccat ggacgt 26
<210> 10
   <211> 24
   <212> DNA
   <213> Artificial Sequence
<220>
   <223> Description of Artificial Sequence: Synthetic primer
<400> 10
   gaggctttcg ctagcagggc tcaa 24
<210> 11
   <211> 2033
   <212> DNA
   <213> Artificial Sequence
<220>
   <223> Description of Artificial Sequence: Synthetic polynucleotide
<400> 11
   tctctcctct aaccatggcg gcggcaacat cgtcttctcc gatctcctta accgctaaac 60
   cttcttccaa atcccctcta cccatttcca gattctccct tcccttctcc ttaaccccac 120
   agaaagactc ctcccgtctc caccgtcctc tcgccatctc cgccgttctc aactcacccg 180
   tcaatgtcgc acctccttcc cctgaaaaaa ccgacaagaa caagactttc gtctcccgct 240
   acgctcccga cgagccccgc aagggtgctg atatcctcgt cgaagccctc gagcgtcaag 300
   gcgtcgaaac cgtctttgct tatcccggag gtgcttccat ggagatccac caagccttga 360
   ctcgctcctc caccatccgt aacgtccttc cccgtcacga acaaggagga gtcttcgccg 420
   ccgagggtta cgctcgttcc tccggcaaac cgggaatctg catagccact tcgggtcccg 480
   gagctaccaa cctcgtcagc gggttagcag acgcgatgct tgacagtgtt cctcttgtcg 540
   ccattacagg acaggtccct cgccggatga tcggtactga cgccttccaa gagacaccaa 600
   tcgttgaggt aacgaggtct attacgaaac ataactattt ggtgatggat gttgatgaca 660
   tacctaggat cgttcaagaa gctttctttc tagctacttc cggtagaccc ggaccggttt 720
   tggttgatgt tcctaaggat attcagcagc agcttgcgat tcctaactgg gatcaaccta 780
   tgcgcttacc tggctacatg tctaggttgc ctcagcctcc ggaagtttct cagttaggtc 840
   agatcgttag gttgatctcg gagtctaaga ggcctgtttt gtacgttggt ggtggaagct 900
   tgaactcgag tgaagaactg gggagatttg tcgagcttac tgggatcccc gttgcgagta 960
   ctttgatggg gcttggctct tatccttgta acgatgagtt gtccctgcag atgcttggca 1020
   tgcacgggac tgtgtatgct aactacgctg tggagcatag tgatttgttg ctggcgtttg 1080
   gtgttaggtt tgatgaccgt gtcacgggaa agctcgaggc tttcgctagc agggctaaaa 1140
   ttgtgcacat agacattgat tctgctgaga ttgggaagaa taagacacct cacgtgtctg 1200
   tgtgtggtga tgtaaagctg gctttgcaag ggatgaacaa ggttcttgag aaccgggcgg 1260
   aggagctcaa gcttgatttc ggtgtttgga ggagtgagtt gagcgagcag aaacagaagt 1320
   tccctttgag cttcaaaacg tttggagaag ccattcctcc gcagtacgcg attcagatcc 1380
   tcgacgagct aaccgaaggg aaggcaatta tcagtactgg tgttggacag catcagatgt 1440
   gggcggcgca gttttacaag tacaggaagc cgagacagtg gctgtcgtca tcaggcctcg 1500
   gagctatggg ttttggactt cctgctgcga ttggagcgtc tgtggcgaac cctgatgcga 1560
   ttgttgtgga tattgacggt gatggaagct tcataatgaa cgttcaagag ctggccacaa 1620
   tccgtgtaga gaatcttcct gtgaagatac tcttgttaaa caaccagcat cttgggatgg 1680
   tcatgcaatg ggaagatcgg ttctacaaag ctaacagagc tcacacttat ctcggggacc 1740
   cggcaaggga gaacgagatc ttccctaaca tgctgcagtt tgcaggagct tgcgggattc 1800
   cagctgcgag agtgacgaag aaagaagaac tccgagaagc tattcagaca atgctggata 1860
   caccaggacc atacctgttg gatgtgatat gtccgcacca agaacatgtg ttaccgatga 1920
   tcccaagtgg tggcactttc aaagatgtaa taacagaagg ggatggtcgc actaagtact 1980
   gagagatgaa gctggtgatc gatcatatgg taaaagactt agtttcagtt tcc 2033
<210> 12
   <211> 2041
   <212> DNA
   <213> Artificial Sequence
<220>
   <223> Description of Artificial Sequence: Synthetic polynucleotide
<400> 12
   tctctcattt ctctctctct ctcatctaac catggcggcg gcaacatcgt cttctccgat     60
   ctccttaacc gctaaacctt cttccaaatc ccctctaccc atttccagat tctcccttcc    120
   cttctcctta accccacaga aaccctcctc ccgtctccac cgtccactcg ccatctccgc    180
   cgttctcaac tcacccgtca atgtcgcacc tgaaaaaacc gacaagatca agactttcat    240
   ctcccgctac gctcccgacg agccccgcaa gggtgctgat atcctcgtgg aagccctcga    300
   gcgtcaaggc gtcgaaaccg tcttcgctta tcccggaggt gcctccatgg agatccacca    360
   agccttgact cgctcctcca ccatccgtaa cgtcctcccc cgtcacgaac aaggaggagt    420
   cttcgccgcc gagggttacg ctcgttcctc cggcaaaccg ggaatctgca tagccacttc    480
   gggtcccgga gctaccaacc tcgtcagcgg gttagccgac gcgatgcttg acagtgttcc    540
   tctcgtcgcc atcacaggac aggtccctcg ccggatgatc ggtactgacg cgttccaaga    600
   gacgccaatc gttgaggtaa cgaggtctat tacgaaacat aactatctgg tgatggatgt 660
   tgatgacata cctaggatcg ttcaagaagc attctttcta gctacttccg gtagacccgg 720
   accggttttg gttgatgttc ctaaggatat tcagcagcag cttgcgattc ctaactggga 780
   tcaacctatg cgcttgcctg gctacatgtc taggctgcct cagccaccgg aagtttctca 840
   gttaggccag atcgttaggt tgatctcgga gtctaagagg cctgttttgt acgttggtgg 900
   tggaagcttg aactcgagtg aagaactggg gagatttgtc gagcttactg ggatccctgt 960
   tgcgagtacg ttgatggggc ttggctctta tccttgtaac gatgagttgt ccctgcagat 1020
   gcttggcatg cacgggactg tgtatgctaa ctacgctgtg gagcatagtg atttgttgct 1080
   ggcgtttggt gttaggtttg atgaccgtgt cacgggaaag ctcgaggcgt ttgcgagcag 1140
   ggctaagatt gtgcacatag acattgattc tgctgagatt gggaagaata agacacctca 1200
   cgtgtctgtg tgtggtgatg taaagctggc tttgcaaggg atgaacaagg ttcttgagaa 1260
   ccgggcggag gagctcaagc ttgatttcgg tgtttggagg agtgagttga gcgagcagaa 1320
   acagaagttc ccgttgagct tcaaaacgtt tggagaagcc attcctccgc agtacgcgat 1380
   tcaggtccta gacgagctaa cccaagggaa ggcaattatc agtactggtg ttggacagca 1440
   tcagatgtgg gcggcgcagt tttacaagta caggaagccg aggcagtggc tgtcgtcctc 1500
   aggactcgga gctatgggtt tcggacttcc tgctgcgatt ggagcgtctg tggcgaaccc 1560
   tgatgcgatt gttgtggaca ttgacggtga tggaagcttc ataatgaacg ttcaagagct 1620
   ggccacaatc cgtgtagaga atcttcctgt gaagatactc ttgttaaaca accagcatct 1680
   tgggatggtc atgcaatggg aagatcggtt ctacaaagct aacagagctc acacttatct 1740
   cggggacccg gcaagggaga acgagatctt ccctaacatg ctgcagtttg caggagcttg 1800
   cgggattcca gctgcgagag tgacgaagaa agaagaactc cgagaagcta ttcagacaat 1860
   gctggataca cctggaccgt acctgttgga tgtcatctgt ccgcaccaag aacatgtgtt 1920
   accgatgatc ccaagtggtg gcactttcaa agatgtaata accgaagggg atggtcgcac 1980
   taagtactga gagatgaagc tggtgatcca tcatatggta aaagacttag tttcagtttt 2040
   c 2041
<210> 13
   <211> 2024
   <212> DNA
   <213> Artificial Sequence
<220>
   <223> Description of Artificial Sequence: Synthetic polynucleotide
<400> 13
   tctctcatct aaccatggcg gcggcaacat cgtcttctcc gatctcctta accgctaaac 60
   cttcttccaa atcccctcta cccatttcca gattctccct tcccttctcc ttaaccccac 120
   agaaaccctc ctcccgtctc caccgtcctc tcgccatctc cgccgttctc aactcacccg 180
   tcaatgtcgc acctgaaaaa accgacaaga tcaagacttt catctcccgc tacgctcccg 240
   acgagccccg caagggtgct gatatcctcg tggaagccct cgagcgtcaa ggcgtcgaaa 300
   ccgtcttcgc ttatcccgga ggtgcttcca tggagatcca ccaagccttg actcgctcct 360
   ccaccatccg taacgtcctc ccccgtcacg aacaaggagg agtcttcgcc gccgagggtt 420
   acgctcgttc ctccggcaaa ccgggaatct gcatagccac ttcgggtccc ggagctacca 480
   acctcgtcag cgggttagcc gacgcgatgc ttgacagtgt tcctctcgtc gccatcacag 540
   gacaggtccc tcgccggatg atcggtactg acgcgttcca agagacgcca atcgttgagg 600
   taacgaggtc tattacgaaa cataactatc tggtgatgga tgttgatgac atacctagga 660
   tcgttcaaga agctttcttt ctagctactt ccggtagacc cggaccggtt ttggttgatg 720
   ttcctaagga tattcagcag cagcttgcga ttcctaactg ggatcaacct atgcgcttgc 780
   ctggctacat gtctaggctg cctcagccac cggaagtttc tcagttaggt cagatcgtta 840
   ggttgatctc ggagtctaag aggcctgttt tgtacgttgg tggtggaagc ttgaactcga 900
   gtgaagaact ggggagattt gtcgagctta ctgggatccc tgttgcgagt acgttgatgg 960
   ggcttggctc ttatccttgt aacgatgact tgtccctgca gatgcttggc atgcacggga 1020
   ctgtgtatgc taactacgct gtggagcata gtgatttgtt gctggcgttt ggtgttaggt 1080
   ttgatgaccg tgtcacggga aagctcgagg cgtttgcgag cagggctaag attgtgcaca 1140
   tagacattga ttctgctgag attgggaaga ataagacacc tcacgtgtct gtgtgtggtg 1200
   atgtaaagct ggctttgcaa gggatgaaca aggttcttga gaaccgggcg gaggagctca 1260
   agcttgattt cggtgtttgg aggagtgagt tgagcgagca gaaacagaag ttcccgttga 1320
   gcttcaaaac gtttggagaa gccattcctc cgcagtacgc gattcaggtc ctagacgagc 1380
   taacccaagg gaaggcaatt atcagtactg gtgttggaca gcatcagatg tgggcggcgc 1440
   agttttacaa gtacaggaag ccgaggcagt ggctgtcgtc ctcaggactc ggagctatgg 1500
   gtttcggact tcctgctgcg attggagcgt ctgtggcgaa ccctgatgcg attgttgtgg 1560
   acattgacgg tgatggaagc ttcataatga acgttcaaga gctggccaca atccgtgtag 1620
   agaatcttcc tgtgaagata ctcttgttaa acaaccagca tcttgggatg gtcatgcaat 1680
   gggaagatcg gttctacaaa gctaacagag ctcacactta tctcggggac ccggcaaggg 1740
   agaacgagat cttccctaac atgctgcagt ttgcaggagc ttgcgggatt ccagctgcga 1800
   gagtgacgaa gaaagaagaa ctccgagaag ctattcagac aatgctggat acacctggac 1860
   cgtacctgtt ggatgtcatc tgtccgcacc aagaacatgt gttaccgatg atcccaagtg 1920
   gtggcacttt caaagatgta ataaccgaag gggatggtcg cactaagtac tgagagatga 1980
   agctggtgat cgatcatatg gtaaaagact tagtttcagt tttc 2024
<210> 14
   <211> 2024
   <212> DNA
   <213> Artificial Sequence
<220>
   <223> Description of Artificial Sequence: Synthetic polynucleotide
<400> 14
   tctctcatct aaccatggcg gcggcaacat cgtcttctcc gatctcctta accgctaaac 60
   cttcttccaa atcccctcta cccatttcca gattctccct tcccttctcc ttaaccccac 120
   agaaaccctc ctcccgtctc caccgtcctc tcgccatctc cgccgttctc aactcacccg 180
   tcaatgtcgc acctgaaaaa accgacaaga tcaagacttt catctcccgc tacgctcccg 240
   acgagccccg caagggtgct gatatcctcg tggaagccct cgagcgtcaa ggcgtcgaaa 300
   ccgtcttcgc ttatcccgga ggtgcttcca tggagatcca ccaagccttg actcgctcct 360
   ccaccatccg taacgtcctc ccccgtcacg aacaaggagg agtcttcgcc gccgagggtt 420
   acgctcgttc ctccggcaaa ccgggaatct gcatagccac ttcgggtccc ggagctacca 480
   acctcgtcag cgggttagcc gacgcgatgc ttgacagtgt tcctctcgtc gccatcacag 540
   gacaggtccc tcgccggatg atcggtactg acgcgttcca agagacgcca atcgttgagg 600
   taacgaggtc tattacgaaa cataactatc tggtgatgga tgttgatgac atacctagga 660
   tcgttcaaga agctttcttt ctagctactt ccggtagacc cggaccggtt ttggttgatg 720
   ttcctaagga tattcagcag cagcttgcga ttcctaactg ggatcaacct atgcgcttgc 780
   ctggctacat gtctaggctg cctcagccac cggaagtttc tcagttaggt cagatcgtta 840
   ggttgatctc ggagtctaag aggcctgttt tgtacgttgg tggtggaagc ttgaactcga 900
   gtgaagaact ggggagattt gtcgagctta ctgggatccc tgttgcgagt acgttgatgg 960
   ggcttggctc ttatccttgt aacgatgact tgtccctgca gatgcttggc atgcacggga 1020
   ctgtgtatgc taactacgct gtggagcata gtgatttgtt gctggcgttt ggtgttaggt 1080
   ttgatgaccg tgtcacggga aagctcgagg cgtttgcgag cagggctaag attgtgcaca 1140
   tagacattga ttctgctgag attgggaaga ataagacacc tcacgtgtct gtgtgtggtg 1200
   atgtaaagct ggctttgcaa gggatgaaca aggttcttga gaaccgggcg gaggagctca 1260
   agcttgattt cggtgtttgg aggagtgagt tgagcgagca gaaacagaag ttcccgttga 1320
   gcttcaaaac gtttggagaa gccattcctc cgcagtacgc gattcaggtc ctagacgagc 1380
   taacccaagg gaaggcaatt atcagtactg gtgttggaca gcatcagatg tgggcggcgc 1440
   agttttacaa gtacaggaag ccgaggcagt ggctgtcgtc ctcaggactc ggagctatgg 1500
   gtttcggact tcctgctgcg attggagcgt ctgtggcgaa ccctgatgcg attgttgtgg 1560
   acattgacgg tgatggaagc ttcataatga acgttcaaga gctggccaca atccgtgtag 1620
   agaatcttcc tgtgaagata ctcttgttaa acaaccagca tcttgggatg gtcatgcaat 1680
   gggaagatcg gttctacaaa gctaacagag ctcacactta tctcggggac ccggcaaggg 1740
   agaacgagat cttccctaac atgctgcagt ttgcaggagc ttgcgggatt ccagctgcga 1800
   gagtgacgaa gaaagaagaa ctccgagaag ctattcagac aatgctggat acacctggac 1860
   cgtacctgtt ggatgtcatc tgtccgcacc aagaacatgt gttaccgatg atcccaaatg 1920
   gtggcacttt caaagatgta ataaccgaag gggatggtcg cactaagtac tgagagatga 1980
   agctggtgat cgatcatatg gtaaaagact tagtttcagt tttc 2024
<210> 15
   <211> 2024
   <212> DNA
   <213> Artificial Sequence
<220>
   <223> Description of Artificial Sequence: Synthetic polynucleotide
<400> 15
   tctctcatct aaccatggcg gcggcaacat cgtcttctcc gatctcctta accgctaaac    60
   cttcttccaa atcccctcta cccatttcca gattctccct tcccttctcc ttaaccccac   120
   agaaaccctc ctcccgtctc caccgtcctc tcgccatctc cgccgttctc aactcacccg   180
   tcaatgtcgc acctgaaaaa accgacaaga tcaagacttt catctcccgc tacgctcccg   240
   acgagccccg caagggtgct gatatcctcg tggaagccct cgagcgtcaa ggcgtcgaaa   300
   ccgtcttcgc ttatcccgga ggtacttcca tggagatcca ccaagccttg actcgctcct   360
   ccaccatccg taacgtcctc ccccgtcacg aacaaggagg agtcttcgcc gccgagggtt   420
   acgctcgttc ctccggcaaa ccgggaatct gcatagccac ttcgggtccc ggagctacca   480
   acctcgtcag cgggttagcc gacgcgatgc ttgacagtgt tcctctcgtc gccatcacag   540
   gacaggtccc tcgccggatg atcggtactg acgcgttcca agagacgcca atcgttgagg   600
   taacgaggtc tattacgaaa cataactatc tggtgatgga tgttgatgac atacctagga   660
   tcgttcaaga agctttcttt ctagctactt ccggtagacc cggaccggtt ttggttgatg   720
   ttcctaagga tattcagcag cagcttgcga ttcctaactg ggatcaacct atgcgcttgc   780
   ctggctacat gtctaggctg cctcagccac cggaagtttc tcagttaggt cagatcgtta   840
   ggttgatctc ggagtctaag aggcctgttt tgtacgttgg tggtggaagc ttgaactcga    900
   gtgaagaact ggggagattt gtcgagctta ctgggatccc tgttgcgagt acgttgatgg    960
   ggcttggctc ttatccttgt aacgatgact tgtccctgca gatgcttggc atgcacggga   1020
   ctgtgtatgc taactacgct gtggagcata gtgatttgtt gctggcgttt ggtgttaggt   1080
   ttgatgaccg tgtcacggga aagctcgagg cgtttgcgag cagggctaag attgtgcaca   1140
   tagacattga ttctgctgag attgggaaga ataagacacc tcacgtgtct gtgtgtggtg   1200
   atgtaaagct ggctttgcaa gggatgaaca aggttcttga gaaccgggcg gaggagctca   1260
   agcttgattt cggtgtttgg aggagtgagt tgagcgagca gaaacagaag ttcccgttga   1320
   gcttcaaaac gtttggagaa gccattcctc cgcagtacgc gattcaggtc ctagacgagc   1380
   taacccaagg gaaggcaatt atcagtactg gtgttggaca gcatcagatg tgggcggcgc   1440
   agttttacaa gtacaggaag ccgaggcagt ggctgtcgtc ctcaggactc ggagctatgg   1500
   gtttcggact tcctgctgcg attggagcgt ctgtggcgaa ccctgatgcg attgttgtgg   1560
   acattgacgg tgatggaagc ttcataatga acgttcaaga gctggccaca atccgtgtag   1620
   agaatcttcc tgtgaagata ctcttgttaa acaaccagca tcttgggatg gtcatgcaat   1680
   gggaagatcg gttctacaaa gctaacagag ctcacactta tctcggggac ccggcaaggg   1740
   agaacgagat cttccctaac atgctgcagt ttgcaggagc ttgcgggatt ccagctgcga   1800
   gagtgacgaa gaaagaagaa ctccgagaag ctattcagac aatgctggat acacctggac   1860
   cgtacctgtt ggatgtcatc tgtccgcacc aagaacatgt gttaccgatg atcccaagtg   1920
   gtggcacttt caaagatgta ataaccgaag ggatggtcgg cactaagtac tgagagatga   1980
   agctggtgat cgatcatatg gtaaaagact tagtttcagt tttc                    2024
<210> 16
   <211> 2024
   <212> DNA
   <213> Artificial Sequence
<220>
   <223> Description of Artificial Sequence: Synthetic polynucleotide
<400> 16
   tctctcatct aaccatggcg gcggcaacat cgtcttctcc gatctcctta accgctaaac      60
   cttcttccaa atcccctcta cccatttcca gattctccct tcccttctcc ttaaccccac     120
   agaaaccctc ctcccgtctc caccgtcctc tcgccatctc cgccgttctc aactcacccg     180
   tcaatgtcgc acctgaaaaa accgacaaga tcaagacttt catctcccgc tacgctcccg     240
   acgagccccg caagggtgct gatatcctcg tggaagccct cgagcgtcaa ggcgtcgaaa     300
   ccgtcttcgc ttatcccgga ggtacttcca tggagatcca ccaagccttg actcgctcct 360
   ccaccatccg taacgtcctc ccccgtcacg aacaaggagg agtcttcgcc gccgagggtt 420
   acgctcgttc ctccggcaaa ccgggaatct gcatagccac ttcgggtccc ggagctacca 480
   acctcgtcag cgggttagcc gacgcgatgc ttgacagtgt tcctctcgtc gccatcacag 540
   gacaggtccc tcgccggatg atcggtactg acgcgttcca agagacgcca atcgttgagg 600
   taacgaggtc tattacgaaa cataactatc tggtgatgga tgttgatgac atacctagga 660
   tcgttcaaga agctttcttt ctagctactt ccggtagacc cggaccggtt ttggttgatg 720
   ttcctaagga tattcagcag cagcttgcga ttcctaactg ggatcaacct atgcgcttgc 780
   ctggctacat gtctaggctg cctcagccac cggaagtttc tcagttaggt cagatcgtta 840
   ggttgatctc ggagtctaag aggcctgttt tgtacgttgg tggtggaagc ttgaactcga 900
   gtgaagaact ggggagattt gtcgagctta ctgggatccc tgttgcgagt acgttgatgg 960
   ggcttggctc ttatccttgt aacgatgact tgtccctgca gatgcttggc atgcacggga 1020
   ctgtgtatgc taactacgct gtggagcata gtgatttgtt gctggcgttt ggtgttaggt 1080
   ttgatgaccg tgtcacggga aagctcgagg cgtttgcgag cagggctaag attgtgcaca 1140
   tagacattga ttctgctgag attgggaaga ataagacacc tcacgtgtct gtgtgtggtg 1200
   atgtaaagct ggctttgcaa gggatgaaca aggttcttga gaaccgggcg gaggagctca 1260
   agcttgattt cggtgtttgg aggagtgagt tgagcgagca gaaacagaag ttcccgttga 1320
   gcttcaaaac gtttggagaa gccattcctc cgcagtacgc gattcaggtc ctagacgagc 1380
   taacccaagg gaaggcaatt atcagtactg gtgttggaca gcatcagatg tgggcggcgc 1440
   agttttacaa gtacaggaag ccgaggcagt ggctgtcgtc ctcaggactc ggagctatgg 1500
   gtttcggact tcctgctgcg attggagcgt ctgtggcgaa ccctgatgcg attgttgtgg 1560
   acattgacgg tgatggaagc ttcataatga acgttcaaga gctggccaca atccgtgtag 1620
   agaatcttcc tgtgaagata ctcttgttaa acaaccagca tcttgggatg gtcatgcaat 1680
   gggaagatcg gttctacaaa gctaacagag ctcacactta tctcggggac ccggcaaggg 1740
   agaacgagat cttccctaac atgctgcagt ttgcaggagc ttgcgggatt ccagctgcga 1800
   gagtgacgaa gaaagaagaa ctccgagaag ctattcagac aatgctggat acacctggac 1860
   cgtacctgtt ggatgtcatc tgtccgcacc aagaacatgt gttaccgatg atcccaaatg 1920
   gtggcacttt caaagatgta ataaccgaag ggatggtcgc cactaagtac tgagagatga 1980
   agctggtgat cgatcatatg gtaaaagact tagtttcagt tttc 2024
<210> 17
   <211> 652
   <212> PRT
   <213> Artificial Sequence
<220>
   <223> Description of Artificial Sequence: Synthetic polypeptide
<400> 17
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Pro Ser Ser Arg Leu His Arg Pro Leu Ala Ile
   35 40 45
   Ser Ala Val Leu Asn Ser Pro Val Asn Val Ala Pro Glu Lys Thr Asp
   50 55 60
   Lys Ile Lys Thr Phe Ile Ser Arg Tyr Ala Pro Asp Glu Pro Arg Lys
   65 70 75 80
   Gly Ala Asp Ile Leu Val Glu Ala Leu Glu Arg Gln Gly Val Glu Thr
   85 90 95
   Val Phe Ala Tyr Pro Gly Gly Ala Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225                 230                 235                 240
   Gln Gln Gln Leu Ala Ile Pro Asn Trp Asp Gln Pro Met Arg Leu Pro
                   245                 250                 255
   Gly Tyr Met Ser Arg Leu Pro Gln Pro Pro Glu Val Ser Gln Leu Gly
               260                 265                 270
   Gln Ile Val Arg Leu Ile Ser Glu Ser Lys Arg Pro Val Leu Tyr Val
           275                 280                 285
   Gly Gly Gly Ser Leu Asn Ser Ser Glu Glu Leu Gly Arg Phe Val Glu
       290                 295                 300
   Leu Thr Gly Ile Pro Val Ala Ser Thr Leu Met Gly Leu Gly Ser Tyr
   305                 310                 315                 320
   Pro Cys Asn Asp Glu Leu Ser Leu Gln Met Leu Gly Met His Gly Thr
                   325                 330                 335
   Val Tyr Ala Asn Tyr Ala Val Glu His Ser Asp Leu Leu Leu Ala Phe
               340                 345                 350
   Gly Val Arg Phe Asp Asp Arg Val Thr Gly Lys Leu Glu Ala Phe Ala
           355                 360                 365
   Ser Arg Ala Lys Ile Val His Ile Asp Ile Asp Ser Ala Glu Ile Gly
       370                 375                 380
   Lys Asn Lys Thr Pro His Val Ser Val Cys Gly Asp Val Lys Leu Ala
   385                 390                 395                 400
   Leu Gln Gly Met Asn Lys Val Leu Glu Asn Arg Ala Glu Glu Leu Lys
                   405                 410                 415
   Leu Asp Phe Gly Val Trp Arg Ser Glu Leu Ser Glu Gln Lys Gln Lys
               420                 425                 430
   Phe Pro Leu Ser Phe Lys Thr Phe Gly Glu Ala Ile Pro Pro Gln Tyr
           435                 440                 445
   Ala Ile Gln Val Leu Asp Glu Leu Thr Gln Gly Lys Ala Ile Ile Ser
       450                 455                 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545 550 555 560
   Tyr Lys Ala Asn Arg Ala His Thr Tyr Leu Gly Asp Pro Ala Arg Glu
   565 570 575
   Asn Glu Ile Phe Pro Asn Met Leu Gln Phe Ala Gly Ala Cys Gly Ile
   580 585 590
   Pro Ala Ala Arg Val Thr Lys Lys Glu Glu Leu Arg Glu Ala Ile Gln
   595 600 605
   Thr Met Leu Asp Thr Pro Gly Pro Tyr Leu Leu Asp Val Ile Cys Pro
   610 615 620
   His Gln Glu His Val Leu Pro Met Ile Pro Ser Gly Gly Thr Phe Lys
   625 630 635 640
   Asp Val Ile Thr Glu Gly Asp Gly Arg Thr Lys Tyr
   645 650
<210> 18
   <211> 652
   <212> PRT
   <213> Artificial Sequence
<220>
   <223> Description of Artificial Sequence: Synthetic polypeptide
<400> 18
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Pro Ser Ser Arg Leu His Arg Pro Leu Ala Ile
   35 40 45
   Ser Ala Val Leu Asn Ser Pro Val Asn Val Ala Pro Glu Lys Thr Asp
   50 55 60
   Lys Ile Lys Thr Phe Ile Ser Arg Tyr Ala Pro Asp Glu Pro Arg Lys
   65 70 75 80
   Gly Ala Asp Ile Leu Val Glu Ala Leu Glu Arg Gln Gly Val Glu Thr
   85 90 95
   Val Phe Ala Tyr Pro Gly Gly Ala Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Asp Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545 550 555 560
   Tyr Lys Ala Asn Arg Ala His Thr Tyr Leu Gly Asp Pro Ala Arg Glu
   565 570 575
   Asn Glu Ile Phe Pro Asn Met Leu Gln Phe Ala Gly Ala Cys Gly Ile
   580 585 590
   Pro Ala Ala Arg Val Thr Lys Lys Glu Glu Leu Arg Glu Ala Ile Gln
   595 600 605
   Thr Met Leu Asp Thr Pro Gly Pro Tyr Leu Leu Asp Val Ile Cys Pro
   610 615 620
   His Gln Glu His Val Leu Pro Met Ile Pro Ser Gly Gly Thr Phe Lys
   625 630 635 640
   Asp Val Ile Thr Glu Gly Asp Gly Arg Thr Lys Tyr
   645 650
<210> 19
   <211> 652
   <212> PRT
   <213> Artificial Sequence
<220>
   <223> Description of Artificial Sequence: Synthetic polypeptide
<400> 19
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Pro Ser Ser Arg Leu His Arg Pro Leu Ala Ile
   35 40 45
   Ser Ala Val Leu Asn Ser Pro Val Asn Val Ala Pro Glu Lys Thr Asp
   50 55 60
   Lys Ile Lys Thr Phe Ile Ser Arg Tyr Ala Pro Asp Glu Pro Arg Lys
   65 70 75 80
   Gly Ala Asp Ile Leu Val Glu Ala Leu Glu Arg Gln Gly Val Glu Thr
   85 90 95
   Val Phe Ala Tyr Pro Gly Gly Ala Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Asp Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545                 550                 555                 560
   Tyr Lys Ala Asn Arg Ala His Thr Tyr Leu Gly Asp Pro Ala Arg Glu
                   565                 570                 575
   Asn Glu Ile Phe Pro Asn Met Leu Gln Phe Ala Gly Ala Cys Gly Ile
               580                 585                 590
   Pro Ala Ala Arg Val Thr Lys Lys Glu Glu Leu Arg Glu Ala Ile Gln
           595                 600                 605
   Thr Met Leu Asp Thr Pro Gly Pro Tyr Leu Leu Asp Val Ile Cys Pro
       610                 615                 620
   His Gln Glu His Val Leu Pro Met Ile Pro Asn Gly Gly Thr Phe Lys
   625                 630                 635                 640
   Asp Val Ile Thr Glu Gly Asp Gly Arg Thr Lys Tyr
                   645                 650
<210> 20
   <211> 652
   <212> PRT
   <213> Artificial Sequence
<220>
   <223> Description of Artificial Sequence: Synthetic polypeptide
<400> 20
   Met Ala Ala Ala Thr Ser Ser Ser Pro Ile Ser Leu Thr Ala Lys Pro
   1               5                   10                  15
   Ser Ser Lys Ser Pro Leu Pro Ile Ser Arg Phe Ser Leu Pro Phe Ser
               20                  25                  30
   Leu Thr Pro Gln Lys Pro Ser Ser Arg Leu His Arg Pro Leu Ala Ile
           35                  40                  45
   Ser Ala Val Leu Asn Ser Pro Val Asn Val Ala Pro Glu Lys Thr Asp
       50                  55                  60
   Lys Ile Lys Thr Phe Ile Ser Arg Tyr Ala Pro Asp Glu Pro Arg Lys
   65                  70                  75                  80
   Gly Ala Asp Ile Leu Val Glu Ala Leu Glu Arg Gln Gly Val Glu Thr
                   85                  90                  95
   Val Phe Ala Tyr Pro Gly Gly Thr Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Asp Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545 550 555 560
   Tyr Lys Ala Asn Arg Ala His Thr Tyr Leu Gly Asp Pro Ala Arg Glu
   565 570 575
   Asn Glu Ile Phe Pro Asn Met Leu Gln Phe Ala Gly Ala Cys Gly Ile
               580                 585                 590
   Pro Ala Ala Arg Val Thr Lys Lys Glu Glu Leu Arg Glu Ala Ile Gln
       595                 600                 605
   Thr Met Leu Asp Thr Pro Gly Pro Tyr Leu Leu Asp Val Ile Cys Pro
   610                 615                 620
   His Gln Glu His Val Leu Pro Met Ile Pro Ser Gly Gly Thr Phe Lys
   625                 630                 635                     640
   Asp Val Ile Thr Glu Gly Asp Gly Arg Thr Lys Tyr
                   645                 650
<210> 21
   <211> 652
   <212> PRT
   <213> Artificial Sequence
<220>
   <223> Description of Artificial Sequence: Synthetic polypeptide
<400> 21
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Pro Ser Ser Arg Leu His Arg Pro Leu Ala Ile
   35 40 45
   Ser Ala Val Leu Asn Ser Pro Val Asn Val Ala Pro Glu Lys Thr Asp
   50 55 60
   Lys Ile Lys Thr Phe Ile Ser Arg Tyr Ala Pro Asp Glu Pro Arg Lys
   65 70 75 80
   Gly Ala Asp Ile Leu Val Glu Ala Leu Glu Arg Gln Gly Val Glu Thr
   85 90 95
   Val Phe Ala Tyr Pro Gly Gly Thr Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Asp Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545 550 555 560
   Tyr Lys Ala Asn Arg Ala His Thr Tyr Leu Gly Asp Pro Ala Arg Glu
   565 570 575
   Asn Glu Ile Phe Pro Asn Met Leu Gln Phe Ala Gly Ala Cys Gly Ile
   580 585 590
   Pro Ala Ala Arg Val Thr Lys Lys Glu Glu Leu Arg Glu Ala Ile Gln
   595 600 605
   Thr Met Leu Asp Thr Pro Gly Pro Tyr Leu Leu Asp Val Ile Cys Pro
   610 615 620
   His Gln Glu His Val Leu Pro Met Ile Pro Asn Gly Gly Thr Phe Lys
   625                 630                 635                 640
   Asp Val Ile Thr Glu Gly Asp Gly Arg Thr Lys Tyr
                   645                 650
<210> 22
   <211> 2013
   <212> DNA
   <213> Arabidopsis thaliana
<400> 22
   atggcggcgg caacaacaac aacaacaaca tcttcttcga tctccttctc caccaaacca      60
   tctccttcct cctccaaatc accattacca atctccagat tctccctccc attctcccta     120
   aaccccaaca aatcatcctc ctcctcccgc cgccgcggta tcaaatccag ctctccctcc     180
   tccatctccg ccgtgctcaa cacaaccacc aatgtcacaa ccactccctc tccaaccaaa     240
   cctaccaaac ccgaaacatt catctcccga ttcgctccag atcaaccccg caaaggcgct     300
   gatatcctcg tcgaagcttt agaacgtcaa ggcgtagaaa ccgtattcgc ttaccctgga     360
   ggtgcatcaa tggagattca ccaagcctta acccgctctt cctcaatccg taacgtcctt     420
   cctcgtcacg aacaaggagg tgtattcgca gcagaaggat acgctcgatc ctcaggtaaa     480
   ccaggtatct gtatagccac ttcaggtccc ggagctacaa atctcgttag cggattagcc     540
   gatgcgttgt tagatagtgt tcctcttgta gcaatcacag gacaagtccc tcgtcgtatg     600
   attggtacag atgcgtttca agagactccg attgttgagg taacgcgttc gattacgaag     660
   cataactatc ttgtgatgga tgttgaagat atccctagga ttattgagga agctttcttt     720
   ttagctactt ctggtagacc tggacctgtt ttggttgatg ttcctaaaga tattcaacaa     780
   cagcttgcga ttcctaattg ggaacaggct atgagattac ctggttatat gtctaggatg     840
   cctaaacctc cggaagattc tcatttggag cagattgtta ggttgatttc tgagtctaag     900
   aagcctgtgt tgtatgttgg tggtggttgt ttgaattcta gcgatgaatt gggtaggttt     960
   gttgagctta cggggatccc tgttgcgagt acgttgatgg ggctgggatc ttatccttgt    1020
   gatgatgagt tgtcgttaca tatgcttgga atgcatggga ctgtgtatgc aaattacgct    1080
   gtggagcata gtgatttgtt gttggcgttt ggggtaaggt ttgatgatcg tgtcacgggt    1140
   aagcttgagg cttttgctag tagggctaag attgttcata ttgatattga ctcggctgag    1200
   attgggaaga ataagactcc tcatgtgtct gtgtgtggtg atgttaagct ggctttgcaa    1260
   gggatgaata aggttcttga gaaccgagcg gaggagctta agcttgattt tggagtttgg    1320
   aggaatgagt tgaacgtaca gaaacagaag tttccgttga gctttaagac gtttggggaa    1380
   gctattcctc cacagtatgc gattaaggtc cttgatgagt tgactgatgg aaaagccata 1440
   ataagtactg gtgtcgggca acatcaaatg tgggcggcgc agttctacaa ttacaagaaa 1500
   ccaaggcagt ggctatcatc aggaggcctt ggagctatgg gatttggact tcctgctgcg 1560
   attggagcgt ctgttgctaa ccctgatgcg atagttgtgg atattgacgg agatggaagc 1620
   tttataatga atgtgcaaga gctagccact attcgtgtag agaatcttcc agtgaaggta 1680
   cttttattaa acaaccagca tcttggcatg gttatgcaat gggaagatcg gttctacaaa 1740
   gctaaccgag ctcacacatt tctcggggat ccggctcagg aggacgagat attcccgaac 1800
   atgttgctgt ttgcagcagc ttgcgggatt ccagcggcga gggtgacaaa gaaagcagat 1860
   ctccgagaag ctattcagac aatgctggat acaccaggac cttacctgtt ggatgtgatt 1920
   tgtccgcacc aagaacatgt gttgccgatg atcccgagtg gtggcacttt caacgatgtc 1980
   ataacggaag gagatggccg gattaaatac tga 2013
<210> 23
   <211> 670
   <212> PRT
   <213> Arabidopsis thaliana
<400> 23
   Met Ala Ala Ala Thr Thr Thr Thr Thr Thr Ser Ser Ser Ile Ser Phe
   1               5                   10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
   130 135 140
   Gln Gly Gly Val Phe Ala Ala Glu Gly Tyr Ala Arg Ser Ser Gly Lys
   145 150 155 160
   Pro Gly Ile Cys Ile Ala Thr Ser Gly Pro Gly Ala Thr Asn Leu Val
   165 170 175
   Ser Gly Leu Ala Asp Ala Leu Leu Asp Ser Val Pro Leu Val Ala Ile
   180 185 190
   Thr Gly Gln Val Pro Arg Arg Met Ile Gly Thr Asp Ala Phe Gln Glu
   195 200 205
   Thr Pro Ile Val Glu Val Thr Arg Ser Ile Thr Lys His Asn Tyr Leu
   210 215 220
   Val Met Asp Val Glu Asp Ile Pro Arg Ile Ile Glu Glu Ala Phe Phe
   225 230 235 240
   Leu Ala Thr Ser Gly Arg Pro Gly Pro Val Leu Val Asp Val Pro Lys
   245 250 255
   Asp Ile Gln Gln Gln Leu Ala Ile Pro Asn Trp Glu Gln Ala Met Arg
   260 265 270
   Leu Pro Gly Tyr Met Ser Arg Met Pro Lys Pro Pro Glu Asp Ser His
   275 280 285
   Leu Glu Gln Ile Val Arg Leu Ile Ser Glu Ser Lys Lys Pro Val Leu
   290 295 300
   Tyr Val Gly Gly Gly Cys Leu Asn Ser Ser Asp Glu Leu Gly Arg Phe
   305 310 315 320
   Val Glu Leu Thr Gly Ile Pro Val Ala Ser Thr Leu Met Gly Leu Gly
   325 330 335
   Ser Tyr Pro Cys Asp Asp Glu Leu Ser Leu His Met Leu Gly Met His
   340 345 350
   Gly Thr Val Tyr Ala Asn Tyr Ala Val Glu His Ser Asp Leu Leu Leu
   355 360 365
   Ala Phe Gly Val Arg Phe Asp Asp Arg Val Thr Gly Lys Leu Glu Ala
   370 375 380
   Phe Ala Ser Arg Ala Lys Ile Val His Ile Asp Ile Asp Ser Ala Glu
   385 390 395 400
   Ile Gly Lys Asn Lys Thr Pro His Val Ser Val Cys Gly Asp Val Lys
   405 410 415
   Leu Ala Leu Gln Gly Met Asn Lys Val Leu Glu Asn Arg Ala Glu Glu
   420 425 430
   Leu Lys Leu Asp Phe Gly Val Trp Arg Asn Glu Leu Asn Val Gln Lys
   435 440 445
   Gln Lys Phe Pro Leu Ser Phe Lys Thr Phe Gly Glu Ala Ile Pro Pro
   450 455 460
   Gln Tyr Ala Ile Lys Val Leu Asp Glu Leu Thr Asp Gly Lys Ala Ile
   465 470 475 480
   Ile Ser Thr Gly Val Gly Gln His Gln Met Trp Ala Ala Gln Phe Tyr
   485 490 495
   Asn Tyr Lys Lys Pro Arg Gln Trp Leu Ser Ser Gly Gly Leu Gly Ala
   500 505 510
   Met Gly Phe Gly Leu Pro Ala Ala Ile Gly Ala Ser Val Ala Asn Pro
   515 520 525
   Asp Ala Ile Val Val Asp Ile Asp Gly Asp Gly Ser Phe Ile Met Asn
   530 535 540
   Val Gln Glu Leu Ala Thr Ile Arg Val Glu Asn Leu Pro Val Lys Val
   545 550 555 560
   Leu Leu Leu Asn Asn Gln His Leu Gly Met Val Met Gln Trp Glu Asp
   565 570 575
   Arg Phe Tyr Lys Ala Asn Arg Ala His Thr Phe Leu Gly Asp Pro Ala
   580 585 590
   Gln Glu Asp Glu Ile Phe Pro Asn Met Leu Leu Phe Ala Ala Ala Cys
   595 600 605
   Gly Ile Pro Ala Ala Arg Val Thr Lys Lys Ala Asp Leu Arg Glu Ala
   610 615 620
   Ile Gln Thr Met Leu Asp Thr Pro Gly Pro Tyr Leu Leu Asp Val Ile
   625                 630                 635                 640
   Cys Pro His Gln Glu His Val Leu Pro Met Ile Pro Ser Gly Gly Thr
                   645                 650                 655
   Phe Asn Asp Val Ile Thr Glu Gly Asp Gly Arg Ile Lys Tyr
               660                 665                 670